# EUROPEAN PATENT APPLICATION

(11) **EP 2 053 403 A2**
(43) Date of publication of application: **29.04.2009**
(21) Application number: 08169915.9
(22) Date of filing: 16.01.2004
(51) Int. Cl.: G01N 33/68

(54) **Affinity fishing for ligands and proteins receptors**

(30) Priority: 16.01.2003 US 346737; 19.05.2003 DK 200300749
(62) Divisional of application: 04702645.5
(71) Applicant: Novo Nordisk A/S, 2880 Bagsværd (DK)
(72) Inventor: Hilaire St., Phaedria Marie, 2400, Copenhagen (DK); Yin, Haifeng, 2000, Frederiksberg (DK); Surve, Sheryl, 22353, Lund (SE); Wenckens, Martin, 4320, Lejre (DK)
(74) Representative: HOEIBERG A/S

(57) **Abstract**

The invention provides putative "drugable" protein targets and actively binding ligands identified in an efficient and reproducible process by determining the affinity of protein mixtures to libraries of ligand compounds of defined size and composition. The libraries are used to isolate and identify previously unknown corresponding protein-ligand binding pairs from a mixture of proteins and a library of compounds, and are particularly useful to identify differentially selective protein-ligand binding pairs, for example, representing a single physiological state or several varied but related states, such as disease versus normal conditions. The invention also provides processes for identifying such protein-ligand binding pairs.

## Description

This application claims priority from US patent application 10/346,737 and Danish patent application PA 2003 00749, both of which are hereby incoporated by reference in their entirety. All patent and non-patent references cited in said applications or in the present application, are also hereby incorporated by reference in their entirety.

### Field of the Invention

The present invention relates to the use of proteomics and combinatorial chemistry in combination to provide powerful tools and methods to identify ligands and their corresponding protein targets, for example for the drug discovery process, in particular to methods providing novel drug targets and lead compound structures simultaneous.

### Background of the Invention

The recognition and binding of ligands to receptors is a fundamental process providing the molecular architecture of most biological phenomena, including immune recognition, cell signaling, catalysis, metastasis, and pathogenic invasion of a host's cells. Consequently, there has been a driving impetus, both in basic and applied research, to identify and characterize receptors and corresponding ligands with the intent to elucidate biological pathways and to develop therapeutics for the amelioration of various disease states.

This selective interaction between a particular protein and a ligand is the cornerstone of the drug discovery process. Traditionally, the search for such receptor/ligand pairs has been carried out in a sequential manner such that the involvement of a protein in a particular disease is first determined from a genomic/proteomic standpoint (gene knockout, gene sequence analysis, proteomics). Once a protein of interest has been identified and validated as a drug target, suitable ligands can be identified using rational drug design, natural product screening, or screening of putative ligand libraries. Alternatively, a particular protein can be purified from a mixture after a particular ligand known to bind to that family of proteins is identified. See, for example, US patents 5,834,318, and 5,783,663, and published PCT patent application 9838329A1.

In the context of receptor-ligand interactions in the pharmaceutical industry, such sequential approaches are not ideal. Designing ligands for drug targets derived solely from analysis and comparison of an organism's genome or proteome can fail to achieve a desired drug effect because the selected target is not "drugable." The target may prove unsuitable for use as a therapeutic drug due to lack of specificity, toxicity, and the like. Traditional approaches for drug screening have proven relatively effective, but are time-consuming and inefficient. In addition, little consideration is given to the potential toxicity of the drug during the initial phases of traditional selection. These inefficiencies lead to failures in later clinical trial, as well as unnecessary development time and expense. Therefore, approaches to matching receptor-ligand interactions at an early stage in the drug discovery program are highly advantageous. The invention described herein achieves this purpose by rapidly matching unknown proteins with unknown ligands, thus short-listing the number of potential drug targets and their putative drug leads in a single process. This invention, furthermore, provides information on the specificity, cross-reactivity, potential toxicity and other characteristics of the drug lead as well as data relating to possible combination therapies. For example, from the ligand-protein matches it is immediately clear whether a ligand interacts with more than one protein, and whether one of the proteins is of vital importance for the functioning of the cell (potential toxicity effect). It will also be apparent whether several ligands interact with a single protein, thus increasing the number of potential drug leads and possibilities for combination therapy.

An alternative approach to identifying ligands and receptors when the precise nature of the ligand and target are unknown has been described by some researchers using phage display. Phage displaying surface peptides target specific receptors in particular organs when applied in an *in vivo* system (see, for example, U.S. patents 5,622,699 and 6,306,365). The phage are then recovered from the organ, the peptide identified, and the receptor subsequently isolated and identified using affinity chromatography. This approach is largely limited to libraries of peptide ligands consisting of the 20 genetically-encoded amino acids, and cannot take advantage of useful synthetic amino acids or diverse small molecule that can modulate biological function. In addition, since the targeting takes place *in viva,* proteolysis of some peptide ligands by adventitious proteases will take place, thus reducing the number of putative ligands, and hence the number of targets that can be identified. Furthermore, it is also essential for phage to be endocytosed by the cell in order to target cytosolic proteins. The primary use of the phage display process is to identify peptides that can be used to deliver drugs to specific cells, organs, and tissues.

In an alternative to displaying peptide libraries on phage, peptide libraries can also be generated in mammalian cells using retroviral vectors (see for example, published PCT patent application WO 09638553 to Inoxell, US patent 6,153,390 to Rigel, and related patents). Libraries of effector molecules (peptides, RNA molecules/ribozymes, or cDNA) are generated in cell lines that model a disease or cellular pathway. After the application of selective pressure and induction of the desired phenotype, the responsible effector molecule(s) is identified, and the corresponding cellular target(s) can be isolated using affinity chromatography and characterized. This approach is again restricted to naturally occurring oligomers. In addition, it is time consuming to develop the appropriate disease model cell. Furthermore, the peptide is expressed in a protein scaffold making it difficult to extrapolate to a small peptide/molecule drug.

The present invention capitalizes on progress made in the field of proteomics. For recent reviews discussing the state of this art see Peng et al., 2001, J. Mass Spectrom., 36: 1083-1091; and Yarmush et al., 2002, Annu. Rev. Biomed. Eng., 4: 349-373. In proteomics, the proteins of a cell are typically separated by 2-dimensional (2-D) gel electrophoresis and
characterized by a combination of enzymatic digests and mass spectrometry (MS). When used to identify proteins that are potentially important in a diseased state, for example, 2-D gels displaying protein obtained from normal and abnormal states are compared and differences in protein expression are identified. Proteins obtained from normal and abnormal samples can be differentially labeled (see for example, Unlu et al., 1997, Electrophoresis, 18: 2071-2077; and Gygi et al., 1999. Nat. Biotechnol., 17: 994-999). The differentially labeled proteins can be separated on a single 2-D gel before tryptic digestion and MS identification of the changed proteins as described, for example, in Unlu et al., 1997, *Supra.* Alternatively, the differentially labeled proteins can be first enzymatically digested and the peptides separated by liquid chromatography before MS analysis. See, for example, Gygi et al., 1999 *Supra;* and Washburn et al., 2001, Nat. Biotechnol., 19: 242-247. The use of two-dimensional gels for profiling an organism's proteome is not simple and is fraught with problems. The entire process from casting gels and protein solubilization to interpreting the protein patterns obtained poses numerous challenges. With careful attention to detail, individual laboratories might reproduce 2-D protein patterns; however, in practice, it is rare for different groups to obtain the same 2-D pattern, rendering comparison of data between laboratories difficult and the creation of shared databases largely pointless (see, Defrancesco, 1999, *The Scientist,* 13: 16). Furthermore, use of 2-D gels limits the size of proteins that can be separated. It is particularly difficult to isolate and identify membrane proteins and low abundance proteins by these techniques. The use of gel-free systems reduces the problems of size and abundance; however, in some cases, labeling procedures are limited as they require the presence of particular amino acids in a protein, and analysis of the mass spectra is difficult.

In a variation on proteomic profiling, proteins from a crude mixture can be captured on the surface of a chip, for example, a 2 mm chip, bearing any of a variety of affinity surfaces: antibodies, known protein receptors, nucleic acids, carbohydrates, and the like. Protein or proteins that bind to the chip can then be analyzed and identified, for example, using a technology called SELDI™ (surface-enhanced laser desorption/ionization) mass spectrometry. See for example, Davies et al., 1999, Biotechniques, 27: 1258-1261 and the world wide web (www) site: ciphergen.com. To utilize this technique, the immobilized binding partner must first be synthesized (peptide, carbohydrate, nucleic acid), isolated (protein receptor), or generated (antibody) before it is immobilized on the surface. Additionally, the immobilization procedure should not affect the nature and active conformation of the ligand. Thus, considerable effort can be expended to optimize immobilization for a particular set of ligands. This technique is also plagued by non-specific binding interactions, due in part to denaturing of the proteins in the crude mixture on the chip surface. Furthermore, relatively few binding partners can be immobilized on a single chip.

In an attempt to extend proteomic profiling to a wider array of compounds, a small, encoded soluble library (six compounds) was synthesized and screened with individual proteins in solution phase (see, for example, Winssinger et al., 200 1, Angew. Chem. Int. Ed. Engl., 40: 3152-3155). The members of the library were laboriously encoded with a polynucleic acid tag (PNA tag) enabling the binding partners to be identified by hybridization to a DNA microarray. After identification of the ligand, the binding protein(s) can be purified by affinity purification after re-synthesis of the identified ligand and attachment to a suitable support, and then identified using mass spectrometry. In this approach, the initial screening takes place in solution and is subject to many well-known problems. For example, as proteins bound to ligands are separated from proteins without ligands using gel-filtration chromatography, some ligands are lost, and only binding interactions that are extremely tight with very slow off rates (high-binding affinities) will be detected. Furthermore, some proteins may interact with the encoding PNA tag (wholly or partially) leading to a false positive or preventing hybridization and identification of a "true" positive binding pair. Finally, once an active ligand(s) is identified, the binding protein is identified using conventional affinity chromatography requiring resynthesis and immobilization of the ligand to a solid support, binding of the protein(s), and elution of the proteins(s) under the appropriate conditions, each procedure adding time and inefficiency to the selection process.

The present invention provides a novel, efficient, and effective process for identifying and matching ligands and putative drug targets with tremendous speed (lending itself to automation) and with few limitations as compared to known processes.

In the prior art, methods for screening arrays of materials for bioactive compounds have been described. WO 00/63694 describes a method for identifying bioactive compounds by screening a library with only one proteome, and subsequently identifying proteins associated with components of said library. The library may be a library of natural oligomers or oligomers of peptide like compounds. The library may be immobilized on for example sepharose or agarose beads.

### Summary of the Invention

In the process of the present invention, previously unknown, specific protein-ligand binding pairs are isolated and identified from a mixture of proteins and a ligand library by virtue of specific binding, isolation, and identification. In a preferred embodiment, a library of spatially separated ligands, immobilized on a solid support, is incubated with a mixture of proteins, such as proteins that have been isolated from cells, tissue, or organisms. The protein mixture can be labeled with a detection probe. After incubation of the immobilized ligands with the protein mixture, active ligands, that is, those ligands that bind protein, are isolated and identified, for example, by mass spectroscopy or NMR, such as high-resolution NMR, preferably directly from the binding complex, for example, "on bead". Protein(s) bound to identified active ligand(s) are identified, preferably from the same binding complex, for example, by mass spectroscopy (peptide mass fingerprinting and peptide sequencing), or other known processes. Alternatively, an identified active ligand can be used to isolate its specific binding protein receptor. The isolated protein receptor is then identified, for example, by mass spectrometry, peptide mass fingerprinting and peptide sequencing)or other useful methods known to the person skilled in the art.

In one particularly preferred embodiment of the invention, a ligand library is incubated with two or more differentially labeled protein mixtures, for example, obtained from two or more different protein sources, such as a normal set of proteins obtained from normal tissue and an abnormal set of proteins obtained from diseased tissue. The protein sets are preferably mixed, and then incubated with a ligand library. Ligand-protein complexes are isolated, for example, according to the specific protein labels. Ligands that selectively and/or differentially bind with one set of proteins are identified. The protein(s) binding to these selective ligands can be identified from the same binding complex, for example, on a single resin bead. Alternatively, the identified selective ligands can be used to isolate the corresponding binding protein(s) that are then identified. Hence, the methods may be useful in the identification of ligands binding differentially to two protein mixtures. Proteins that are differentially expressed in a particular diseased state may be useful drug targets and the methods according to the invention thus allow for example identification of potential drug targets and binding ligands for the treatment of a particular disease.

The inventive process provides a rapid and efficient identification of specific members of a previously unknown ligand-protein binding pair. The process can be readily automated, providing greater efficiencies. In a most preferred embodiment, efficiencies are achieved by carrying out multiple process steps using the same reactants, for example, synthesizing the ligand library directly onto a solid support that is then used for incubating the ligand with the protein mixture; detecting the specific ligand-protein binding pairs while immobilized on the same solid support, and identifying each of the ligand and protein from the same immobilized binding complex. "On-bead" identification allows idenfication of even very small amounts of ligand and/or protein. Accordingly, the process of the invention eliminates transfers, additional synthetic transformations, purification, and other steps that reduce efficiency, and otherwise impede in the discovery of ligand-binding interactions. Using the process of the invention, novel ligand-protein binding pairs are efficiently detected and identified, and provided as drug leads and targets. Further verification of the proteins and ligands usefulness as drug target may be obtained by, for example, comparison with known drug targets and leads, comparison of an organism's genome, an analysis of the proteins function and by testing of the identified ligands in biological assays.

It is furthermore an objective of the invention to provide ligands identified by the methods according to the invention as well as ligand-protein binding pairs identified by the methods according to the invention.

### Brief Description of the Drawings

The invention may be more completely understood with reference to the following detailed description of various embodiments of the invention and specific working Examples in connection with the accompanying Figures, in which:
Figure 1 is a schematic representation of one embodiment of the process invention, showing a ligand library incubated with a single set of proteins to identify specific ligand-protein binding pairs.
Figure 2 is a schematic representation of one embodiment of the process invention, showing a ligand library incubated with two or more different sets of proteins to identify specific and selective differential ligand-protein binding pairs.

Figure 3 demonstrates inhibition of DNA Synthesis (Cardiac Hypertrophy) by selected peptides identified using the process of this invention. The columns indicate average induction of DNA synthesis in cardiac myocytes treated with peptides. The Y-axis indicates fold induction (control basal =100%).

### Detailed Description of the Invention

### Definitions:

As used herein, the following words are intended to have the specified definitions:
**Amino acids** may be any compound of natural or synthetic origin, containing an amino-group and carboxylic acid. Naturally occurring amino acids are identified using either their 1-letter or 3-letter code throughout the description. Amino acids may for example be either D-amino acids or L-amino acids.

**Affinity probe** refers to a detection probe that uses a binding (affinity) interaction as part of the detection process, for example biotin-avidin, antibody-antigen and the like.

Detection probe refers to a compound, generally a small molecule, peptide or protein, polynucleotide, and the like, that is used for detecting a binding interaction, such as ligand binding to protein. The detection probe may produce a detectable signal, such as color, fluorescence, and the like, or may react with a known probe, such as an affinity probe that provides the detection signal.

**Immobilized** as used herein, means that a molecular entity is covalently attached to a solid support.

**Low abundance proteins** refers to proteins present in low amounts in a protein sample so as to be masked by other proteins in typical detection methods, and include, for example, transcription factors, protein kinases, and phosphatases.

**Library** refers to a collection of molecular entities obtained after a series of one or more synthetic transformations.

**Ligand** refers herein to a molecule that binds to a biological macromolecule, for example a protein and the like.

**Linker** refers herein to a molecular entity that can be used to bind a ligand to a solid support. Preferred in this invention are molecular entities that can be specifically cleaved. Examples include acid labile (Rink amide), base labile (HMBA), photolabile (2-nitrobenzyl and 2 nitrovaleryl), other specific cleavage entities (allyl, silyl, safety catch sulfonamide), and the like.

**Parallel Array** refers to a collection of molecular entities in a ligand library generated by parallel synthesis.

**Peptidomimetic** refers to non-peptide molecules that mimic the binding characteristics of peptides.

**Photoprotein** refers to a protein that emits fluorescence or chemoluminescence, for example green flourescent protein (GFP) or luciferase.

**Previously unknown protein-ligand binding pair** refers to a protein and ligand that are found to bind to each other through the implementation of this process but that specific ligand and protein binding interaction was not known before.

**Protein mixture or mixture of proteins** refer to a solution comprising different proteins. Preferably, the protein mixture has been isolated from one or more kinds of cells, for example from characterized cell cultures, specific cells, cells from a whole organism or tissue, mixtures of cells from normal and/or diseased tissue, and the like. The terms are used interchangeably herein.

**Protein receptor or receptor** refers to a protein that binds to a ligand, and includes, for example, surface receptors, enzymes such as proteases, protein kinases, phosphatases, and the like, transcription factors, co-factors, adaptor proteins, structural proteins, and the like.

**Small organic molecules or compounds** refer herein to non-oligomeric, carbon containing compounds produced by chemical synthesis and generally having a size of less than 600 mass units.

The present invention provides processes for identifying the structure of previously unknown members of specific ligand and protein binding pairs. The invention provides processes using libraries of compositionally defined, spatially separated, yet structurally unknown ligands to isolate protein receptors from protein mixtures by virtue of specific binding affinity. In one embodiment, using the process and tools of the invention, specific proteins characteristic of biological processes and their matching binding ligands are simultaneously identified. In particular, the invention provides a novel process for identifying particular proteins as potential drug targets together with a matched potential drug lead (ligand).

In one preferred embodiment, the present invention relates to a process for identifying specific members of a previously unknown protein-ligand binding pairs, comprising the steps of:
(a) synthesizing a ligand library onto resin beads to form an immobilized ligand library, wherein each bead of the immobilized library comprises one member of the ligand library;
(b) incubating the immobilized ligand library with two or more differentially labeled protein mixtures;
(c) detecting an immobilized ligand-protein binding pair from the incubation mixture;
(d) identifying the ligand of the specific ligand-binding pair; and
(e) identifying the protein of the ligand-protein binding pair,
wherein the identified ligand and protein are specific members of a previously unknown differential ligand-protein binding pair.

It is preferred, that the step of detecting an immobilised ligand-protein binding pair comprises detecting a ligand of the library that binds differentially with the differentially labeled protein mixtures to form a differential ligand-protein binding pair. This allows identification of ligands for example binding preferentially to one protein mixture rather than another protein mixture.

In another embodiment the present invention relates to a process for identifying specific members of a previously unknown protein-ligand binding pair, comprising the steps of:
(a) synthesizing a ligand library onto resin beads comprising polyethylene glycol to form an immobilized ligand library, wherein each bead of the immobilized library comprises one member of the ligand library;
(b) incubating the immobilized ligand library with one or more protein mixture;
(c) detecting an immobilized ligand-protein binding pair from the incubation mixture;
(c) identifying the ligand of the ligand-binding pair; and
(d) identifying the protein of the ligand-binding pair;
wherein the identified ligand and protein are specific members of a previously unknown ligand-protein binding pair.

In yet another embodiment, the present invention relates to a process for identifying specific members of a previously unknown protein-ligand binding pair, comprising the steps of:
(a) synthesizing a ligand library comprising small organic molecules onto resin beads to form an immobilized ligand library, wherein each bead of the immobilized library comprises one member of the ligand library;
(b) incubating the immobilized ligand library with one or more protein mixture;
(c) detecting an immobilized ligand-protein binding pair from the incubation mixture;
(d) identifying the ligand of the ligand-binding pair; and
(e) identifying the protein of the ligand-binding pair;
wherein the identified ligand and protein are specific members of a previously unknown ligand-protein binding pair.

As used herein, the term "ligand" refers to a molecule that binds to a protein. In particular, ligands are molecules capable of specifically associating with one or more proteins, The identified members of a ligand-protein binding pair are useful as potential drug targets and lead compounds. For example, the protein of an identified ligand-protein binding pair may be useful as a drug target, whereas the ligand of an identified ligand-protein binding pair may be useful as a pharmaceutical compound or as a lead compound during drug development. Furthermore, the protein-ligand complexes isolated and identified by the process invention are also useful to aid in mapping out biological pathways and pointing to functions of the identified protein. The process invention provides preliminary information on potential combination drug therapy as well as potential toxic effects of the drug or drug candidate.

This process invention provides significant advantages over alternative processes of drug discovery by virtue of its ease, speed, broad generality and applicability, and yields a large amount of information in a short time. Furthermore, as demonstrated in the Examples below (for example, Examples 32 and 33), the process of the invention provides matching ligand-protein pairs for low abundance proteins, hydrophobic proteins, and membrane proteins (for example, G-coupled protein receptors) that are typically difficult to isolate and match with a binding partner.

### The Solid Phase Library

In the present invention, libraries of compounds are used to screen biological mixtures. As used herein, the term "library" means a collection of molecular entities obtained after a series of chemical transformation. In one embodiment, these molecular entities can be natural oligomers (occurring in Nature) such as peptides, glycopeptides, lipopeptides, nucleic acids (DNA or RNA), or oligosaccharides. The libraries may comprise different natural oligomers or the libraries may comprise only one kind of natural oligomer, for example the library may be a peptide library. In another embodiment, they can be unnatural oligomers (not occurring in Nature) such as chemically modified peptides, glycopeptides, nucleic acids (DNA or RNA), or, oligosaccharides, and the like. Said chemical modification may for example be the use of unnatural building blocks connected by the natural bond linking the units (for example, the peptide/amide as shown in Example 5), the use of natural building blocks with modified linking units (for example, oligoureas as discussed in Boeijen et al, 2001, J. Org. Chem., 66: 8454-8462; oligosulfonamides as discussed in Monnee et al, 2000, Tetrahedron Lett., 41: 7991-95), or combinations of these (for example, statine amides as discussed in Dolle et al, 2000, J. Comb. Chem., 2: 716-31.). Preferred unnatural oligomers include oligomers comprising unnatural building blocks connected to each other by a naturally occurring bond linking. Said oligomers may thus comprise a mixture of naturally occurring and unnatural building blocks linked to each other by naturally occurring bonds. By way of example, the oligomer may comprise naturally occurring amino acids and unnatural building blocks linked by peptide bonds. Thus, in one embodiment of the invention preferred oligomers comprise modified amino acids or amino acid mimics, for example, the oligomers may comprise any of the compounds mentioned in Table 2 or 3 or 9, for example the oligomers may consist of one or more of the monomers mentioned in table 2 and 9. Other preferred unnatural oligomers include, for example oligoureas, poly azatides, aromatic C-C linked oligomers and aromatic C-N linked oligomers. Still other preferred oligomers comprise a mixture of natural and unnatural building blocks and natural and unnatural linking bonds. For example, the unnatural oligomer may be any of the oligomers mentioned in recent reviews see: Graven et al., 2001, J. Comb. Chem., 3: 441-52; St. Hilaire et al., 2000, Angew. Chem. Int. Ed. Engl., 39: 1162-79; James, 2001, Curr. Opin. Pharmacol., 1: 540-6; Marcaurelle et al., 2002, Curr. Opin. Chem. Biol., 6: 289-96; Breinbauer et al., 2002, Angew. Chem. Int. Ed. Engl., 41: 2879-90. In yet another embodiment, the molecular entities may comprise non-oligomeric molecules such a peptidomimetics or other small organic molecules. Peptidomimetics are compounds that mimic the action of a peptidic messenger, such as bicyclic thiazolidine lactam peptidomimetics of L-proplyl-L-leucyl-glycinamide (Khalil et al, 1999, J. Med. Chem., 42: 2977-87). In a preferred embodiment of the invention, the library comprises or even more preferably consists of small organic molecules. Small organic molecules are non-oligomeric compounds of less than about 600 mass units containing any of a variety of possible functional groups and are the product of chemical synthesis, or isolated from nature, or isolated from nature and then chemically modified, and include, for example, Bayer's urea-based kinase inhibitors (Smith et al., 2001, Bioorg. Med. Chem. Lett., 11: 2775-78). Non-limiting examples of small organic molecule libraries that may be used with the present invention and methods of producing them may for example be found in the reviews Thompson et al., 1996, Chem. Rev., 96: 555-600; Al-Obeidi et al., 1998, Mol. Biotechnol., 9: 205-23; Nefzi et al., 2001, Biopolymers, 60: 212-9; Dolle, 2002, J. Comb. Chem., 4: 369-418. In a preferred embodiment the small organic molecule libraries are prepared starting from one or more basic structures. Said basic structures may for example be selected from the group consisting of alkoxy, aryloxy, acyloxy, thiol, alkylthio, arylthio, heteroarylthio, alkylamino, dialkylamino, acylamino, diacylamino, alkoxyacylamino, dialkoxyacylamino, amides, alkyl, branched alkyl, aryl, heteroaryl, keto, heterocycles, fused ring systems, fused heterocycles and mixtures thereof, wherein each of the aforementioned may be substituted with one or more groups selected from the group consisting of -H, -OH, -SH, halogen, carboxyl, carbonyl, alkoxy, aryloxy, acyloxy, alkylthio, arylthio, heteraarylthio, sulphonyl, sulphoxy, amino, alkylamino, dialkylamino, acylamino, diacylamino, alkoxyacylamino, dialkoxyacylamino, amides, alkyl, aryl, heteroaryl, nitro, cyano, halogeno, silyloxy, keto, heterocycles, fused ring systems, and fused heterocycles. In one embodiment, the basic structure may for example be a halide containing aromatic or heteroaromatic carboxylic acid or acid halide, such as any of the compounds mentioned in table 7. In another embodiment the basic structure may any of the compounds mentioned in table 8. Accordingly, small organic compound libraries may for example comprise compounds comprising one or more aromatic or hetero aromatic ring system(s), wherein the heteroatoms is preferably O, S and/or N, one or more non-aromatic ring systems, which may or may not comprise heteroatoms or various substituted alkyls. Said aromatic or non-aromatic ringsystems may be fused. Each of the aforementioned may be substituted with various substituents e.g. Halide(s) (F, Cl), CH₃-, CF₃-, Methoxy-, thiomethyl-, aldehyde(s)-, carboxylic acids-, esters-, nitrogroups, -H, -OH, -SH, carbonyl, alkoxy, aryloxy, acyloxy, alkylthio, arylthio, heteroarylthio, sulphonyl, sulphoxy, amino, alkylamino, dialkylamino, acylamino, diacylamino, alkoxyacylamino, dialkoxyacylamino, amides, alkyl, aryl, heteroaryl, cyano, halogeno, silyloxy or keto. The small organic compound libraries according to the invention may also comprise mixtures of any of the above mentioned compounds.

The libraries according to the invention may comprise at least 20, such as at least 100, for example at least 1000, such as at least 10,000, for example at least 100,000, such as at least 1,000,000 different compounds. Preferably, the libraries comprises in the range of 20 to 10⁷, more preferably 50 to 7,000,000, even more preferably 100 to 5,000,000, yet more preferably 250 to 2,000,000 different compounds. In a very preferred embodiment of the present invention the libraries comprises in the range of 1000 to 20,000, such as in the range of 20,000 to 200,000 different compounds. The libraries may in one preferred embodiment be synthesized using a split/mix method *(vide infra*) and give rise to one-bead-one-compound libraries. Split/mix methods in general comprise the steps of:
1. Providing several pools of resin beads
2. Performing one or more different chemical synthesis steps on each pool of resin beads
3. Mixing said pools, thereby obtaining a single pool
4. Splitting said pools to obtain new pools
5. Repeating steps 2 to 4.

Selection of the ligand library is dependent upon the desired screening and identification desired. For example, the process invention can utilize a totally random library designed to contain interesting and greatly diverse compounds. An advantage of this approach is that the outcome of the screening is not prejudiced in any specific manner. Since the process invention permits screening of millions of diverse compounds, for example, immobilized in 10 g of resin, a large number, for example in the range of 3 to 5 million, of random molecules can be used in the ligand library.

Alternatively, a smaller, targeted library (hundreds to thousands of compounds) can be used, for example, starting with a known compound or compounds, and providing numerous variations of these known compounds for targeted screening for new ligand-protein binding pairs. The smaller, targeted library can also comprise random molecules.

The library may contain a parallel array of random modifications of one or more ligands. In one embodiment, the library may be formed as a parallel array of random modifications to a known compound or compounds. The array of compounds is preferably prepared on solid phase using techniques known by those skilled in the art. Briefly, the resin may be portioned into a number of vessels or wells, usually less than 500 and the reagents added. There is in general no mixing step and after the appropriate washing steps, subsequent reactions are carried out by addition of additional reagents to the wells. There is no exponential increase in the number of compounds generated and that is equal to the number of vessels used. The ligand can be easily identified by keeping track of the reagent added to each well.

Non-limiting examples of useful libraries are given in the Examples herein below. In one embodiment of the invention the library is a small organic molecule library. Non-limiting, preferred examples of small organic molecule libraries are given in the Examples 36 and 40 (Libraries 5 and 7).

Attachment of a label to a ligand may alter the properties of said ligand. Hence, in one embodiment of the present invention, the ligands are not labelled, i.e. the ligands are not connected to a detectable label, such as a fluorescent component, a nucleic acid or a nucleic acid homologue such as PNA, a dye, a probe comprising a reactive moiety or the like. In particular it is preferred that all ligands are not connected to the same detectable label.

### Solid Support

In this invention, the compounds of the library are preferably bound to a solid support, conferring the advantage of compartmentalized "mini-reaction vessels" for the binding of proteins with an optimal ligand(s). The solid support can be, for example, a polymer bead, thread, pin, sheet, membrane, silicon wafer, or a grafted polymer unit; for example, a Lantern™ (Mimotopes^{®}, found at the website mimotopes.com under combichem/lantems.html).The solid support is preferably not an array to which different library members are bound. Use of resin beads allows easier manipulation than use of an array. In general more compounds may be screened and several of the steps in the procedure may be performed on one bead with sufficient material. Hence, preferably, the library is bound to resin beads. Each member of the library is a unique compound and is physically separated in space from the other compounds in the library, preferably, by immobilizing the library on resin beads, wherein each bead at the most comprises one member of the library. Depending on the mode of library synthesis, each library member may contain, in addition, fragments of the library member. Since ease and speed are important features of this process invention, it is preferred that the screening (incubating) step take place on the same solid support used for synthesis of the library, and also that identification of the members of the binding pair can take place on the same support, such as on a single resin bead. Thus, preferred solid supports useful in the process invention satisfy the criteria of not only being suitable for organic synthesis, but are also suitable for screening procedures, such as "on-bead" screening as described in the Examples below. It is furthermore preferred that the solid support is suitable for "on-bead" identification of ligand/protein as described herein below. Hydrophilic supports described below are useful supports. Screening of libraries and ligands with purified individual proteins or cells has been attempted on individual resin beads such as TentaGel (commercially available from Rapp polymere, Tübingen, Germany), ArgoGel (commercially available from Argonaut Technologies Inc., San Carlos, CA), PEGA (commercially available from Polymer Laboratories, Amherst, MA), POEPOP (Renil et al., 1996, Tetrahedron Lett., 37: 6185-88; available from Versamatrix, Copenhagen, Denmark) and SPOCC (Rademann et al, 1999, J. Am. Chem. Soc., 121: 5459-66; available from Versamatrix, Copenhagen, Denmark). Examples of on-bead screening attempts are described in the following references: Chen et al., 1996, Methods Enzymol., 267: 211-19; Leon et al., 1998, Bioorg. Med. Chem. Lett., 8: 2997-3002; St. Hilaire et al., 1999, J. Comb. Chem., 1: 509-23; Smith et al., 1999, J. Comb. Chem., 1: 326-32; Graven et al., 2001, J. Comb. Chem. 3: 441-52; Park et al., 2002, Lett. Peptide Sci., 8: 171-78). TentaGel and ArgoGel are made up of polyethylene chains grafted on to a polystyrene core. However, use of these supports in biological screening is limited by a size restriction, and by denaturation of certain proteins, particularly enzymes. Solid supports such as acrylamide derivatives, agarose, cellulose, nylon, silica or magnetised particles are described in the prior art. These supports all have certain limitations. For example, acrylamide derivatives, agarose, cellulose, nylon, silica cannot be used in a split/mix library synthesis, and are limited to use in parallel arrays of compounds which have limited diversity. Furthermore, there are severe limitation to the types of chemistry that can be carried out directly on these surfaces thus restricting solid phase library synthesis and ligand analysis. Magnetised particles, depending on their make up, may be useful in a split/mix library synthesis but again the presence of iron particles restricts the types of chemistry and analysis that can be preformed. Whereas Tentagel and Argogel are useful for library synthesis, they are unsuitable for solid phase screening methods because of a non-specific binding, restriction of the size of the biological molecule, denaturation of certain proteins, particularly enzymes. Furthermore, they are unsuitable for identification of the ligand by high resolution-NMR

Preferred solid supports according to the present invention are resin beads, useful for on-bead library synthesis, screening and identification of ligand/protein. Hence, preferred resins according to the present invention are resin comprising polyethylene glycol. More preferably, the resin is PolyEthyleneGlycol Acrylamide copolymer (PEGA), Super Permeable Organic Combinatorial Chemistry (SPOCC) or PolyOxyEthylene-PolyOxyPropylene (POEPOP) resin.

PEGA (PolyEthyleneGlycol Acrylamide copolymer; Meldal M., 1992, Tetrahedron Lett., 33: 3077-80), POEPOP (PolyOxyEthylene-PolyOxyPropylene; Renil et al., 1996, Tetrahedron Lett., 37: 6185-88) and SPOCC (Super Permeable Organic Combinatorial Chemistry; Rademann et al, 1999, J. Am. Chem. Soc., 121: 5459-66) resins are made primarily of polyethylene glycol and swell well in organic as well as aqueous solvents. Because they have very reduced or no non-specific binding, PEGA and SPOCC resins have been effectively used in the screening of myriad proteins including enzymes of different classes. Furthermore, these resins are available in different pore sizes and can allow large proteins to enter while retaining activity. For example, PEGA6000 resins allow proteins up to 600 kDa to enter. In the Examples below, PEGA4000 and PEGA1900 resin with a molecular weight cut off of 200 and 90 kDa, respectively, were used for screening. In principle, any hydrophilic support that is useful for compartmentalized synthesis, retains the activity of the proteins, and has minimal non-specific binding, may be used in this process invention.

### Ligand Library Synthesis

The ligand library may be synthesized by known processes, for example, by parallel synthesis giving rise to small libraries (10 to 1000 members) (for a recent review see: Dolle et al., 2002, J. Comb. Chem., 4: 369-418), or by split/mix or split and combine methodology, as described, for example, in Furka et al., 1991, Int. J. Peptide Protein Res., 37:487-493 and Lam et al., 1991, Nature, 354: 82-84. The split/mix or split and combine method is a preferred method for generating a large library, due to the exponential increase in the number of varied compounds produced. The split/mix method gives rise to a one-bead-one-compound library of large size (1000 to millions of members). In this invention, the one-bead-one-compound library is preferred, and non-limiting examples of such libraries are demonstrated in the Examples below.

The ligand library members may be built up by performing all compound forming reactions directly on a solid phase. Alternatively, the ligand library members can be prepared by linking together preformed building blocks on a solid phase. The resulting library members can be small organic molecules or oligomeric compounds. In both cases, the molecules contain a variety of functional groups. The functional groups can be, for example, alkynes, aldehydes, amides, amines, carbamates, carboxylates, esters, hydroxyls, ketones, thiols, ureas, and the like. The small organic molecule can belong to various classes of compounds, including but not limited to, heterocycles (for example, hydantoins, benzodiazepines, pyrrolydines, isoquinalines), carbocyclic compounds, steroids, nucleotides, alkaloids, and lipids (for reviews containing examples see: Thompson et al., 1996, Chem. Rev., 96: 555-600; Al-Obeidi et al., 1998, Mol. Biotechnol., 9: 205-23; Nefzi et al., 2001, Biopolymers, 60: 212-9; Nicolau et al., 2001, Biopolymers, 60: 171-193; Dolle, 2002, J. Comb. Chem., 4: 369-418).

Where the ligand library members are oligomeric, as demonstrated in the Examples below, the building blocks may be selected from a wide repertoire of suitably protected bi- or tri-functional compounds, for example, amino acids, sulfonic acids, aliphatic acids, aromatic acids, glycosyl amino acids, lipidyl amino acids, heterocyclic amino acids, haloamines, aminohydroxy compounds, diamines, and azido acids. The building blocks may be connected using various types of chemical bonds, for example, an amide, a thioamide, an amine, a sulfonamide, a urea, a thiourea, an ether, a thioether, an ester, a sulfate, a phosphate, a phosphine, a carbonate, a -C-C-bond, , -C-N-bond, a double bond, a triple bond, or a silane. The oligomer may be linked using only one type of chemical bond or using a mixture of bonds. When the library members are amino acids, they preferably are molecules containing about 2 to 40 amino acids. More preferred are molecules of about 3 to 20 amino acids, and most preferred have about 3 to 12 amino acids. For example, molecules of 3, 4, 5, 6, 7, 8, 9, 10, 11, or 12 amino acids work well in the ligand library.

The ligand library members may be directly attached to a solid support or indirectly attached via a variety of linkers, preferably by covalent bonds (For reviews describing linkers for solid phase synthesis, see: Backes et al., 1997, Curr. Opin. Chem. Biol., 1: 86-93; Gordon et al., 1999, J. Chem. Techno Biotechnol., 74: 835-851). The linkers may be acid labile (for example, the Rink amide as described in Rink, 1987, Tetrahedrorrt Lett., 28: 387 and traceless silyl linkers as described in Plunkett et al., 1995, J. Org. Chem., 60: 6006-7), base labile (for example, HMBA as described in Atherton et al. 1981, J. Chem. Soc. Perkin Trans, 1: 538), or photolabile (for example, 2-nitrobenzyl type as described in Homles et al., 1995, J. Org. Chem., 60: 2318-2319). The linkers may be more specific and restrictive of the type of chemistry performed, such as silyl linkers (for example, those cleaved with fluoride as described in Boehm et al., 1996, J. Org. Chem., 62: 6498-99), allyl linkers (for example, Kunz et al., 1988, Angew. Chem. Int. Ed. Engl., 27: 711-713), and the safety catch sulfonamide linker (for example, as described in Kenner et al., 1971, Chem. Commun., 12: 636-7). In one embodiment of the invention the linker may comprise or consist of methionine, such as one Met residue. When the ligand is bound to the resin via Met, it is possible to simultaneously break down the protein and release the ligand in the same chemical step. In the Examples below, the invention is illustrated by the use of a pliotolabile linker, 2-nitrovaleryl (1) as described in Homles et al., 1995, *Supra,* that proves very robust to a wide variety of chemistries and hastens the identification process.

In some embodiments a spacer molecule may be used. When used, the spacer molecule can be a peptide or non-peptide molecule and does not interact with most or all proteins, and thereby does not interfere in the screening process. Such spacers are useful for aiding the identification of the ligand by MALDI-TOF-MS. In other embodiments, a spacer is not used and the ligands may then preferably be identified using high resolution-NMR, Tandem mass spectrometry, of a combination of both.

### Screening Processes

Protein mixtures to be used with the present invention may be derived from a variety of different sources. Protein mixtures to be used with the present invention should comprise at least 100, preferably at least 200, more preferably at least 300, such as at least 500, for example at least 1000 different proteins. In general, the protein mixture will be derived from one or more natural sources, such as for example from living cells, from tissues, from entire individuals, from body fluids such as urine, sputum, serospinal fluid, serum or blood, or from an extracellular matrix. In some embodiments of the present invention more than one different protein mixtures is applied, for example 2, such as 3, for example 4, such as 5, for example in the range of 5 to 10, such as more than 10 different protein mixtures. It is preferred that at least 2 different protein mixtures are used. Preferably, said at least 2 different protein mixtures are mixtures that are desirable to compare. For example, the protein mixture may be mixtures derived from a healthy and a diseased population, respectively, protein mixtures derived from different organisms, protein mixtures derived from different species, protein mixture derived from different tissues, protein mixtures derived from differentially developed organisms or protein mixtures derived from cells or organisms in different states, i.e. cycling versus non-cycling cells. Diseased populations include cells/body fluids/tissues derived from diseased tissue, body fluid or cells derived from an individual with a disease. Said disease could for example be a neoplastic or preneoplastic disease, an infectious disease, an autoimmune disease, a cardiovascular disease, an inflammatory disease, CNS disorders, metabolic diseases or endocrine diseases. In one embodiment of the present invention at least one protein mixture is derived from an infectious species, such as for example fungi, viruses, protozoa or bacteria.

If for example protein mixtures derived from a healthy and a diseased source, respectively are used, the methods may be used to identify ligands capable of specifically interacting with diseased or healthy cells or tissue may be identified. Such ligands may be potential drug candidates. If for example protein mixtures derived from different species,
wherein one species is an infectious agent, ligands interacting specifically with said infectious agent may be identified. Such ligands may also be potential drug candidates.

In one embodiment of the invention, the library is a parallel array of random modifications of a known compound. Then the protein mixture preferably comprises one or more proteins not previously known to associate with said compound, and said proteins may thus be identified by the methods of the invention.

In another embodiment of the invention the protein mixture comprises one or more families of proteins, which enables detection of ligand-protein binding pairs by immunoassays, for example by the aid of antibodies recognising said families of proteins.

In the process invention, biological material is isolated and protein mixtures obtained for screening of the ligand libraries. The proteins can be obtained from any source, including, for example, simple organisms such as fungi, viruses, protozoans and bacteria to more complex organisms such as plants and animals, including mammals and particularly, humans. The biological material may be extracted from individual cell lines, (illustrated in the Examples with myocytes), from cellular organisms (illustrated in the Examples with E. *coli*), or from tissue containing a large variety of cell types or from entire multicellular organisms. In a preferred embodiment at least one protein mixture is a mixture of mammalian proteins, preferably human proteins, such as mammalian (human) tissue cell proteins. Protein mixtures may also comprise recombinantly engineered proteins, for example the protein mixtures may also be obtained from cellular systems expressing a cDNA library that may be tagged, for example, with a genetic label that is co-expressed and used for detection analysis. Suitable genetic tags include, for example, myc and photoproteins such as Green Fluorescent Protein (GFP). Alternatively, the protein mixture may comprise proteins encoded by mutagenised, recombined or otherwise manipulated nucleic acids.

The intricacy of the extraction procedures increases with the complexity of the source of the biological material. There are various known ways of isolating proteins from cells, tissue, and organisms while preserving the activity of the protein. The protein mixtures according to the present invention may be isolated using any standard method known to the person skilled in the art. The proteins can for example be extracted and solubilized using a variety of auxiliary substances such as detergents and ureas. This extraction procedure is particularly important for larger, hydrophobic proteins such as membrane proteins. The use of detergents, ureas, and salt is compatible with screening on solid phase resins in contrast to methods using 2-D gels. Proteins can be extracted using standard equipment such as the French Press and sonicator. The extraction procedure can be manipulated to enrich for low abundance proteins or to isolate a particular class of proteins. General protocols for the extraction of proteins from different organisms are readily available. See, for example, 2-D Proteome Analysis Protocols, A.J. Link (Ed), 1st Ed, 1999, Humana Press: Totowa)

### Detecting

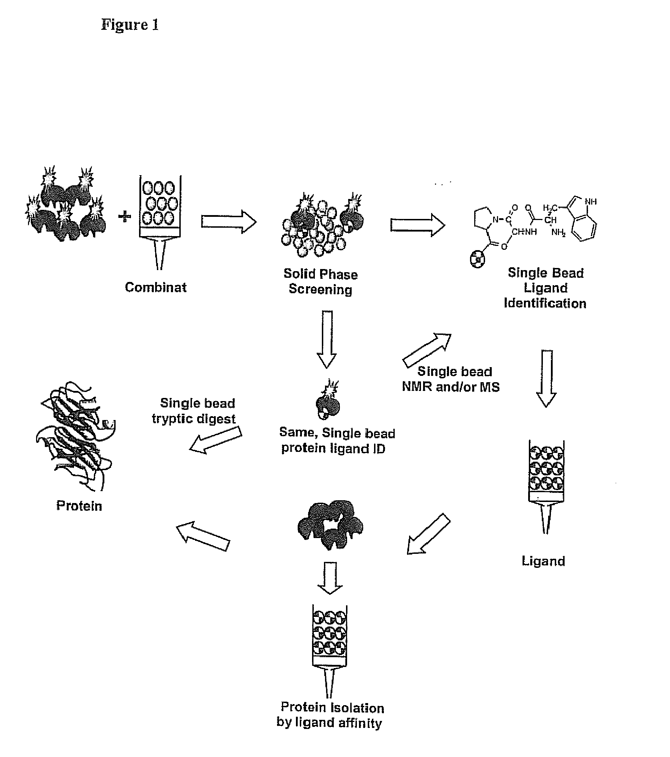

A variety of suitable methods are useful for detecting the ligand-protein binding pairs. For example, where a single protein mixture is used (see Figure 1), the extracted protein may be immediately incubated with the immobilized ligand library, and, after washing, bound protein can be detected directly in the binding complex by the application of a detection molecule to the incubation mixture, such as silver or fluorescent dye that does not interact with the ligand or the solid support. It is however generally preferred that the protein mixture is labeled with a detection probe prior to incubation with the ligand library. Hence, in another embodiment, the mixture of proteins may be labeled with a detection probe, for example, with a fluorescent dye such as Oregon Green 514 (green; See Example 11), N-mehtyl anthranilate (blue; See Example 13), Rhodamine red (red; See Example 11), cyanine dye 2, cyanine dye 3, cyanine dye 5 or other commonly used fluorescent probes. See for example, Richard P. Haugland, "Handbook of Fluorescent Probes and Research Products", 9th Edition, 2002, Molecular Probes Europe BV: Leiden or world wide web (WWW) sites "probes com" and "amershambio-sciences.com/aptrix/upp00919.nsf/Content/DrugScr+CyDye+Fluors+introduction" for a description of cyanine fluorescent dyes. The detection probe may also be a fluorescent protein, such as Green fluorescent proteins or fluorescent mutants thereof. The detection probe can also be a probe that produces chemoluminescence, such as luciferase or aequorin. In these embodiments, after incubation of ligands with proteins, the library is washed and ligand-protein binding complexes will be detected via the label, for example, fluorescence or color. These ligand-protein binding pairs can be immediately isolated using automatic or manual sorting procedures. If the detection probe is a fluorescent probe, then automatic sorting preferably involves the use of a FABS and/or a fluorescence activated beads sorter. The detection probe may furthermore be a compound capable of producing chemiluminescence, such as for example luciferase or aequorin. The detection probe may furthermore be an enzyme capable of catalyzing a detectable reaction, such as for example phosphatase or peroxidase. The detection probe may furthermore be a metal, for example gold. The protein mixture may be labeled with the detection probe by any conventional method depending on the nature of the detection probe.

In particular, when more than one protein mixture is used it is preferred that the protein mixtures are labeled with a detection probe prior to incubation with the ligand library. The individual protein mixtures may be labeled using different detection probes or similar detection probes. It is however preferred that different protein mixture are labeled with different detection probes, to allow identification of from what protein mixture the protein is derived. Hence, it is preferred that 2, such as 3, for example 4, such as 5, for example in the range of 5 to 10 differentially labeled protein mixtures are used with the invention. Detecting an immobilised ligand-protein binding pair may thus involve detectecting a ligand of the library that binds differentially with 2 or more differentially labeled protein mixtures.

It is comprised within the present invention that individual proteins of a protein mixture may be differentially labeled, it is however preferred that all proteins of one protein mixture are labelled with the same kind of detection probe. Any of the detection probes described herein above or below may be used and similar labeling procedures can also be applied to the identification of differential matched ligand-protein binding pairs from multiple, related sources (see Example 11). For example, as taught in Example 11, a mixture of proteins from normal tissue and a mixture of proteins from diseased tissue can be differentially labeled, a different dye or fluorescent label (and the like) for each of the protein mixtures. After incubation with the ligand library and washing away unbound protein, the differential protein-ligand binding pairs, those that demonstrate selectivity, that is, are specific to one set of proteins, are detected and isolated automatically or manually on the basis of the particular label or detection probe.

In another embodiment, proteins are labeled with a detection probe, which is an affinity probe (tag) such as biotin. After incubation and washing of the proteins and ligand library to remove unbound protein, ligands bound with tagged, for example, biotinylated proteins may be detected, for example, using streptavidin complexed with a phosphatase or a peroxidase. After addition of a suitable phosphatase or peroxidase substrate, the ligand-protein binding complex is detected.

In another embodiment, proteins bound to ligands can be detected using radioactivity, i.e. the detection probe may be a radioactive compound. The proteins may be labeled with said radioactive compound by any conventional method. For example, the organism or cell is fed with a radioactive amino acid that is incorporated into its proteins. Af ter incubation of the radioactive proteins with the ligand library and washing, bound radioactive protein-ligand is detected by, for example, autoradiography, and the protein-ligand binding pairs are isolated.

In yet another embodiment, particular classes of proteins that bind to ligands can be detected using specific probes, for example, a family-specific antibody in an immunoassay such as an ELISA assay. Treatment with a conjugated monoclonal antibody for a family of proteins after incubation and washing, for example, provides information about the expression of related proteins. Where the protein mixtures are obtained from related protein sources, for example, from diseased and normal tissue, the ligand libraries can be incubated separately with each set of proteins. After detection and identification of the ligand-protein binding pairs, an assessment of the expression of the particular protein class in each state (for example, normal vs. diseased) can be determined. A monoclonal antibody may be conjugated to a fluorescent dye or to an enzyme such as peroxidase or alkaline phosphatase for quantification by ELISA. The antibody may also be conjugated to ferro-magnetic beads by known, routine techniques. The magnetic beads concentrate near the location of the protein forming a "rosette" around solid support beads, or on the membrane sheet, or thread for detection.

In a final embodiment, for example, where a single protein mixture is used, the extracted protein may be immediately incubated with the immobilized ligand library, and, after washing, detection of bound protein and isolation of the specific ligand-protein binding pair is done without any labelling, for example by measuring refractive index changes of the resin beads. Beads containing both proteins and ligand will have a different refractive index than beads containing only ligand. The refractive index changes could be detected from the light scattering when using an automated bead sorter as described below; or by using a custom-made instrument based on the principles of surface plasmon resonance or (SPR).

It is preferred that at least one protein mixture is labelled using a fluorescent label, it is even more preferred that all protein mixtures are labelled using different fluorescent labels.

### Isolation

Bound protein-ligand complexes or pairs can be isolated from the bulk of the ligand library by various means depending on the nature of the detection probe. Isolation methods include for example, manually sorting beads containing bound labeled protein by detecting the detection probe for example with the aid of a microscope or sorting by fluorescence or by color depending on the screening process used. Alternatively, the sorting process may be automated with the use of a beads sorter, such as by use of "fluorescence activated beads sorting" (FABS), for example specially designed, commercially available bead sorters may be used (e.g. Union Biometrica, Sommerville, Mass.) and detecting fluorescence intensity (Meldal, 2002, Biopolymers, 66: 93-100). In general, resin beads can be sorted at a rate of about 100 to 200 beads per second, or even faster depending on the equipment used and its reading capacity. A range of about 5 to 500, such as 5 to 110, preferably about 5 to 50 beads per second is sorted with known instruments. Slower rates may be used to increase accuracy. Preferred, is a rate where reading for example, only one resin bead passes through the detector at a time.

### Process for the Identification of Protein and Ligand Binding Partners

The protein and ligand binding partners may be identified using any conventional technique known to the person skilled in the art, for example any of the techniques described herein below. It is preferred that the resin bead comprising the ligand-protein binding pair is isolated and identifying the ligand and/or identifying the protein is carried out on the isolated resin bead. It is thus preferred that either the ligand or the protein or more pref erably both are identified using "on-bead" mehods. "On-bead" refers to methods wherein the identification process or part thereof is performed directly on a bead, for example methods
wherein the ligand and/or protein are identified on the bead by for example spectroscopy or to methods wherein the ligand and/or protein is enzymatically digested directly on the bead. In a preferred embodiment of the invention, identification of protein and ligand binding partners is identification from the protein/ligand complex on same, single bead. In this embodiment, beads comprising polyethylene glycol, preferably PEG-based resins with a size in the range of 500 - 800 □m is used. In one embodiment, a resin bead containing the binding pair is cut into two unequal portions. One portion of the bead is used to identify the ligand, while the other portion is used to identify the protein. In another embodiment, the protein in first broken down into its constitutive peptides enzymatically or chemically (vide infra) and the ligand then released. Both the ligand and the protein peptides are simultaneously analysed by mass spectrometry (vide infra). In this embodiment, the ligand may be first analysed by NMR (vide infra) before break down of the protein and release of the ligand. In one particular embodiment, especially for NMR analysis the ligand is linked to the solid support via a methionine residue and the protein and ligand can be simultaneously broken down and released by treatment with CNBr.

### Identification of Ligands

After detection and isolation of the protein-ligand complexes, the ligand can be identified. The process for identification of the ligand depends on the type of library used. For example the ligand may be identified using mass spectrometry, NMR spectroscopy, infrared (IR), elemental analysis or combinations of the aforementioned.

In one embodiment of the invention it is preferred that the ligand is identified using "on-bead" methods (see herein above and below).

For a library of primarily oligomeric compounds, the complexed ligand can be analyzed by Mass Spectroscopy (MS), particularly if the library was synthesized in such a way that the synthetic history of the compound is captured, for example, using a capping procedure to generate fragments of the compound that differ in mass by one building block (see, for example, Youngquist et al., 1995, J. Am Chem. Soc., 117: 3900-06). This capping procedure is most efficient when the cap and the building block are reacted at the same time. The capping agent can be any class of compound that has at least one functional group in common with the building block used to generate the oligomer, so that both the capping agent and the building block can react when added to the resin in an appropriate ratio. Alternatively, the capping agent can have two functional groups in common with the building block where one of the groups in common, such as the group in the building block that is used for the elongation of the oligomer, is orthogonally protected. For example, in a synthesis of a peptide using the Fmoc strategy shown in the examples below, the capping agent could be the same as the building block but with a Boc group protecting the reactive amine instead of the Fmoc group (see Examples 5, 6, and 7 and St. Hilaire et al., 1998, J. Am. Chem. Soc., 120: 13312-13320). In another example, if the building block is a protected haloamine, the capping agent could be the corresponding alkylhalide.

Where the ligand library is synthesized by parallel synthesis (a parallel array), the binding ligand can be identified simply by the knowledge of what specific reaction components were reacted in a particular compartment. The structure can be confirmed by cleavage of a small portion of compound from the solid support and analyzed using routine analytical chemistry methods such as infrared (IR), nuclear magnetic resonance (NMR), mass spectroscopy (MS), and elemental analysis. For a description of various analytical methods useful in combinatorial chemistry, see: Fitch, 1998-99, Mol. Divers., 4: 39-45; and Analytical Techniques in Combinatorial Chemistry, M.E. Swartz (Ed), 2000, Marcel Dekker: New York.

In the case of libraries synthesized by the split-mix approach where the precise structure of the compound is unknown, the complexed ligand can be identified using a variety of methods. The compound may be cleaved off the solid support, for example, resin bead, and then analyzed using IR, MS, or NMR. For NMR analysis, larger beads containing approximately 5 nmoles of material can be used for the acquisition of 1-dimensional (1-D) and 2-dimensional (2-D) NMR spectra. Furthermore, these spectra can be attained using high-resolution MAS NMR techniques. Alternatively, high resolution-MAS NMR spectra can be acquired while the ligand is still bound to the solid support, as described for example, in Gotfredsen et al., 2000, J. Chem. Soc., Perkin Trans., 1: 1167-71. Thus in one preferred embodiment of the invention, the ligand is identified using High resolution NMR. Preferably the resin used in this embodiment is a resin comprising polyethylene glycol, for example PEG-Based resins like PEGA, SPOCC and POEPOP.

Typically, resin beads used for library synthesis contain about 100 to 500 pmoles of material, which is generally insufficient for direct analysis using NMR techniques. In such situations, the ligand libraries can be synthesized with special encoding to facilitate identification of the ligand For a review of encoding strategies employed in combinatorial chemistry see: Barnes et al., 2000, Curr. Opin. Chem. Biol., 4: 346-50. Most coding strategies include the parallel synthesis of the encoding molecule (for example, DNA, PNA, or peptide) along with the library compounds. This strategy is not preferred, as it requires a well-planned, time consuming, orthogonal protecting group scheme. Furthermore, the encoding molecule itself can sometimes interact with the protein receptor leading to false positives. Alternatively, the ligand library members can be encoded using radiofrequency tags. This method alleviates the problem of false positives stemming from the coding tags, but is generally only useful for small ligand libraries in the one-bead-one-compound system due to the sheer bulk of the radiofrequency tag. Alternatively, single beads can be analyzed in a non-destructive manner using infrared imaging. However, this method gives limited information and while useful for pre-screening, is not recommended for conclusive structural determination. MS can be used alone to identify the ligand library member. The ligand can be cleaved from the solid support, the molecular mass determined, and subsequently fragmented into sub-species to conclusively determine the structure. MS-based methods of ligand identification are useful in this invention, as they require very little material, and can utilize pico- to femtomole amounts of compound.

A combination of both High resolution-NMR and mass spectrometry can also be used to identify the ligands in this invention.

### Isolation and Identification of Binding Protein Member

The binding protein may be identified using any conventional method known to the person skilled in the art. For example, the protein may be extracted from beads and identified by for example gel electrophoresis, such as 2D gel electrophoresis, mass spectrometry, such as MALDI-TOF-MS, NMR, peptide sequencing, for example by Edman degradation, peptide mass fingerprinting or any other suitable method. It is however generally preferred that the protein is identified using "on-bead" methods (see herein above and below).

In general, once the binding ligand member has been identified and isolated with its bound protein, the binding protein member can be identified. However, in some embodiments of the invention the protein binding member may be identified prior to the ligand or they may be identified simultaneously.

In one embodiment, a resin bead containing the binding pair is cut into two portions. One portion of the bead is used to identify the ligand, while the other portion is used to identify the protein. This can be accomplished, for example, by performing systematic degradation of the protein on-bead. Most often, the protein can be broken down into its constituent peptides enzymatically, for example using a protease, such as trypsin or other known peptidases. General protocols for enzymatic breakdown of proteins during proteomic analysis can be found, for example, in 2-D Proteome Analysis Protocols, A.J. Link (Ed), 1st Ed, 1999, Humana Pr: Totowa. Given the hydrophilic nature of the resins, trypsin works efficiently on-bead, and can efficiently cleave native proteins as well as proteins that have been covalently modified with a detection probe. The number of reasonably sized peptides generated by enzymatic cleavage is improved if the proteins are first denatured. Denaturation is easily accomplished on-bead, for example, on PEG-based resins that are robust and solvated in most denaturants used, such as guanidine HCl and urea. Otherwise denaturation may be obtained by drastic changes in temperature and pH. Other cleavage enzymes may be used, for example, endoprotease Arg-C, endoprotease Lys-C, clrymotrypsin, endoprotease Asp-N, and endoprotease Glu-C. Sometimes, chemicals such as CNBr (as described, for example, in Compagnini et al., 2001, Proteomics, 1: 967-74) and [*cis*-Pd(en)(H20)₂]²⁺ (as described, for example, in Milovic et al., 2002, J. Am. Chem. Soc., 124: 4759-69) may be used to degrade a protein into its constituent peptides.

Alternatively, the identified ligand can be resynthesized and coupled to an affinity support such as sepharose or sephacryl, and the protein member purified by affinity chromatography. Unlabelled protein mixture is applied to the affinity column and, after washing of the unbound protein, bound protein is eluted with solubilized ligand. This route is time and reagent consuming. The ligand must first be synthesized and purified, and then attached to the affinity support. It should also be produced in sufficient quantities that the required concentration can be used to elute protein from the affinity column. Alternatively, buffers of different pH, high salt and/or denaturants can be used to elute protein. It can sometimes be difficult to elute multimeric proteins from affinity columns using a monovalent ligand because of avidity effects.

To expedite the process and alleviate the aforementioned problems, the protein can be degraded into peptides while still bound to its ligand-binding partner, and the generated peptides analyzed. For example, the ligand is resynthesized on small scale (25-50 beads) on a useful resin, preferably the same resin used for library synthesis, such as PEGA4000 resin or PEGA6000 resin,. After binding of unlabelled protein from the mixture and washing off the unbound protein, the protein-ligand complex can be immediately degraded into the constituent peptides either enzymatically or chemically, using known processes and reagents and the peptides analyzed, for example, by peptide mass fingerprinting, or other known methods. Using this process several ligand-protein complexes can rapidly be digested. This process can be readily automated.

The protein bound to the ligand can be identified by any suitable method such as MS or Edman degradation sequencing. For general protocols on the identification of proteins using proteomics techniques, see, for example, 2-D Proteome Analysis Protocols, A.J. Link (Ed), 1st Ed, 1999, Humana Pr: Totowa. Protein can be identified from its peptide mass fingerprint, for example, using the mass of some of the constituent peptides obtained from enzymatic digests. The mass of the mixture of peptides generated from the digested proteins can be determined using MALDI-TOF-MS or ES-MS. The peptide masses or fingerprints are used to search databases of known proteins and gene products to identify the protein(s). To increase accuracy of the protein identification in the absence of other limiting information such as pI and mass, the results of several digests using different processes for cleavage are combined. Instead of, or in addition to, generating peptide fingerprints, a single peptide from the protein can be fragmented, and its amino acid sequence determined. The sequence can be used to identify known and unknown proteins, for example, by comparing to protein databases. The use of MS to identify the proteins(s) is well suited to the degradation of protein complexes on single beads, since very little material is required for identification (pico - femtomole). Alternatively, proteins can be identified using N-terminal sequencing via Edman degradation; provided that the N- terminus is not blocked. This generally requires larger quantities of material (picomole).

### Ligand and proteins

It is also an objective of the present invention to provide ligands, proteins and ligand/protein binding pair identified by the methods according to the invention.

In one embodiment of the present invention the ligand is a potential drug candidate. The ligand may for example be a drug candidate for treatment of a neoplastic or preneoplastic disease, an autoimmune disease, an infectious disease, a cardiovascular disease, CNS disorders, metabolic diseases, endocrine diseases or an inflammatory disease.

The ligands may be the ligands directly identified using the invention or functional homologues thereof. By the term "functional homologue" is meant a molecule preferably structurally similar, that is capable of specifically associating with the same protein(s). Preferably, "functional homologues" are structural homologues. Preferably, ligands according to the invention are isolated, more preferably isolated and purified ligands.

Hence, in one embodiment the ligands according to the present invention may be selected from the group consisting of ligands comprising or more preferably consisting of Pip-Pal-Pal-Phe-Pya-Pip [SEQ ID NO: 7]; Pya-Hyp-Hyp-Phe-Acm-Tyr [SEQ ID NO: 8]; Pya-Gua-Pip-Acc-Phe-Pip [SEQ ID NO: 9]; Phe-Aze-Gly-His-Gly-Aze [SEQ ID NO: 10]; Phe-Thr-Pya-Pip-Asp-His [SEQ ID NO: 11]; Phe-Ppy-Acc-Ala-Ppy-Hpy [SEQ ID NO: 12]; Phe-Thr-Tyr-Phe-Ala-Lys [SEQ ID NO: 51]; His-Tyr-Pip-Thr-Acm-Abi [SEQ ID NO: 52]; Tyr-Pip-Thr-Acm-Aze-His [SEQ ID NO: 53]; Phe-Phe-Phe-Pip-Aze-Gua [SEQ ID NO: 54]; Phe-Gua-Asp-Abi-His-Aze [SEQ ID NO: 55]; Flte-Abi-Pal-Hyp-Thr-Hyp [SEQ ID NO: 65]; Phe-Gua-Pal-Tyr-Gua-Tyr [SEQ ID NO: 66]; Pal-Abi-Gly-Gly-Abi-His [SEQ ID NO: 67]; Abi-Thr-Hyp-Hyp-His--[SEQ ID NO: 68]; Pya-Gua-Abi-Asp-Abi-Tyr [SEQ ID NO: 69]; Abi-Phe-Abi-Phe-Che-Tyr [SEQ ID NO: 18]; Pal-Gly-Abi-Hyp-Pya-Trp [SEQ ID NO: 56]; Lys-Met-Hyp-Trp-Tyr-Gua [SEQ ID NO: 57]; Phe-Asp-Trp-Gua-Thr-Gua [SEQ ID NO: 58]; T(Sa)-F-N-H-S [SEQ ID NO: 19]; T(Sa)-F-A-L-V [SEQ ID NO: 20]; T(Sa)-F-G-I-W [SEQ ID NO: 21]; T(Sa)-F-G-I-M [SEQ ID NO: 22]; T(Sa)-G-V-F-L [SEQ ID NO: 23]; T(Sa)-Y-S-M-P [SEQ ID NO: 24]; T(Sa)-L-S-W-W [SEQ ID NO: 25]; T(Sa)-H-W-H-I [SEQ ID NO: 26]; T(Sa)-H-W-V-V [SEQ ID NO: 27]; T(Sa)-H-L-G-Y [SEQ ID NO: 28]; T(Sa)-I-Y-L-F [SEQ ID NO: 29]; T(Sa)-F-G-L-M [SEQ ID NO: 30]; T(Sa)-W-V-N-M [SEQ ID NO: 31]; T(Sa)-M-V-N-W [SEQ ID NO: 32]; T(Sa)-H-I-G-Y [SEQ ID NO: 33]; T(Sa)-L-Y-L-F [SEQ ID NO: 34]; T(Sa)-H-W-H-L [SEQ ID NO: 35]; T(Sa)-F-V-W-H [SEQ ID NO: 36]; T(Sa)-Y-G-A-M [SEQ ID NO: 59]; T(Sa)-L-Y-I-F [SEQ ID NO: 37]; T(Sa)-S-V-W-F [SEQ ID NO: 60]; T(Sa)-H-Y-F-F [SEQ ID NO: 61]; T(Sa)-I-Y-Y-F [SEQ ID NO: 62]; T(Sa)-Q-P-G-M [SEQ ID NO: 63]; T(Sa)-G-P-H-G [SEQ ID NO: 64]; ManS-Gly-ManS-Asp-Asn-Ala [SEQ ID NO: 38]; ManS-Gly-GlcNN-Asn-ManS-Tyr [SEQ ID NO: 39]; ManN-Phe-Trp-Ser-Lys-His [SEQ ID NO: 40]; GlcNN-Trp-Phe-Asp-Trp-Pro [SEQ ID NO: 41]; GleNN-Val-GicNN-His-ManS-Gly [SEQ ID NO: 42]; ManN-ManS-ManN-Trp-Ser-Trp [SEQ ID NO: 43]; Gly-Pro-Lys-Lys-Tyr-His [SEQ ID NO: 44]; or His-Thr-Trp-Gly-Tyr-Trp [SEQ ID NO: 45]; or functional homologues thereof.

Functional homologues are preferably structurally related compound capable of interacting with the same protein(s). Preferably, functional homologues comprises 1, such as 2, for example 3 substitutions, preferably one substitution of one monomer for another, preferably substitution of one amino acid for another. Preferably said substitution is a conservative substitution.

Hence, the present invention relates to a ligand comprising or consisting of Pip-Pal-Pal-Phe-Pya-Pip [SEQ ID NO: 7] as well as to an isolated ligand-protein binding pair comprising said ligand and Ca2+/Calmodulin activated Myosin light chain kinase, Regulator of G-Protein Signalling (RGS14) variant, ATP Synthase component (subunit e), Cytochrome P450, Ribosomal proteins (60s) or SPTR.

The present invention also relates to a ligand comprising or consisting of Pya-Hyp-Hyp-Phe-Acm-Tyr [SEQ ID NO: 8] as well as to an isolated ligand-protein binding pair comprising said ligand and Troponin T, Ca2+/Calmodulin activated Myosin light chain kinase or eGMP-dependent protein kinase..

The present invention also relates to a ligand comprising or consisting of Pya-Gua-Pip-Acc-Phe-Pip [SEQ ID NO: 9] as well as to an isolated ligand-protein binding pair comprising said ligand and NADH dehydrogenase, ATP binding component, Myosin, or Histone associated protein.

The present invention also relates to a ligand comprising or consisting of Phe-Aze-Gly-His-Gly-Aze [SEQ ID NO: 10] as well as to an isolated ligand-protein binding pair comprising said ligand and Hypothetical proteins (gi 20474763); Cysteine and tyrosine rich proteins of unknown function, Mitochondrial ATP synthase; Ribosomal proteins (60s L series) or SPTR.

The present invention also relates to a ligand comprising or consisting of Phe-Thr-Pya-Pip-Asp-His [SEQ ID NO: 11] as well as to an isolated ligand-protein binding pair comprising said ligand and Sodium channel (gi 18591322), Chloride channel (gi 6978663/4502867), Troponin I (gi 1351298); Zn Finger protein (gi 18591322); SPTR (peroxisomal Ca dependent solute carrier (putative) (gi 12853685); Beta-2 adnergic receptor (gi 12699028).

The present invention also relates to a ligand comprising or consisting of Phe-Ppy-Acc-Ala-Ppy-Hpy [SEQ ID NO: 12] as well as to an isolated ligand-protein binding pair comprising said ligand and Troponin T, Phospholipase C, or Phosphatidylcholine sterol acyl transferase.

The present invention also relates to a ligand comprising or consisting of Phe-Thr-Tyr-Phe-Ala-Lys [SEQ ID NO: 13] as well as to an isolated ligand-protein binding pair comprising said ligand and Serine/threonine Protein kinase (gi 5730055); Carbonic anhydrase VII (gi10304383).

The present invention also relates to a ligand comprising or consisting His-Tyr-Pip-Thr-Acm-Abi [SEQ ID NO: 14] as well as to an isolated ligand-protein binding pair comprising said ligand and Chain C P27 cyclin A-CDK2 complex (gi2392395); Hypothetical protein XP_154035.

The present invention also relates to a ligand comprising or consisting of Phe-Abi-Pal-Hyp-Thr-Hyp [SEQ ID NO: 65] as well as to an isolated ligand-protein binding pair comprising said ligand and Zinc finger associated protein, Ribosomal proteins, or Protein phosphatase.

The present invention also relates to a ligand comprising or consisting of Phe-Gua-Pal-Tyr-Gua-Tyr [SEQ ID NO: 66] as well as to an isolated ligand-protein binding pair comprising said ligand and Glucose-6-Phosphatase, Succinate dehydrogenase, ARL-interacting protein, SPTR, or Nucleic acid binding protein.

The present invention also relates to a ligand comprising or consisting of Pal-Abi-Gly-Gly-Abi-His [SEQ ID NO: 67] as well as to an isolated ligand-protein binding pair comprising said ligand and 60s Ribosomal protein, 40s Ribosomal protein, or Low density lipoprotein receptor.

The present invention also relates to a ligand comprising or consisting of Abi-Thr-Hyp-Hyp-His- [SEQ ID NO: 68] as well as to an isolated ligand-protein binding pair comprising said ligand and Phosphofructokinase, Selenium binding protein, Serine arginine rich protein kinase, Guanylate kinase, Protein tyrosine kinase, Alkaline phosphatase, Symporter, SPTR, WAP-protein, GTP Hydrolase, or Actin filament.

The present invention A ligand comprising or consisting of Pya-Gua-Abi-Asp-Abi-Tyr [SEQ ID NO: 69] as well as to an isolated ligand-protein binding pair comprising said ligand and SPTR (gi 20869775); Ribosomal proteins (60s) (gi 20875941/6677773); (Calcium channel (gi 3202010); Slo channel protein isoform (gi 3644046) ; Potassium conductance calcium activated channel (gi 6754436,NP_034740); Regulator of G-protein signalling 8(gi 9507049).

The present invention also relates to a ligand comprising or consisting of Abi-Phe-Abi-Phe-Che-Tyr [SEQ ID NO: 18] as well as to an isolated ligand-protein binding pair comprising said ligand and Cathepsin E, Ribosomal protein, Actin binding protein, or Amino acid transferase.

The present invention also relates to a ligand comprising or consisting of T(Sa)-F-N-H-S [SEQ ID NO: 19] as well as to an isolated ligand-protein binding pair comprising said ligand and Phosphate acetyl transferase, acid shock protein, or molybdopterin converting factor subunit.

The present invention also relates to a ligand comprising or consisting of T(Sa)-F-A-L-V [SEQ ID NO: 20] as well as to an isolated ligand-protein binding pair comprising said ligand and Chaperone DnaK or transposase.

The present invention also relates to a ligand comprising or consisting of T(Sa)-F-G-I-W [SEQ ID NO: 21] as well as to an isolated ligand-protein binding pair comprising said ligand and Histidine synthetase or aspartate carbamoyl transferase.

The present invention also relates to a ligand comprising or consisting of T(Sa)-F-G-I-M [SEQ ID NO: 22] as well as to an isolated ligand-protein binding pair comprising said ligand and Transposase, transcriptional regulator, or GroEL.

The present invention also relates to a ligand comprising or consisting of T(Sa)-G-V-F-L [SEQ ID NO: 23] as well as to an isolated ligand-protein binding pair comprising said ligand and 50 S ribosomal protein, heme binding lipoprotein, regulator for D-glucarate, D-glycerate and D-galactarate, or glutamine tRNA synthetase.

The present invention also relates to a ligand comprising or consisting of T(Sa)-Y-S-M-P [SEQ ID NO: 24] as well as to an isolated ligand-protein binding pair comprising said ligand and Biotin synthetase, UDP-glucose dehydrogenase, tyrosine protein kinase, or fatty acid oxidase complex proteins.

The present invention also relates to a ligand comprising or consisting of T(Sa)-L-S-W-W [SEQ ID NO: 25] as well as to an isolated ligand-protein binding pair comprising said ligand and NAD-dependent 7-alpha-hydroxysteriod dehydrogenase, homocysteine transferase, nitrate reductase, lactate dehydrogenase, or citrate synthetase.

The present invention also relates to a ligand comprising or consisting of T(Sa)-H-W-H-I [SEQ ID NO: 26] as well as to an isolated ligand-protein binding pair comprising said ligand and Mannose-1-phasphate guanyl transferase or isopropyl malate dehydrogenase.

The present invention also relates to a ligand comprising or consisting of T(Sa)-H-W-V-V [SEQ ID NO: 27] as well as to an isolated ligand-protein binding pair comprising said ligand Pyruvoyl dependent aspartate decarboxylase, colicin E2, or Histidine kinase.

The ligand comprising or consisting of T(Sa)-H-L-G-Y [SEQ ID NO: 28] as well as an isolated ligand-protein binding pair comprising said ligand and phosphomannose isomerase.

The present invention also relates to a ligand comprising or consisting of T(Sa)-I-Y-L-F [SEQ ID NO: 29] as well as to an isolated ligand-protein binding pair comprising said ligand and Membrane bound ATP synthetase or ATP hydrolase.

The present invention also relates to a ligand comprising or consisting of T(Sa)-F-G-L-M [SEQ ID NO: 30] as well as to an isolated ligand-protein binding pair comprisingsaid ligand and Hemolysin C, high affinity potassium transport system, quinone oxidoreductase, or ferrodoxin dependent NA(D)PH oxidoreductase.

The present invention also relates to a ligand comprising or consisting ofT(Sa)-W-V-N-M [SEQ ID NO: 31] as well as to an isolated ligand-protein binding pair comprising said ligand and Transposase or inner membrane protein for phage attachment.

The present invention also relates to a ligand comprising or consisting of T(Sa)-M-V-N-W [SEQ ID NO: 32] as well as to an isolated ligand-protein binding pair comprising said ligand and ATP dependent helicase or mob C.

The present invention also relagtes to a ligand comprising or consisting of T(Sa)-H-I-G-Y [SEQ ID NO: 33] as well as to a ligand-protein binding pair comprising said ligand and Fimbrial subunit or outer membrane pyruvate kinase.

The present invention also relates to a ligand comprising or consisting of T(Sa)-L-Y-L-F [SEQ ID NO: 34] as well as to an isolated ligand-protein binding pair comprising said ligand and Fimbrial protein precursor, alkaline phosphatase, cytochrome related proteins.

The present invention also relates to a ligand comprising or consisting of T(Sa)-H-W-H-L [SEQ ID NO: 35] as well as to an isolated ligand-protein binding pair comprising said ligand and Chorismate mutase, xanthine dehydrogenase, or carbamoyl phosphate synthetase.

The present invention also relates to a ligand comprising or consisting of T(Sa)-F-V-W-H [SEQ ID NO: 36] as well as to an isolated ligand-protein binding pair comprising said ligand and NADH dehydrogenase, protein involved in flagellar biosynthesis and motor switching component, Lysine-arginine-ornithine-binding protein, or ATP-binding component of glycine-betaine-proline transport protein.

The present invention also relates to a ligand comprising or consisting of T(Sa)-L-Y-I-F [SEQ ID NO: 37] as well as to an isolated ligand-protein binding pair comprising said ligand and Colicin, outer membrane lipoprotein, or arylsulfatase.

The present invention also relates to a ligand comprising or consisting of ManS-Gly-ManS-Asp-Asn-Ala [SEQ ID NO: 38] as well as to an isolated ligand-protein binding pair comprising said ligand and Con A or *P. sativum* lectin.

The present invention also relates to a ligand comprising or consisting of ManS-Gly-GlcNN-Asn-ManS-Tyr [SEQ ID NO: 39] as well as to an isolated ligand-protein binding pair comprising said ligand and Con A, *P. sativum* lectin, or *L. culinaris* lectin.

The present invention also relates to a ligand comprising or consisting of ManN-Phe-Trp-Ser-Lys-His [SEQ ID NO: 40] as well as to an isolated ligand-protein binding pair comprising said ligand and Con A or *P. sativum* lectin.

The present invention also relates to a ligand comprising or consisting of GlcNN-Trp-Phe-Asp-Trp-Pro [SEQ ID NO: 41] as well as to an isolated ligand-protein binding pair comprising said ligand and Con A.

The present invention also relates to a ligand comprising or consisting of GlcNN-Val-GlcNN-His-ManS-Gly [SEQ ID NO: 42] as well as to an isolated ligand-protein binding pair comprising said ligand and Con A or P. *sativum* lectin.

The present invention also relates to a ligand comprising or consisting of ManN-ManS-ManN-Trp-Ser-Trp [SEQ ID NO: 43] as well as to an isolated ligand-protein binding pair comprising said ligand and Con A, *P. sativum* lectin, or *L. culinaris* lectin.

The present invention also relates to a ligand comprising or consisting of Gly-Pro-Lys-Lys-Tyr-His [SEQ ID NO: 44] as well as to an isolated ligand-protein binding pair comprising said ligand and Con A, *P. sativum* lectin, or *L. culinaris* lectin.

The present invention also relates to a ligand comprising or consisting of His-Thr-Trp-Gly-Tyr-Trp [SEQ ID NO: 45] as well as to an isolated ligand-protein binding pair comprising said ligand and Con A.

The present invention also relates to a ligand comprising or consisting of Pal-Gly-Abi-Hyp-Pya-Trp [SEQ ID NO: 56] as well as to an isolated ligand-protein binding pair comprising said ligand and NAS putative unclassified (gi 12861084) or Putative Zn finger protein 64 (gi 12849329).

The present invention also relates to ligands of the formula I,

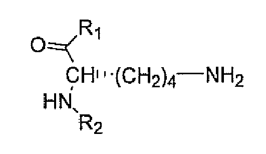

Such ligands are generally referred to as "AJn", wherein n is an integer. Each integer n, identifies a specific R₁ and R₂ group, see for example table 10. R₁ and R₃ may individually be selected from any suitable substituent, for example alkyl, alken, alkyn, aryl, amines, amino acids, ketones, esters, carboxylic acids, alcohols, thio-alcohols, aldehydes, aromatic and non-aromatic ringsystems optionally comprising one or more heteroatoms, wherein each of the aforementioned may be substituted with one or more groups, for example, halogen, carbonyl, amino, methoxy, ethoxy and/or alkoxy. Preferably, R₁ and R₂ may individually be selected from the group consisting of any of the compounds mentioned in tables 1, 2, 3, 7, 8 and 9, more preferably R₁ is an amine and R₂ is a ketone, even more preferably R₁ is selected from the group consisting of the compounds numbered 3, 4, 7, 8, 10, 15, 60, 61 and 62 in said tables and R2 is selected from the group consisting of the compounds numbered 56, 57, 58 and 59 in said tables. Yet more preferably ligands according to formula I are selected from the group consisting of the identified ligands mentioned in table 10 herein below, even more preferably said ligands are selected from the group consisting of compounds AJ10, AJ18, AJ 19 and AJ15, outlined in table 10.

Hence, in one embodiment of the invention the ligand is AJ10, in another embodiment of the invention the ligand is AJ18, in yet another embodiment of the invention the ligand is AJ19, in a still further embodiment of the invention the ligand is AJ15.

The present invention also relates to isolated ligand-protein binding pairs comprising a ligand of formula I. Preferably said ligand-protein binding pair is selected from the group consisting of the identified ligands and the corresponding identified proteins as outlined in table 10 herein below.

In a preferred embodiment of the invention the ligand-protein binding pair consists of:
- Ligand AJ10 of table 10; and
- Angiotensin converting enzyme (P22967) or U2 nuclear ribonucleoprotein auxilary factor (gi 2842676). In another preferred embodiment of the invention the ligand-protein binding pair consists of:

- Ligand AJ18 8 of table 10; and
- Inward rectifier potassium channel 13 (Q9QZ65, IRKD_CAVPO); Sulfonylurea receptor 2 (Q63563); or small conductance potassium channel (P58391) In another preferred embodiment of the invention the ligand-protein binding pair consists of:

- Ligand AJ19 of table 10; and
- Heat Shock protein 70kDa protein 12A (Q8KOU4); Serine/threonine protein kinase (088866) or Ras GTPase activating protein 2 (GAPlm) (P58069) In another preferred embodiment of the invention the ligand-protein binding pair consists of:

- Ligand AJ15 of table 10; and
- Glycogen synthase kinase-3 beta factor (AQ9WV60).

### The present invention also relates to ligand of formula II:

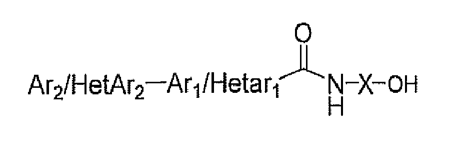

Such ligands are generally referred to as "MWn", wherein n is an integer. Each integer n, identifies specific X, Ar₁/Hetar₁ and Ar₂/Hetar₂ groups, see for example table 11 and 12. X is a side chain of a natural or unnatural amino acid, preferably any of the amino acids indicated in tables 1, 2, 3 and 7. More preferably, X is selected from the group consisting of side chains of the compound 3 to 47, 64 to 75 and 106 as indicated in tables 1, 2, 3 and 7 herein below. Ar₁/Hetar₁ is an aryl or heteroaryl group from a haloacid. Preferably Ar₁/Hetar₁ is selected from the group consisting of the compounds 76 to 81 as indicated in table 7 herein below. Ar₂/Hetar₂ is an aryl or heteroaryl, group from a boronic ester. Preferably Ar₂/Hetar₂ is selected from the group consisting of compounds 82 to 101 as indicated in table 8 herein below. More preferably, the ligand of formula II is selected from the group of ligand identified in tables 11 and 12 herein below, even more preferably the ligand of formula II is MW1 or MW2 or MW14 or MW15 or MW16 or MW20 or MW21 or MW22 or MW23 or MW37 as identified in tables 11 and 12 herein below. The present invention also relates to isolated ligand-protein binding pairs comprising a ligand of formula II. Preferably said ligand-protein binding pair is selected from the group consisting of the identified ligands and the corresponding identified proteins as outlined in tables 11 and 12 herein below. In a preferred embodiment of the invention the ligand-protein binding pair consists of:
- Ligand MW1 of table 11; and
- APK1 antigen (2134769) or Hyaluronan synthase 2 (gi12049586) In another preferred embodiment of the invention the ligand-protein binding pair consists of:

- Ligand MW2 of table 11; and
- APK1 antigen (2134769) or Hyaluronan synthase 2 (gi12049586) In another preferred embodiment of the invention the ligand-protein binding pair consists of:

- Ligand MW14 of table 11; and
- Unnamed protein product (26336134); Serine/threonine kinase 9/cylin dependent Serine/threonine kinase 9(23271297/4507281); Caveolin 1(gi 4972627/AAD347221) or (V) Envelope glycoprotein (gi 3832399) In another preferred embodiment of the invention the ligand-protein binding pair consists of:

- Ligand MW 15 of table 11; and
- Unnamed protein product (26336134); Serine/threonine kinase 9/cylin dependent Serine/threonine kinase 9(2327129714507281); Caveolin 1(gi 4972627/AAD347221) or (V) Envelope glycoprotein (gi 3832399) In another preferred embodiment of the invention the ligand-protein binding pair consists of:

- Ligand MW16 of table 11; and
- 7 Transmembrane helix receptor (gi 21928697); DEAD (Asp-Glu-Ala-Asp) box polypeptide. (gi 19527256); 60s ribosomal protein L12 mito precursor (gi 1710600); (V) HLA-binding protein (gi 1279435) or Large subunit of ribonucleotide reductase (gi 30984467) In another preferred embodiment of the invention the ligand-protein binding pair consists of;
- Ligand MW20 of table 12; and
- Cytochrome P450 2C23 (Arachidonic acid epoxygenase) (CYPIIC23, P24470); Splice isoform Calcitonin, variant displaced from P41547 Calcitonin precursor (CAL0_CANFA, P22892) or Adaptor protein complex AP-1 gamma-1 subunit with Golgi adaptor HA1/AP1 adaptin gamma-1 subunit) (P41547-00-00-00). In another preferred embodiment of the invention the ligand-protein binding pair consists of:

- Ligand MW21 of table 12; and
- Cytochrome P450 2C23 (Arachidonic acid epoxygenase) (CYPIIC23, P24470); Splice isoform Calcitonin, variant displaced from P41547 Calcitonin precursor (CALO_CANFA, P22892) or Adaptor protein complex AP-1 gamma-1 subunit with Golgi adaptor HA1/AP1 adaptin gamma-1 subunit) (P41547-00-00-00). In another preferred embodiment of the invention the ligand-protein binding pair consists of:

- Ligand MW22 of table 12; and
- Cytochrome P450 2C23 (Arachidonic acid epoxygenase) (CYPIIC23, P24470); Splice isoform Calcitonin, variant displaced from P41547 Calcitonin precursor (CALO_CANFA, P22892) or Adaptor protein complex AP-1 gamma-1 subunit with Golgi adaptor HA1/AP1 adaptin gamma-1 subunit) (P41547-00-00-00).

In another preferred embodiment of the invention the ligand-protein binding pair consists of:
- Ligand MW23 of table 12; and
- Zinc finger protein 198 (Fused in myeloproliferative disorders protein) (Q9UbW7) or Unnamed protein product (gi 12844245). In another preferred embodiment of the invention the ligand-protein binding pair consists of:

- Ligand MW37 oftable 12; and
- Serine-threonine kinase (gi 474842); Hemopexin precursor (Beta-1B-glycoprotein) (P02790) or 116 kDa U5 small nuclear ribonucleoprotein component (008810).

The present invention also relates to ligands of formulas IIIa), IIIb), IIIc), IIId), IIIe) or IIIf):

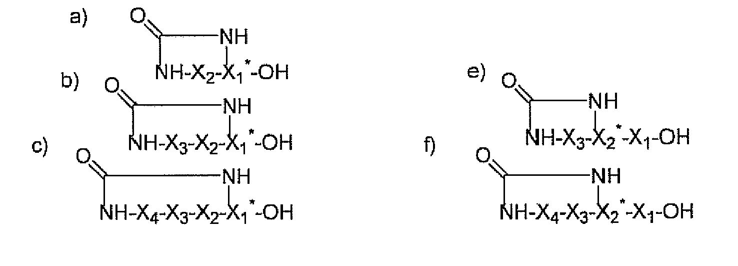

Such ligands are generally referred to as "HYn", wherein n is an integer. Each integer n, identifies specific X*, X₁, X₂, X₃ and X₄ groups, see for example table 13. X₁, X₂, X₃ and X₄ are individually selected from the group consisting of natural and unnatural amino acids. Preferably, X₁, X₂, X₃ and X₄ may individually be selected from the group consisting of amino acids mentioned in tables 1, 2, 3, 7 and 9, more preferably X₁, X₂, X₃ and X₄ may individually be selected from the group consisting of compounds 3, 4, 5, 7, 9, 12, 13, 15, 17, 19 to 22, 24, 28, 45, 47, 73 106 and 127 as outlined in tables 1, 2, 3, 7 and 9 herein below. X* is a natural or unnatural amino acid, preferably any of the amino acids mentioned in tables 1, 2, 3, 7 and 9 herein below, even more preferably X* is selected from the group consisting of compounds 8, 39, 40, 41 and 43 as outlined in tables I and 3 herein below. Preferred ligands of formulas IIIa), IIIb), IIIc), IIId), IIIe) or IIIf) are HY1 or HY2 indicated in table 13 herein below.

The present invention also relates to isolated ligand-protein binding pairs comprising a ligand of any of formulas IIIa), IIIb), IIIc), IIId), IIIe) or IIIf). In a preferred embodiment of the invention the ligand-protein binding pair consists of:
- Ligand HY1 of table 13; and
- Protein kinase C binding protein 1 also contains a zinc finger domain with protein 8) (Q9U1U4) or Guanine nucleotide-binding protein G(S), alpha subunit (Adenylate cyclase-stimulating G alpha protein) (P048949). In another preferred embodiment of the invention the ligand-protein binding pair consists of:
- Ligand HY2 of table 13; and
- Protein kinase byr2 (Protein kinase ste8) (MAP kinase) (P28829); Similar to protein kinase 2 (Testicular) (Q885R6) or Thiamine-triphosphatase (Q8CgV7).

### The present invention also relates to ligands according to formula IV:

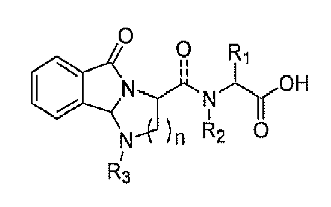

Such ligands are generally referred to as "HYn", wherein n is an integer. Each integer n, identifies specific R₁, R₂ and R₃ groups, see for example table 14. R₁ is a side chains of a natural or unnatural amino acid, preferably the side chain of an amino acid selected from the group consisting of amino acids mentioned in tables 1, 2, 3, 7 and 9, more preferably R₁ is the side chain of an amino acid selected from the group consisting of compound 3, 4, 12, 13, 15, 20 to 24, 28, 29, 31, 47, 64, 66, 67, 71, 73, 74, 103 to 106 and 128 as outlined in tables 1, 2, 3, 7 and 9 herein below or R₁ is the side chain of an amino acid selected from the group consisting of compound 3 to 47 as outlined in tables 1, 2, and 3 herein below. R₂ is an acyl group or hydrogen group. In particular, if the optional carbonyl group is present it is preferred that R₂ is hydrogen. If said optional carbonyl group is not present, R₂ is preferably an acyl group, more preferably R₂ is selected from the group consisting of compounds 117 to 126 as outlined in table 9 herein below. R₃ is an aryl or alkyl, which may optionally be substituted, preferably R₃ is selected from the group consisting of compounds 103 to 116 and 128 as outlined in table 9 herein below. Preferred ligands of formula IV are any of the identified ligands mentioned in table 14, more preferred ligands are HY6 or HY7 or HY8 or HY9 as indicated in table 14 herein below.

The present invention also relates to isolated ligand-protein binding pairs comprising a ligand of formula IV. Preferably said ligand-protein binding pair is selected from the group consisting of the identified ligands and the corresponding identified proteins as outlined in table 14 herein below. In a preferred embodiment of the invention the ligand-protein binding pair consists of:
- Ligand HY6 of table 14; and
- Myosin chain (Q63358) or NF-kappa B-repressing factor (Transcription factor ITBA4 protein) (O15226). In another preferred embodiment of the invention the ligand-protein binding pair consists of:

- Ligand HY7 of table 14; and
- Zinc finger protein 339 (Q9BRPO) or DNA repair protein RAD52 homolog (P43351). In another preferred embodiment of the invention the ligand-protein binding pair consists of:
- Ligand HY8 of table 14; and
- Zinc finger protein 339 (Q9BRPO) or DNA repair protein RAD52 homolog (P43351). In another preferred embodiment of the invention the ligand-protein binding pair consists of:
- Ligand HY9 of table 14; and
- Zinc finger protein 339 (Q9BRPO) or DNA repair protein RAD52 homolog (P43351).

Furthermore, preferred ligands according to the present invention are ligands capable of associating, preferably specifically associating with one or more proteins selected from the group consisting of Ca2+/Calmodulin activated Myosin light chain kinase (gi 284660), Regulator of G-Pratein Signalling (RGS14) variant (gi 2708808), ATP Synthase component (subunit e) (gi 258788), Cytochrome P450 (gi 544086), Ribosomal proteins (60s) (gi 21426891), SPTR (gi 20837095), Troponin T (gi 547047), cGMP-dependent protein kinase (gi 284660), NADH dehydrogenase, ATP binding component (gi 18598538), Myosin heavy polypeptide 9 (gi 13543854), Histone associated proteins (gi 20893760), Hypothetical proteins (gi 20474763), Cysteine and tyrosine rich proteins of unknown function (gi 17064178), Mitochondrial ATP synthase (gi 13386040), SPTR (gi 12842570), (Sodium channel (gi 18591322), Chloride channel (gi 6978663/4502867), Troponin I (gi 1351298); Zn Finger protein (gi 18591322), SPTR - peroxisomal Ca dependent solute carrier (putative) (gi 12853685), Beta-2 adnergic receptor (gi 12699028), Hypothetical proteins, Phospholipase C, Phosphatidylcholine sterol acyl transferase (400167;LCAT-PIG_9), Serine/threonine Protein kinase (gi 5730055), Carbonic anhydrase VII (gi10304383), Chain C P27 cyclin A-CDK2 complex (gi 2392395); Hypothetical protein XP_154035, N4-(□-glucosaminyl-L-asparaginase; (gi7435941), Membrane spanning 4-domain subfamily A member II (gi7435941), Hypothetical protein XP_043250 (gi 14773490), Zinc finger associated protein (gi 20304091), Ribosomal proteins 40S L series (gi 206736/133023), Glucose-6-Phosphatase (gi 6679893/15488608), Succinate dehydrogenase, ARL-interacting protein (gi 4927202), SPTR (gi 12834839), Nucleic acid binding protein, Ribosomal protein (60s + 40s) (gi 20875941/6677773 and gi 20846353), Low density lipoprotein receptor (gi 20846353), Phosphofructokinase (gi 7331123), Selenium binding protein (gi 8848341/6677907); Serine arginine rich protein kinase, Guanylate kinase (gi 20986250), Actin interacting protein, SPTR (gi 20869775), Calcium channel (gi 3202010), Slo channel protein isoform (gi 3644046), Potassium conductance calcium activated channel (gi 6754436,NP_034740), Regulator of G-protein signalling 8 (gi 9507049), (Cathepsin E (gi 4503145), Ribosomal proteins (60s L series) (gi 20826861), NAS putative unclassified (gi 12861084), Putative Zn finger protein 64 (gi 12849329), Cell surface glycoprotein (gi 23603627), Hypothetical protein (XP-179829; gi 14720727), Orphan Nuclear receptor similar to hsp40 (NRID 26166582), Phosphate acetyl transferase (gi 1799680), Acid shock protein (gi 1742632), molybdopterin biosynthesis protein C (gi 15800534), Chaperone DnaK (dnak_E.coli), putative hydrolase (yhaG_E.coli), transposase (gi 158316821), Cytochrome C peroxidase (yhjA_E.coli), Histidine synthetase (gi 15803037), aspartate carbamoyl transferase (pyrI_E. coli), putative permease transport protein (b0831_E.co),,Orf hypothetical protein (yids_E.coli). Transposase, transcriptional regulator (gi 18265863), GroEL (GroEL_E.coli), protein involved in the taurine transport system (tauC_E.coli), Heme binding lipoprotein (gi 4062402/40624079), Regulator for D-glucarate, D-glycerate and D-galactarate (gi 158294209), Glutamine tRNA synthetase (gi146168), Biotin synthetase (gi 145425), UDP-glucose dehydrogenase (ugd_E.coli), tyrosine protein kinase (gi 20140365), Fatty acid oxidase complex proteins (gi 145900), NAD-dependent 7-alpha-hydroxysteriod dehydrogenase (gi 15802033), homocysteine transferase, nitrate reductase, lactate dehydrogenase (dld_E.coli), citrate synthetase (CISY_E.coli), Mannose-1-phosphate guanyl transferase (gi 3243143/324314), isopropyl malate dehydrogenase (guaB_E.coli), Pyruvoyl dependent aspartate decarboxylase (gi 3212459), Colicin E2 (gi809671/809683), Histidine kinase (part belongs to narQ_E.coli ), Protein involved in lipopolysaccharide biosynthesis (gi 16131496), Phosphomannose isomerase (gi147164), Cytochrome C type protein (gi 15802755), TrwC protein (TrwC_E.coli). Membrane bound ATP synthetase Fo sector subunit b (atpF_E.coli), ATP hydrolase (gi 1407605), Hemolysin C (gi7416115; gi 7438629), High affinity potassium transport system (kdpC_E.coli), quinone oxidoreductase (qor_E.coli), ferrodoxin dependent NA(D)PH oxidoreductase (fpil_E.coli), Transposase (gi 161295379), inner membrane protein for phage attachment (pspA_E.coli), ATP dependent helicase (gi 2507332/16128141), Mob C (gi 78702), Orf hypothetical protein (yciL_E.coli), TraI protein (Tri6_E.coli), Putative Transposase (gi 16930740), Fimbrial subunit (gi 2125931), outer membrane pyruvate kinase (gi 16129807/15831818), Fimbrial protein precursor (gi 120422), alkaline phosphatase (gi 581186), Cytochrome - zinc sensitive ATP component (cydD_E.coli), Putative aldolase, Chorismate mutase (gi 1800006), Xanthine dehydrogenase (gi 157999), Carbamoyl phosphate synthetase (carB_E.coli), Glutamate synthase (NaDPH) (gi 2121143), NADH dehydrogenase (gi 1799644), protein involved in flagellar biosynthesis and motor switching component (gi 1580237). Lysine-arginine-ornithine-binding protein (ArgT_E.coli), ATP-binding component of glycine-betaine-proline transport protein (gi 16130591), Colicin (gi 809683), Hypothetical membrane protein (yhiU_E.coli), Outer membrane lipoprotein (blc_E.coli), Acetly CoA carboxylase: beta subunit (gi 146364), Cytochrome b (cybC_E.coli), Phosphate acetyl transferase (gi 1073573), Urease: beta subunit (gi 418161), Molybdenum transport protein (gi1709069), Glycerol 3-phosphate dehydrogenase, subunit C (gi 146179), Cell division protein (ftsN_E.coli), Transposase (gi10955467), Serine tRNA synthetase (gi15830232), Methylase (gi1709155), Coenzyme A transferase (gi1613082), TraD membrane protein (TraD_E.coli), ATP dependent helicase: HrpA homolog (NCBIBAA15034), Putative protease ydcP percursor (NCBI P76104), Uroporphyrinogen Decarboxylase (hemE_E.coli), Putative export protein J for general secretory pathway (yheJ_E.coli), Concanavalin A lectin from C. *ensiformis* (gi:1705573), lectin from *P. sativum* (gi:490035), lectin from *L. culinaris* (gi:126145).

Numbers indicated in brackets after the name of a protein, (i.e. gi:xxxxxx) refer to the accession number of said protein in the NCBI protein database.

The present invention furthermore relates isolated ligand-protein binding pairs identified by the methods disclosed herein. Preferably, said isolated ligand-protein binding pairs comprises at least one of the ligands mentioned herein above and/or at least one of the proteins mentioned herein above. Preferred ligand-protein binding pairs are any of the ligand-protein binding pairs described in the examples herein below.

### Drug targets

It is also an objective of the present invention to provide proteins that are suitable as drug targets, hence in one embodiment the present invention relates to use of a protein identified by the methods according to the invention as a drug target, in a method to identify one or more drugs for the treatment of a clinical condition.

A drug target, as used herein, is a protein that is involved in causing a diseased state. This means that if that particular protein is prevented from being expressed or prevented from excerting its function in a model system, the disease does not occur at all or symptoms are fewer and/or less significant. A protein is also considered a drug target when its interaction with a compound (potential drug) results in modulation of the disease in in vitro and in vivo models.

Treatment may be prophylactic, curative and/or ameliorating treatment. The clinical condition may be any suitable clinical condition, for example cancer, cardiovascular diseases, autoimmune diseases, infections, inflammatory diseases, CNS disorders, metabolic diseases, endocrine diseases

Cardiovascular diseases for example include cardiac hypertrophy, coronary heart disease, cardiac arrythmias, rheumatic heart disease, endocardiosis and hypertrophic cardiomyopathy..

In particular, "drugable proteins" may be used as drug target. The term "drugable proteins" is meant to include proteins, to which one or more ligands bind specifically. Hence, proteins identified by the present method are in general "drugable".

It is preferred that the proteins for use as drug target according to the present invention are selected from the group consisting of Ca2+/Calmodulin activated Myosin light chain kinase (gi 284660), Regulator of G-Protein Signalling (RGS14) variant (gi 2708808), ATP Synthase component (subunit e) (gi 258788), Cytochrome P450 (gi 544086), Ribosomal proteins (60s) (gi 21426891), SPTR (gi 20837095), Troponin T (gi 547047), cGMP-dependent protein kinase (gi 284660), NADH dehydrogenase, ATP binding component (gi 18598538), Myosin heavy polypeptide 9 (gi 13543854), Histone associated proteins (gi 20893760), Hypothetical proteins (gi 20474763), Cysteine and tyrosine rich proteins of unknown function (gi 17064178), Mitochondrial ATP synthase (gi 13386040), SPTR (gi 12842570), (Sodium channel (gi 18591322), Chloride channel (gi 6978663/4502867), Troponin I (gi 1351298); Zn Finger protein (gi 18591322), SPTR - peroxisomal Ca dependent solute carrier (putative) (gi 12853685), Beta-2 adnergic receptor (gi 12699028), Hypothetical proteins, Phospholipase C, Phosphatidylcholine sterol acyl transferase (400167;LCAT-PIG_9), Serine/threonine Protein kinase (gi 5730055), Carbonic anhydrase VII (gi10304383), Chain C P27 cyclin A-CDK2 complex (gi 2392395); Hypothetical protein XP_154035, N4-(□-glucosaminyl-L-asparaginase; (gi7435941), Membrane spanning 4-domain subfamily A member II (gi7435941), Hypothetical protein XP_043250 (gi 14773490), Zinc finger associated protein (gi 20304091), Ribosomal proteins 40S L series (gi 206736/133023), Glucose-6-Phosphatase (gi 6679893/15488608), Succinate dehydrogenase, ARL-interacting protein (gi 4927202), SPTR (gi 12834839), Nucleic acid binding protein, Ribosomal protein (60s + 40s) (gi 20875941/6677773 and gi 20846353), Low density lipoprotein receptor (gi 20846353), Phosphofructokinase (gi 7331123), Selenium binding protein (gi 8848341/6677907); (Serine arginine rich protein kinase, Guanylate kinase (gi 20986250), Actin interacting protein, SPTR (gi 20869775), Calcium channel (gi 3202010), Slo channel protein isoform (gi 3644046), Potassium conductance calcium activated channel (gi 6754436,NP_034740), Regulator of G-protein signalling 8 (gi 9507049), (Cathepsin E (gi 4503145), Ribosomal proteins (60s L series) (gi 20826861), NAS putative unclassified (gi 12861084), Putative Zn finger protein 64 (gi 12849329), Cell surface glycoprotein (gi 23603627), Hypothetical protein (XP-179829; gi 14720727), Orphan Nuclear receptor similar to hsp40 (NRID 26166582), Phosphate acetyl transferase (gi 1799680), Acid shock protein (gi 1742632), molybdopterin biosynthesis protein C (gi 15800534), Chaperone DnaK (dnak_E.coli), putative hydrolase (yhaG_E.coli), transposase (gi 158316821), Cytochrome C peroxidase (yhjA_E.coli), Histidine synthetase (gi 15803037), aspartate carbamoyl transferase (pyrI_E. coli), putative permease transport protein (b0831_E.co),,Orf hypothetical protein (yids_E.coli). Transposase, transcriptional regulator (gi 18265863), GroEL (GroEL_E.coli), protein involved in the taurine transport system (tauC_E.coli), Heme binding lipoprotein (gi 4062402/40624079), Regulator for D-glucarate, D-glycerate and D-galactarate (gi 158294209), Glutamine tRNA synthetase (gi146168), Biotin synthetase (gi 145425), UDP-glucose dehydrogenase (ugd_E.coli), tyrosine protein kinase (gi 20140365), Fatty acid oxidase complex proteins (gi 145900), NAD-dependent 7-alpha-hydroxysteriod dehydrogenase (gi 15802033), homocysteine transferase, nitrate reductase, lactate dehydrogenase (dld_E.coli), citrate synthetase (CISY_ E.coli), Mannose-1-phosphate guanyl transferase (gi 3243143/ 324314), isopropyl malate dehydrogenase (guaB_E.coli), Pyruvoyl dependent aspartate decarboxylase (gi 3212459), Colicin E2 (gi809671/809683), Histidine kinase (part belongs to narQ_E.coli), Protein involved in lipopolysaccharide biosynthesis (gi 16131496), Phosphomannose isomerase (gi 147164), Cytochrome C type protein (gi 15802755), TrwC protein (TrwC_E.coli). Membrane bound ATP synthetase Fo sector subunit b (atpF_E.coli), ATP hydrolase (gi 1407605), Hemolysin C (gi7416115; gi 7438629), High affinity potassium transport system (kdpC_E.coli), quinone oxidoreductase (qor_E.coli), ferrodoxin dependent NA(D)PH oxidoreductase (fpr_E.coli), Transposase (gi 161295379), inner membrane protein for phage attachment (pspA_E.coli), ATP dependent helicase (gi 2507332/16128141), Mob C (gi 78702), Orfhypothetical protein (yciL_E.coli), TraI protein (Tri6_E.coli), Putative Transposase (gi 16930740), Fimbrial subunit (gi 2125931), outer membrane pyruvate kinase (gi16129807/15831818), Fimbrial protein precursor (gi 120422), alkaline phosphatase (gi 581186), Cytochrome - zinc sensitive ATP component (cydD_E.coli), Putative aldolase, Chorismate mutase (gi 1800006), Xanthine dehydrogenase (gi 157999), Carbamoyl phosphate synthetase (carB_E.coli), Glutamate synthase (NaDPH) (gi 2121143), NADH dehydrogenase (gi 1799644), protein involved in flagellar biosynthesis and motor switching component (gi 1580237). Lysine-arginine-ornithine-binding protein (ArgT_E.coli), ATP-binding component of glycine-betaine-proline transport protein (gi 16130591), Colicin (gi 809683), Hypothetical membrane protein (yhiU_E.coli), Outer membrane lipoprotein (blc_E.coli), Acetly CoA carboxylase: beta subunit (gi 146364), Cytochrome b (cybC_E.coli), Phosphate acetyl transferase (gi 1073573), Urease: beta subunit (gi 418161), Molybdenum transport protein (gi1709069), Glycerol 3-phosphate dehydrogenase subunit C (gi 146179), Cell division protein (ftsN_E.coli), Transposase (gi10955467), Serine tRNA synthetase (gi15830232), Methylase (gi1709155), Coenzyme A transferase (gi 1613082), TraD membrane protein (TraD_E.coli), ATP dependent helicase:
HrpA homolog (NCBIBAA15034), Putative protease ydcP percursor (NCBI P76104), Uroporphyrinogen Decarboxylase (hemE_E.coli), Putative export protein J for general secretory pathway (yheJ_E.coli), Concanavalin A lectin from C. *ensiformis* (gi:1705573), lectin from *P. sativum* (gi:490035), lectin from *L. culinaris* (gi:126145).

Preferably a protein selected from the group consisting of Troponin T (gi 547047); Growth hormone receptor; Chain C P27 cyclin A-CDK2 complex (gi2392395); Hypothetical protein XP_154035; Sodium channel (gi 18591322) (M); Chloride channel (gi 6978663/4502867) (M)) Troponin I (gi 1351298); Zn Finger protein (gi 18591322) (MA); SPTR (peroxisomal Ca dependent solute carrier (putative) (gi 12853685); Beta-2 adnergic receptor (gi 12699028), Zinc finger associated protein (gi 20304091); Glucose-6-Phosphatase (gi 6679893/15488608), Succinate dehydrogenase, ARL-interacting protein (gi 4927202); Nucleic acid binding protein; Phosphofructokinase (gi 7331123), Selenium binding protein (gi 8848341/6677907), Serine arginine rich protein kinase; Guanylate kinase (gi 20986250), Alkaline phosphatase; Symporter; WAP-protein; GTP Hydrolase; Actin filament; SPTR (gi 20869775 ) (M); Ribosomal proteins (60s) (gi 20875941/6677773); (Calcium channel (gi 3202010) (M); Slo channel protein isoform (gi 3644046 ) (M); Potassium conductance calcium activated channel (gi 6754436,NP_034740) (M); Regulator of G-protein signalling 8 (gi 9507049); Cathepsin E (gi 4503145), Ribosomal proteins (60s L series)(gi20826861), Actin binding protein; Amino acid transferase; NAS putative unclassified (gi 12861084); Putative Zn finger protein 64 (gi 12849329); Histidine synthetase (gi 15803037); aspartate carbamoyl transferase (pyrI_E.coli); 50 S ribosomal protein; heme binding lipoprotein (gi 4062402/40624079), regulator for D-glucarate, D-glycerate and D-galactarate (gi 158294209); glutamine tRNA synthetase (gi 146168); Pyruvoyl dependent aspartate decarboxylase (gi 3212459), colicin E2 (gi 809671/809683); Histidine kinase (part belongs to narQ_E.coli); phosphomannose isomerase (gi 147164); ATP dependent helicase (gi 2507332/16128141); mob C (gi 78702); Chorismate mutas (gi 1800006), xanthine dehydrogenase (gi 157999); carbamoyl phosphate synthetase (carB_E.coli); ATP dependent helicase: HrpA homolog (NCBIBAA15034); Putative protease ydcP percursor (NCBI P76104); Concanavalin A, lectin from *P. sativum* (gi:490035) and lectin from *L. culinaris* (gi:126145) may be used as a drug target, in a method to identify one or more drugs for the treatment of a clinical condition according to the invention.

More preferably the protein to be used as drug target is selected from the group consisting of Sodium channel (gi 18591322) Chloride channel (gi 6978663/4502867)); Zn Finger protein (gi 18591322) SPTR (peroxisomal Ca dependent solute carrier (putative) (gi 12853685); Beta-2 adrenergic receptor (gi 12699028), Zinc finger associated protein (gi 20304091); Glucose-6-Phosphatase (gi 6679893/15488608), Succinate dehydrogenase, ARL-interacting protein (gi 4927202); Nucleic acid binding protein; Phosphofructokinase (gi 7331123), Selenium binding protein (gi 8848341/6677907), Serine arginine rich protein kinase; Guanylate kinase (gi 20986250),Calcium channel (gi 3202010 Slo channel protein isoform (gi 3644046 ) Potassium conductance calcium activated channel (gi 6754436,NP_034740) Regulator of G-protein signalling 8 (gi 9507049); Regulator of G-protein signalling 14, Cathepsin E (gi 4503145), Putative Zn finger protein 64 (gi 12849329); Histidine synthetase (gi 15803037); aspartate carbamoyl transferase (pyrI_E.coli); 50 S ribosomal protein; heme binding lipoprotein (gi 4062402/40624079glutamine tRNA synthetase (gi 146168); Pyruvoyl dependent aspartate decarboxylase (gi 3212459), Histidine kinase (part belongs to narQ_E.coli); phosphomannose isomerase (gi 147164); ATP dependent helicase (gi 2507332/16128141); mob C (gi 78702); Chorismate mutase (gi 1800006), carbamoyl phosphate synthetase (carB_E.coli); ATP dependent helicase: HrpA homolog (NCBIBAA15034); Inward rectifier potassium channel 13 (Q9QZ65, IRKD_CAVPO); Sulfonylurea receptor 2 (Q63563); small conductance potassium channel (P58391), Serine/threonine protein kinase (088866); Ras GTPase activating protein 2 (GAP1m) (P58069), Glycogen synthase kinase-3 beta factor (AQ9WV60), Serine/threanine kinase 9/cylin dependent Serine/threonine kinase 9(23271297/4507281); NF-kappa B-repressing factor (Transcription factor ITBA4 protein) (015226), HLA-binding protein (gi 1279435) and Large subunit of ribonucleotide reductase (gi 30984467.

In one embodiment of the present invention, preferred proteins for use as drug target according to the present invention may be selected from the group consisting of Chaperone DnaK (dnak_E.coli), putative hydrolase (yhaG_E.coli), transposase (gi 158316821), Histidine synthetase (gi 15803037), aspartate carbamoyl transferase (pyrI_E. coli), transcriptional regulator (gi 18265863 glutamine tRNA synthetase (gi146168), tyrosine protein kinase (gi 20140365), citrate synthetase (CISY_E.coli), Pyruvoyl dependent aspartate decarboxylase (gi 3212459), colicin E2. (gi809671/809683), Histidine kinase (part belongs to narQ_E.coli), Protein involved in lipopolysaccharide biosynthesis (gi 16131496), phosphomannose isomerase (gi147164), high affinity potassium transport system (kdpC_E.coli), ATP dependent helicase (gi 2507332/16128141), mob C (gi 78702); Orf hypothetical protein (yciL_E.coli), outer membrane pyruvate kinase (gi 16129807/15831818), Fimbrial protein precursor (gi120422), alkaline phosphatase, Putative aldolase, Chorismate mutase (gi 1800006), carbamoyl phosphate synthetase (carB_E.coli); Glutamate synthase (NaDPH) (gi 2121143), protein involved in flagellar biosynthesis and motor switching component, Lysine-arginine-ornithine-binding protein (argT_E.coli), ATP-binding component of glycine-betaine-proline transport protein (gi 16130591), hypothetical membrane protein (yhiU_E.coli), outer membrane lipoprotein (blc_E.coli), Molybdenum transport protein (gi1709069), Serine tRNA synthetase (gi15830232), ATP dependent helicase: HrpA homolog (NCBIBAA15034), Putative export protein J for general secretory pathway (yheJ_E.coli), molybdopterin biosynthesis protein C (gi 15800534). protein involved in the taurine transport system (tauC_E.coli). Said proteins are in particular useful as drug targets to identify drugs for treatment of infections.

In a preferred embodiment the protein to be used as drug target is selected from the group consisting of Chaperone DnaK (dnak_E.coli), Histidine synthetase (gi 15803037), aspartate carbamoyl transferase (pyrI_E. coli), glutamine tRNA synthetase (gi146168). tyrosine protein kinase (gi 20140365), citrate synthetase (CISY_E.coli), Pyruvoyl dependent aspartate decarboxylase (gi 3212459), colicin E2. (gi809671/809683), Histidine kinase (part belongs to narQ_E.coli), Protein involved in lipopolysaccharide biosynthesis (gi 16131496), phosphomannose isomerase (gi 147164), high affinity potassium transport system (kdpC_E.coli), ATP dependent helicase (gi 2507332/16128141), mob C (gi 78702); Orf hypothetical protein (yciL_E.coli), outer membrane pyruvate kinase (gi16129807/15831818), alkaline phosphatase, Chorismate mutase (gi 1800006), carbamoyl phosphate synthetase (carB_E.coli); Glutamate synthase (NaDPH) (gi 2121143), protein involved in flagellar biosynthesis and motor switching component,), Serine tRNA synthetase (gi15830232) and ATP dependent helicase: HrpA homolog (NCBIBAA15034),

Even more preferred proteins for use as drug targets according to the invention may be selected from the group consisting of Chaperone DnaK (dnak_E.coli), putative hydrolase (yhaG_E.coli), transposase (gi 158316821), Histidine synthetase (gi 15803037), aspartate Carbamoyl transferase (pyrI_E. coli), transcriptional regulator (gi 18265863 glutamine tRNA synthetase (gi146168). tyrosine protein kinase (gi 2,0140365), citrate synthetase (CISY_E.coli), Pyruvoyl dependent aspartate decarboxylase (gi 3212459), Histidine kinase (part belongs to narQ_E.coli ), Protein involved in lipopolysaccharide biosynthesis (gi 16131496), phosphomannose isomerase (gi147164), ATP dependent helicase (gi 2507332/16128141), Orf hypothetical protein (yciL_E.coli), outer membrane pyruvate kinase (gi16129807/15831818), Chorismate mutase (gi 1800006), carbamoyl phosphate synthetase (carB_E.coli); Glutamate synthase (NaDPH) (gi 2121143), Lysine-arginine-ornithine-binding protein (argT_E.coli), hypothetical membrane protein (yhiU_E.coli), outer membrane lipoprotein (blc_E.coli), Serine tRNA synthetase (gi15830232), ATP dependent helicase: HrpA homolog (NCBIBAA15034), Putative export protein J for general secretory pathway (yheJ_E.coli).

Yet more preferably, the protein used as drug target is selected from the group consisting of transpoase, proteins involved in Chaperone DnaK (dnak_E.coli), transposase (gi 158316821), Histidine synthetase (gi 15803037), aspartate carbamoyl transferase (pyrI_E. coli), transcriptional regulator (gi 18265863 glutamine tRNA synthetase (gi146168). tyrosine protein kinase (gi 20140365), citrate synthetase (CISY_E.coli), Pyruvoyl dependent aspartate decarboxylase (gi 3212459), Histidine kinase (part belongs to narQ_E.coli), Protein involved in lipopolysaccharide biosynthesis (gi 16131496), phosphomannose isomerase (gi147164), ATP dependent helicase (gi 2507332/16128141), Chorismate mutase (gi 1800006), carbamoyl phosphate synthetase (carB_E.coli); Glutamate synthase (NaDPH) (gi 2121143), Serine tRNA synthetase (gi15830232), ATP dependent helicase: HrpA homolog (NCBIBAA15034), HLA-binding protein (gi 1279435) and Large subunit of ribonucleotide reductase (gi 30984467)

Said proteins are in particular useful as drug targets to identify drugs for treatment of infections.

In another embodiment of the present invention, preferred proteins for use as drug targets may be selected from the group consisting of Ga2+/Calmadulin activated Myosin light chain kinase (gi 284660), Regulator of G-Protein Signalling (RGS14) variant (gi 2708808), SPTR (gi 20837095), Hypothetical proteins (gi 20474763); Cysteine and tyrosine rich proteins of unknown function (gi17064178) SPTR (gi12842570), Sodium channel (gi 18591322); Chloride channel (gi 6978663/4502867); Zn Finger protein (gi 18591322); SPTR (peroxisomal Ca dependent solute carrier (putative) (gi 12853685); Beta-2 adnergic receptor (gi 12699028); Serine/threonine Protein kinase (gi 5730055); Chain C P27 cyclin A-CDK2 complex (gi2392395); Hypothetical protein XP_154035; Membrane spanning 4-domain subfamily A member II (gi7435941); Hypothetical protein XP_043250 (gi 14773490); Zinc finger associated protein (gi 20304091); Serine arginine rich protein kinase; SPTR (gi 20869775); Calcium channel (gi 3202010); Slo channel protein isoform (gi 3644046); Potassium conductance calcium activated channel (gi 6754436,NP_034740);; Regulator of G-protein signalling 8 (gi 9507049); Cathepsin E (gi 4503145); NAS putative unclassified (gi 12861084); Putative Zn finger protein 64 (gi 12849329); Cell surface glycoprotein (gi 23603627); Hypothetical protein (XP-179829; gi 14720727); Orphan Nuclear receptor similar to hsp40 (NRID 26166582). Said proteins are in particular useful as drug targets to identify drugs for treatment of cardiovascular disease, for example cardiac hyper-throphy, coronary heart disease, cardiac arrythmias, rheumatic heart disease, endocardiosis and hypertrophic cardiomyopathy

Even more preferred proteins for use as drug targets may be selected from the group consisting of Ca2+/Calmodulin activated Myosin light chain kinase (gi 284660), Regulator of G-Protein Signalling (RGS14) variant (gi 2708808), SPTR (gi 20837095), Hypothetical proteins (gi 20474763); Cysteine and tyrosine rich proteins of unknown function (gi17064178) SPTR (gi 12842570), Zn Finger protein (gi 18591322); SPTR (peroxisomal Ca dependent solute carrier (putative) (gi 12853685); Beta-2 adnergic receptor (gi 12699028); Serine/threonine Protein kinase (gi 5730055); Chain C P27 cyclin A-CDK2 complex (gi2392395); Hypothetical protein XP_154035; Membrane spanning 4-domain subfamily A member II (gi7435941); Hypothetical protein XP_043250 (gi 14773490); Zinc finger associated protein (gi 20304091); Serine arginine rich protein kinase; SPTR (gi 20869775); Regulator of G-protein signalling 8 (gi 9507049); Cathepsin E (gi 4503145); Putative Zn finger protein 64 (gi 12849329); Hypothetical protein (XP-179829; gi 14720727); Orphan Nuclear receptor similar to hsp40 (NRID 26166582). Said proteins are in particular useful as drug targets to identify drugs for treatment of cardiovascular disease, for example cardiac hyper-throphy, coronary heart disease, cardiac arrythmias, rheumatic heart disease, endocardiosis and hypertrophic cardiomyopathy.

In another preferred embodiment the protein to be used as druig target is selected from the group consisting of Ca2+/Calmodulin activated Myosin light chain kinase (gi 284660), Regulator of G-Protein Signalling (RGS14) variant (gi 2708808), SPTR (gi 20837095), Sodium channel (gi 18591322); Chloride channel (gi 6978663/4502867); Zn Finger protein (gi 18591322); SPTR (peroxisomal Ca dependent solute carrier (putative) (gi 12853685); Beta-2 adnergic receptor (gi 12699028); Serine/threonine Protein kinase (gi 5730055); Chain C P27 cyclin A-CDK2 complex (gi2392395); Membrane spanning 4-domain subfamily A member II (gi7435941); Zinc finger associated protein (gi 20304091); Serine arginine rich protein kinase; SPTR (gi 20869775); Calcium channel (gi 3202010); Slo channel protein isoform (gi 3644046); Potassium conductance calcium activated channel (gi 6754436,NP_034740);; Regulator of G-protein signalling 8 (gi 9507049); Cathepsin E (gi 4503145); NAS putative unclassified (gi 12861084); Putative Zn finger protein 64 (gi 12849329); Cell surface glycoprotein (gi 23603627); Hypothetical protein {XP-179829; gi 14720727); Orphan Nuclear receptor similar to hsp40 (NRID 26166582, Inward rectifier potassium channel 13 (Q9QZ65, IRKD_CAVPO); Sulfonylurea receptor 2 (Q63563); small conductance potassium channel (P58391), Serine/threonine protein kinase (088866); Ras GTPase activating protein 2 (GAP1m) (P58059), Glycogen synthase kinase-3 beta factor (AQ9WV60), Serine/threonine kinase 9/cylin dependent Serine/threonine kinase 9(23271297/4507281) and NF-kappa B-repressing factor (Transcription factor ITBA4 protein) (015226).

Preferably said protein is selected from the group consisting of Ca2+/Calmodulin activated Myosin light chain kinase (gi 284660), Regulator of G-Protein Signalling (RGS14) variant (gi 2708808), SPTR (gi 20837095), Zn Finger protein (gi 18591322); SPTR (peroxisomal Ca dependent solute carrier (putative) (gi 12853685); Beta-2 adrenergic receptor (gi 12699028); Serine/threonine Protein kinase (gi 5730055); Chain C P27 cyclin A-CDK2 complex (gi2392395); Zinc finger associated protein (gi 20304091); Serine arginine rich protein kinase; SPTR (gi 20869775); Regulator of G-protein signalling 8 (gi 9507049); Cathepsin E (gi 4503145) and Putative Zn finger protein 64 (gi 12849329) and Orphan Nuclear receptor similar to hsp40 (NRID 26166582).

### Advantages of the Invention

The high throughput process invention described herein provides several advantages over known processes for drug discovery. Among these advantages are increased speed and accuracy in the simultaneous identification of a ligand molecule and its matched protein-binding partner.

The process of the invention can be accomplished on a solid support, preferably on resin beads, and permits synthesis, screening, isolation, and identification of ligand and protein steps to be quickly and efficiently processed using a single bead. The process can be readily automated, for example, using known automatic systems for synthesis, incubation, isolation, and identification steps, such as robotic systems for cleavage of ligand, spotting on to MS targets, adding enzymes for protein digestion, and the like. Using Mass Spectrometry, MALDI, and NMR as described in the Examples, each of the ligand and protein can be identified "on bead."

The process invention also provides an advantage due to the large diversity of libraries that can be used, for example, in excess of 10,000 different compounds. Virtually millions of compounds can be rapidly screened, for example, in resin systems employing one compound per bead, for example, using about 3 to 5 million beads available in about 10 g of resin.

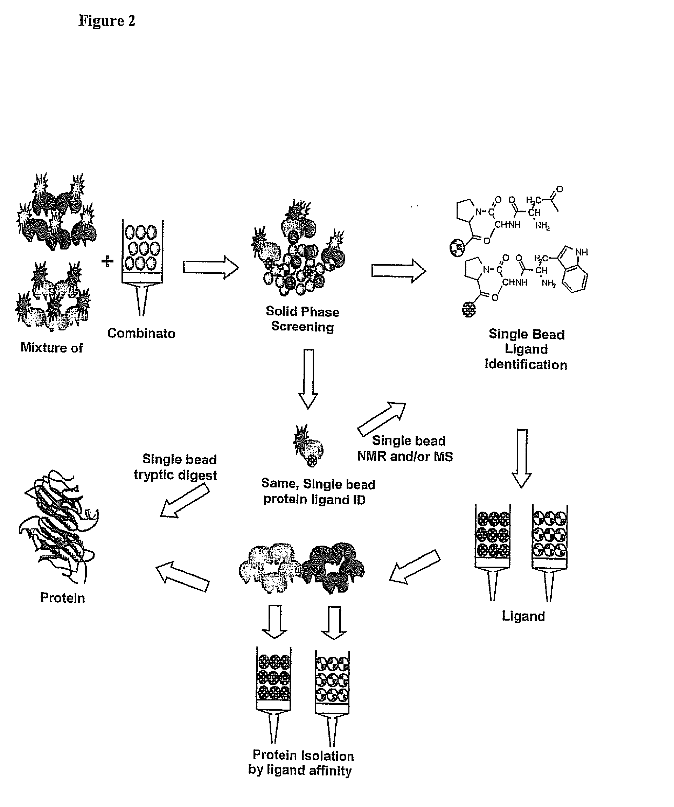

Using the claimed process, it is possible to rapidly identify ligand protein binding complexes that are "drugable," that is, to identify useful ligands that bind precise proteins and to avoid non-useful ligands. The process further enables the rapid identification of families of proteins and/or non-related proteins that bind to the same or similar ligands. Such information delineates potential selectivity of a drug candidate and provides preliminary toxicological information to aid the drug selection process. The process invention further provides identification of classes of ligands that bind a particular protein increasing the number of "hits" that can be developed into lead compounds for a particular protein target. When the process invention is carried out in the differential manner as described in Figure 2, that is, using differentially labeled proteins, for example, from a normal and diseased tissue, all the above features are added to the determination of selective ligand/protein pairs that can be used for diagnostic and therapeutic product development.

In sum, one great advantage of the claimed invention is the ability to take a very large number of unknown ligands and/or unknown proteins, and in a very short time and efficient manner identify particular, previously unknown ligand/protein binding pairs that are identified, matched, and characterized as described above.

### EXAMPLES

The invention may be better understood with reference to the following Examples. These Examples illustrate the invention, and are not intended to limit its scope.

In the Examples below, the synthesis of ligand libraries and use of these libraries in the process invention is exemplifies. The chemical entities used in the examplified syntheses are shown in the tables and schemes below, with identification numbers in parentheses (x). In the tables and schemes shown below, the compound numbers (x) are denoted "a" where R₁ is Fmoc, "b" where R₁ is Boc and "c" where R₁ is H.

**TABLE 1**

| **Linker, spacer, and genetically encoded (natural) amino acid building blocks used in the synthesis of Libraries 1, 2, 3, 4, 5, 6 and 7.** |
|---|
| 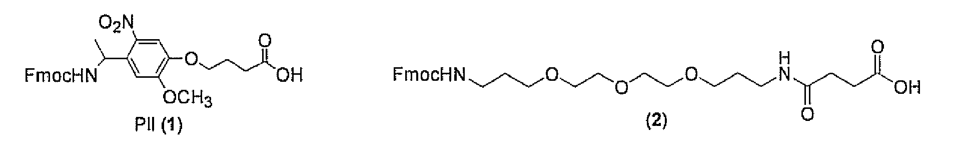 |
| 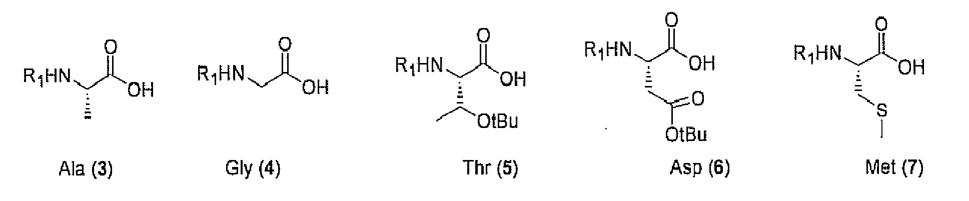 |
| 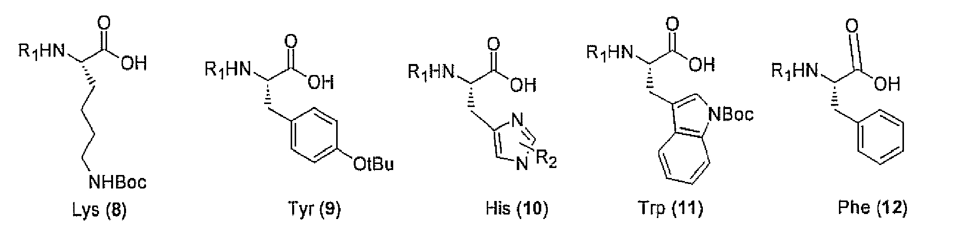 |
| 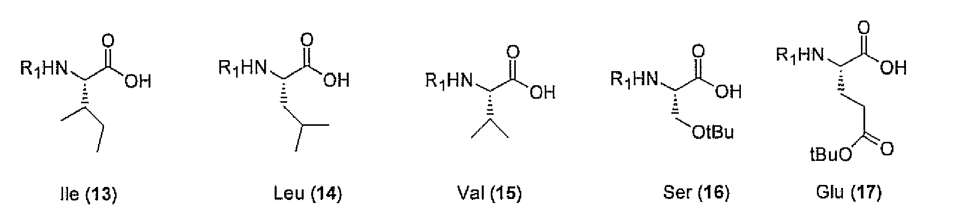 |
| 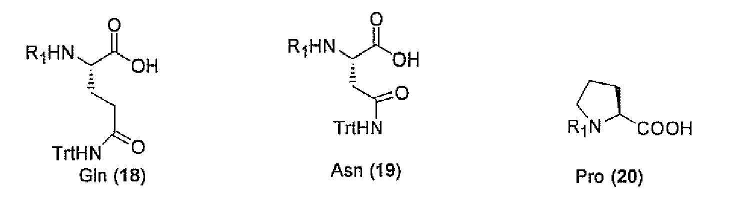 |
| R₁ = Fmoc, Boc, H (a) (b) (c) For 10, R₂ = Boc or Trt |

**TABLE 2**

| **Aliphatic encoding tags and other building blocks used in the synthesis of Libraries 1, 2, 3, 4, 5, 6 and 7.** |
|---|
| 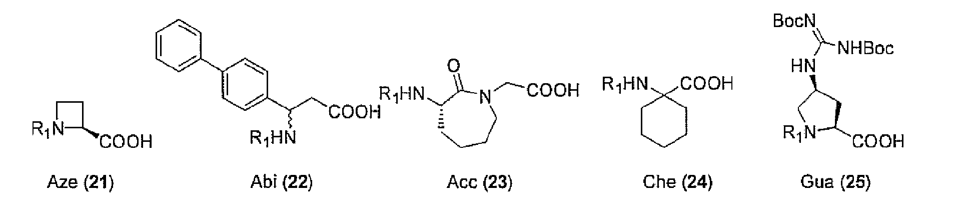 |
| 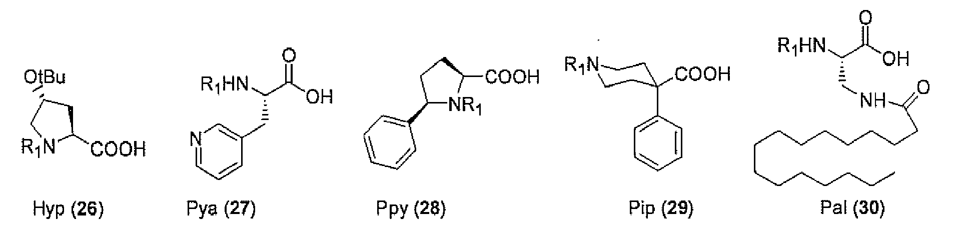 |
| 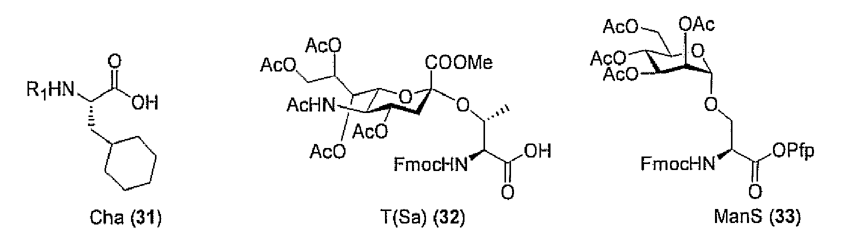 |
| 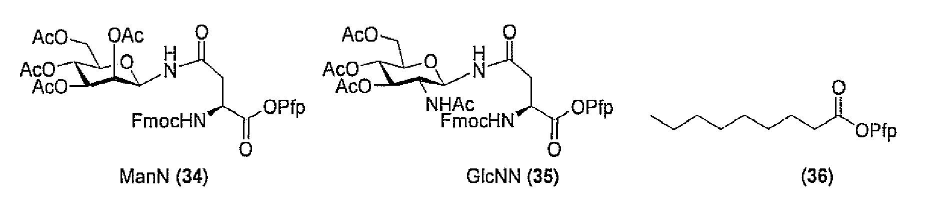 |
| 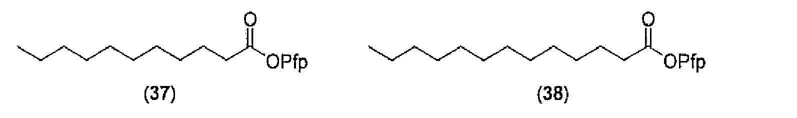 |
| R₁ = Fmoc, Boc (a) (b) |

**TABLE 3:**

| **Compounds Used for Synthesis of Library 4, 5 and 7** |
|---|
| 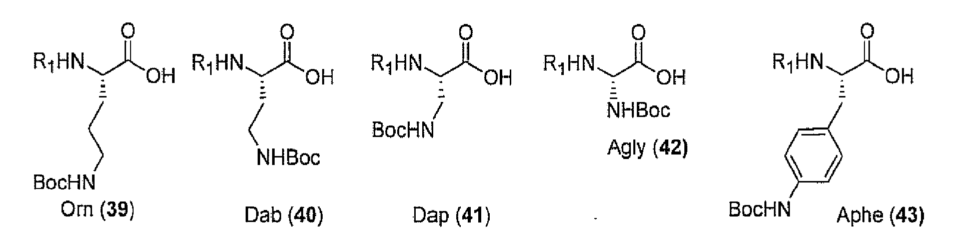 |
| 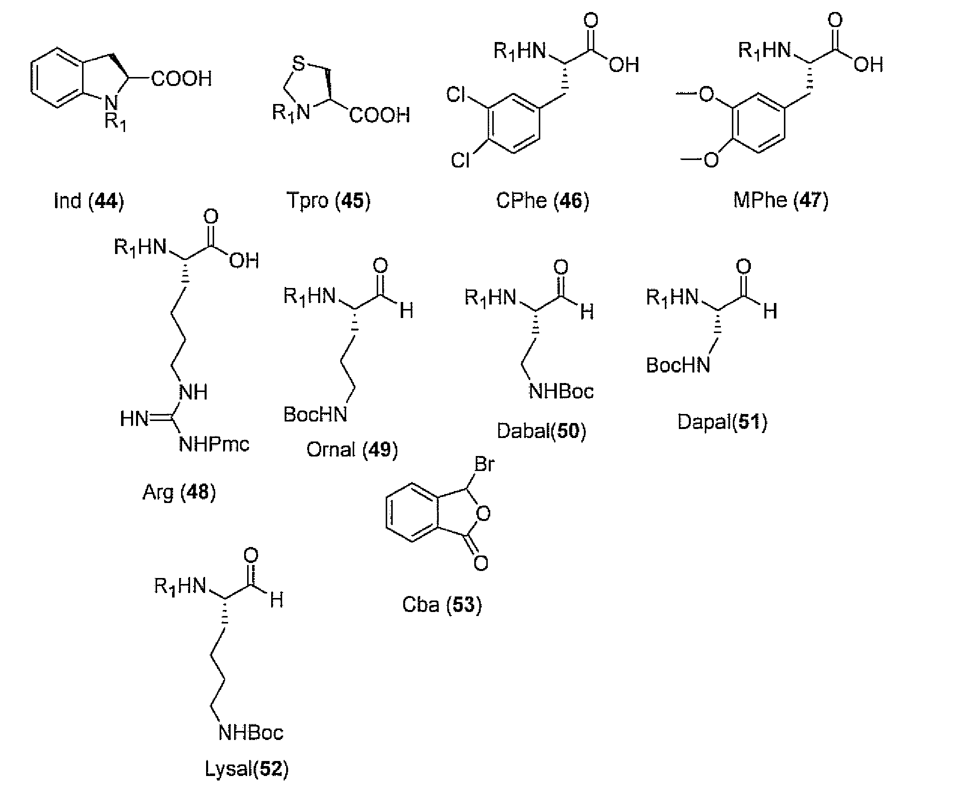 |

**TABLE 7:**

| **Compounds Used for Synthesis of Library 4, 5, 6 and 7.** |
|---|
| 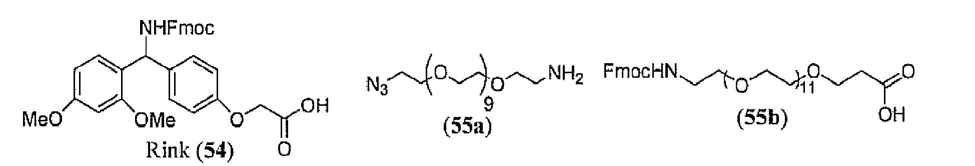 |
| 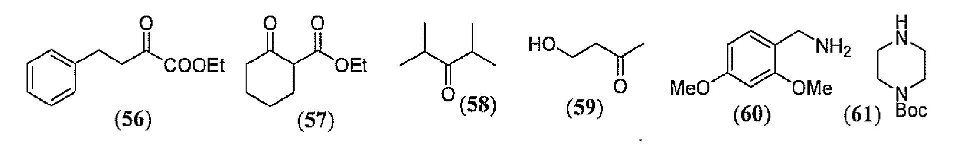 |
| 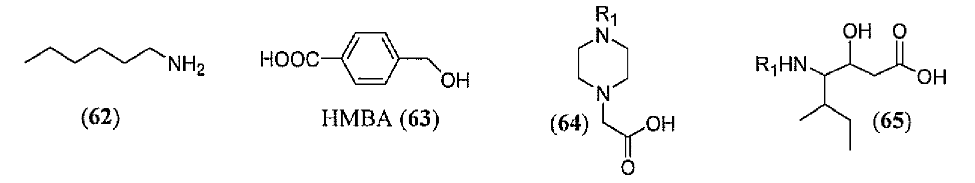 |
| 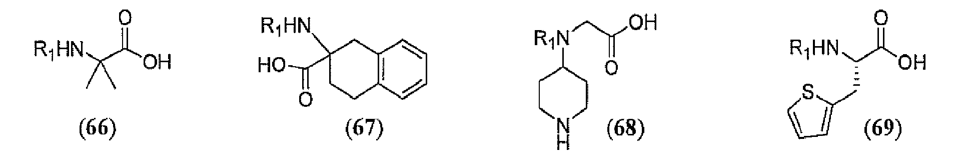 |
| 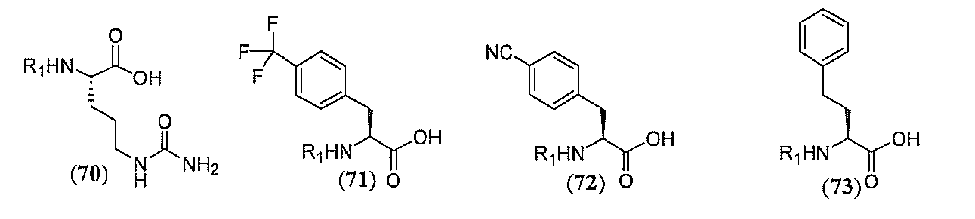 |
| 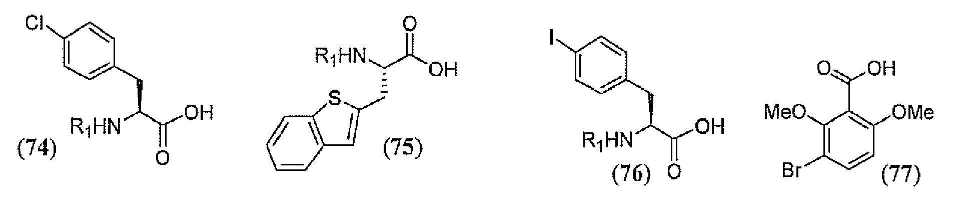 |
| 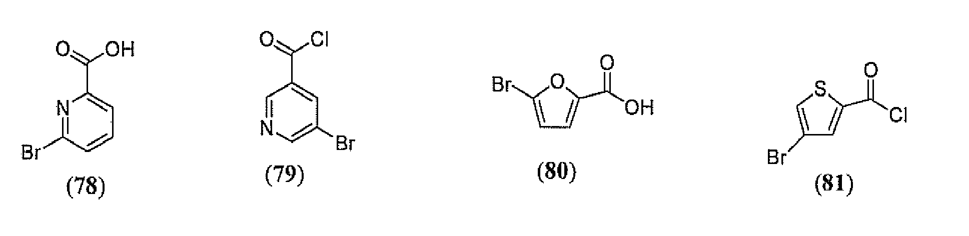 |

**TABLE 8**

| **Compounds Used for Synthesis of Library 7** |
|---|
| 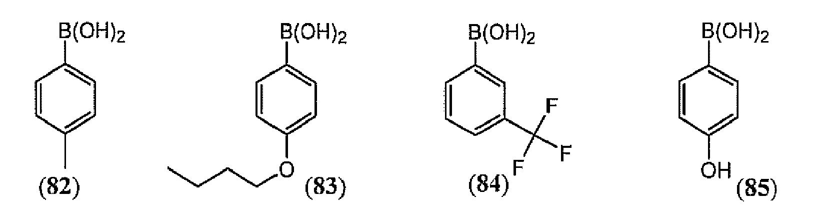 |
| 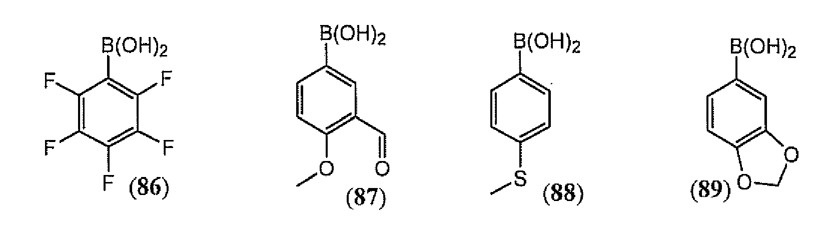 |
| 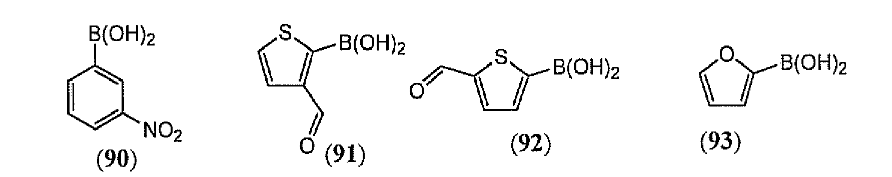 |
| 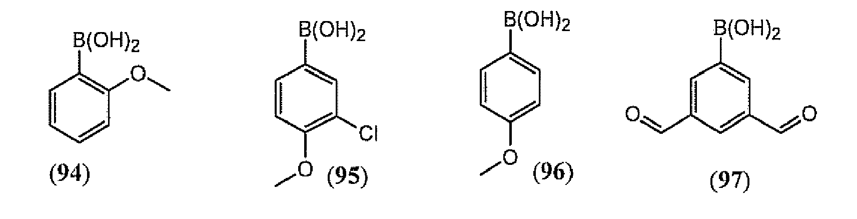 |
| 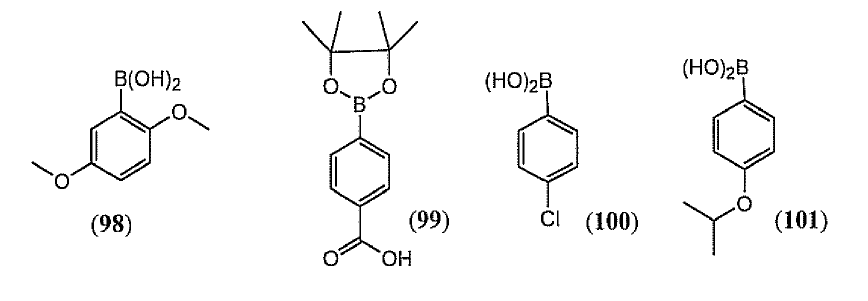 |

**TABLE 9:**

| **Compounds Used for Synthesis of Library 4, 5 and 7** |
|---|
| 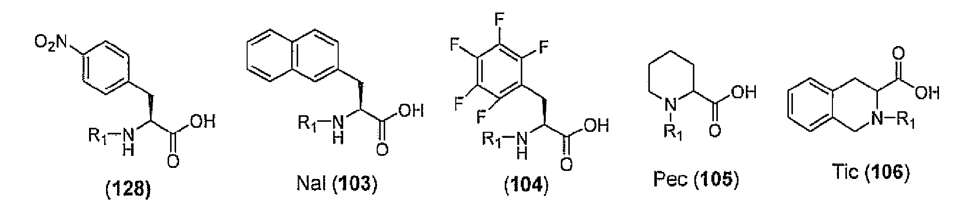 |
| 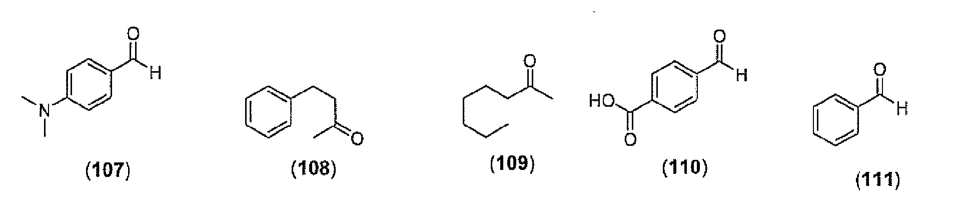 |
| 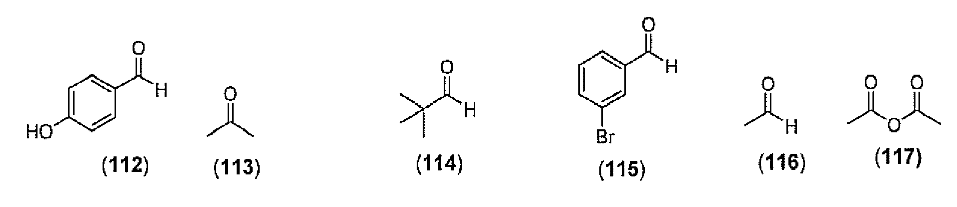 |
| 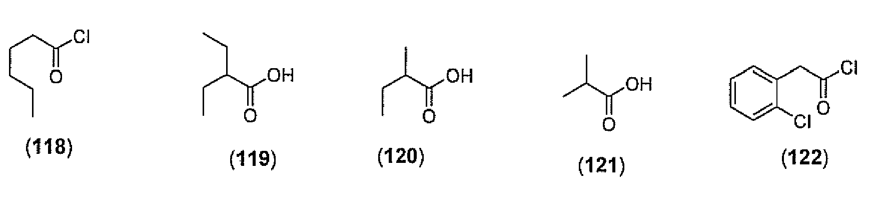 |
| 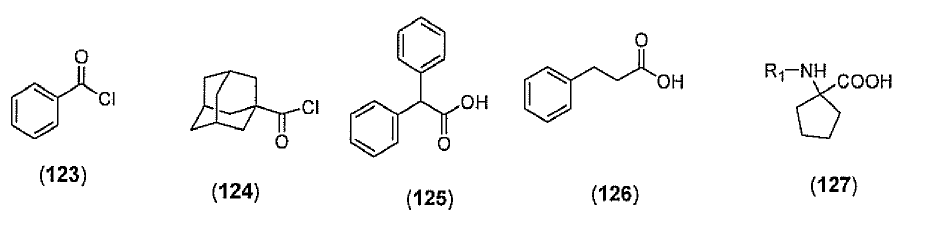 |

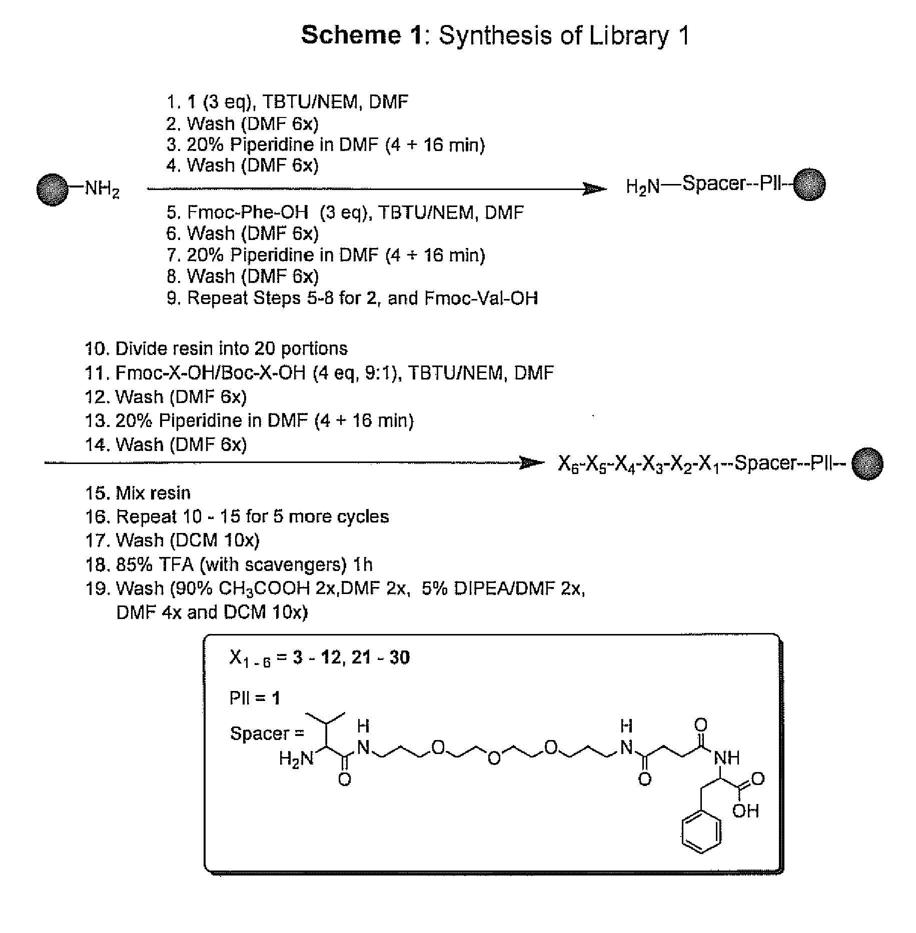

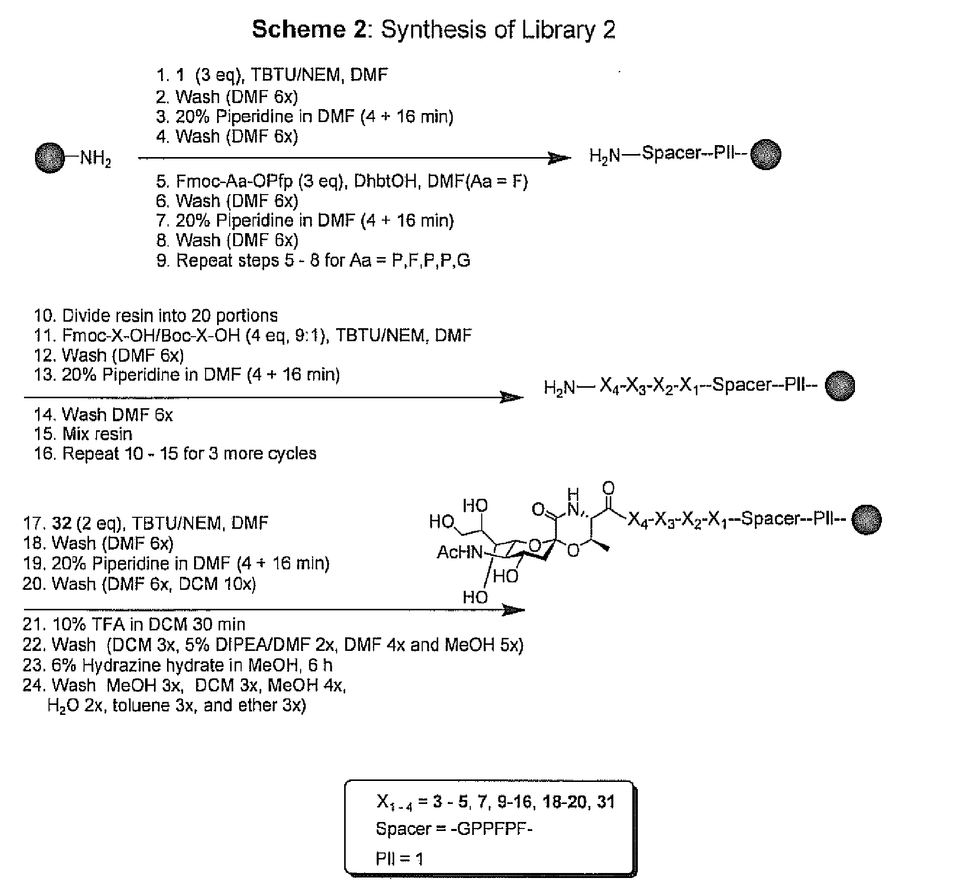

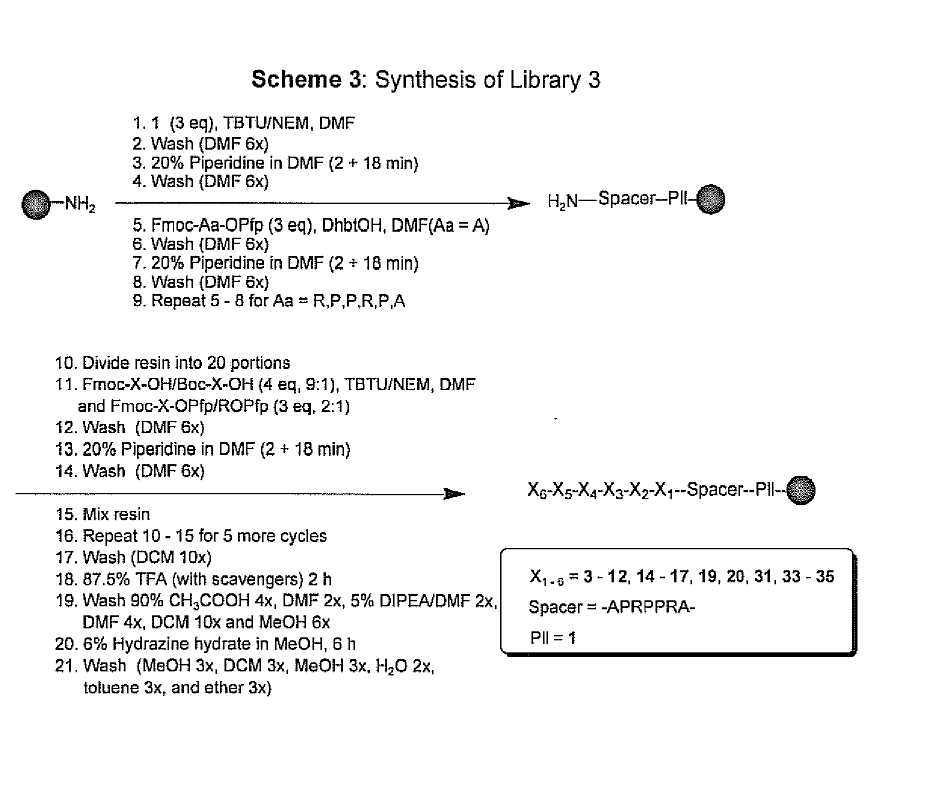

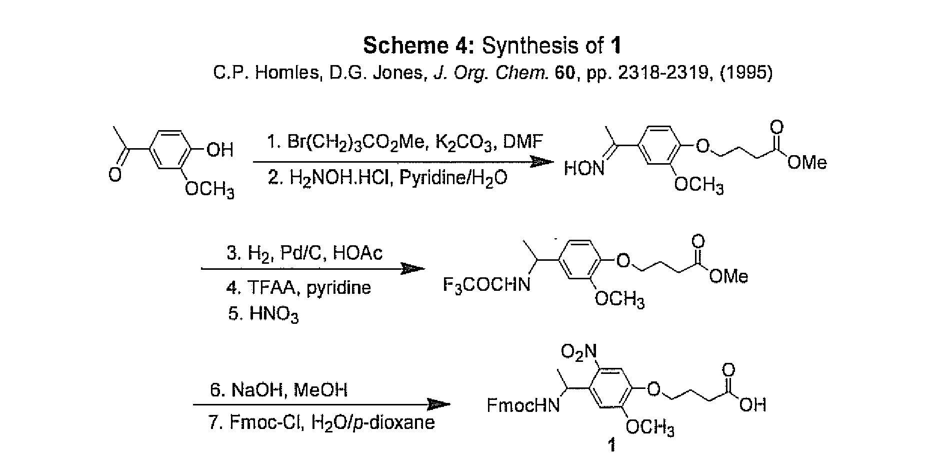

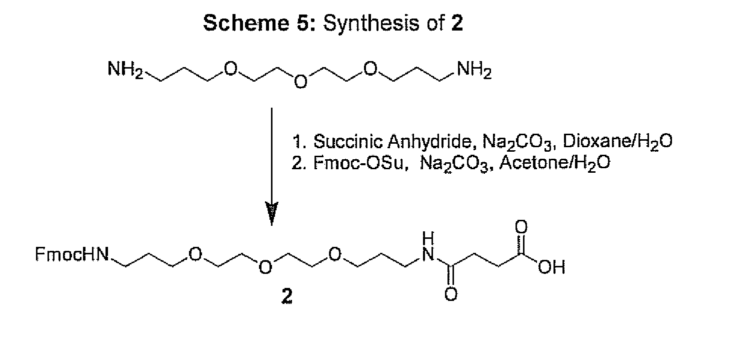

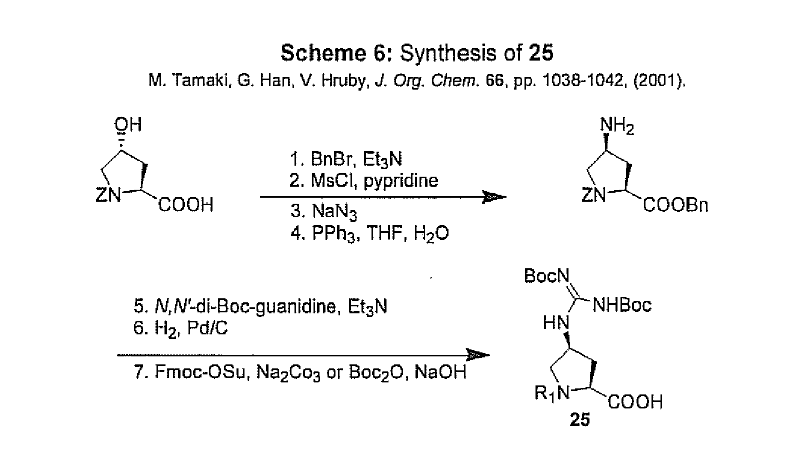

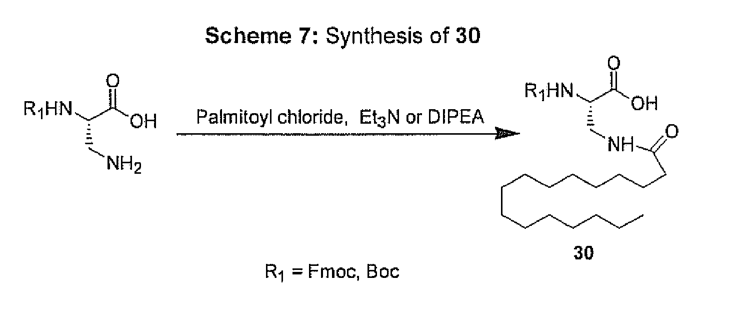

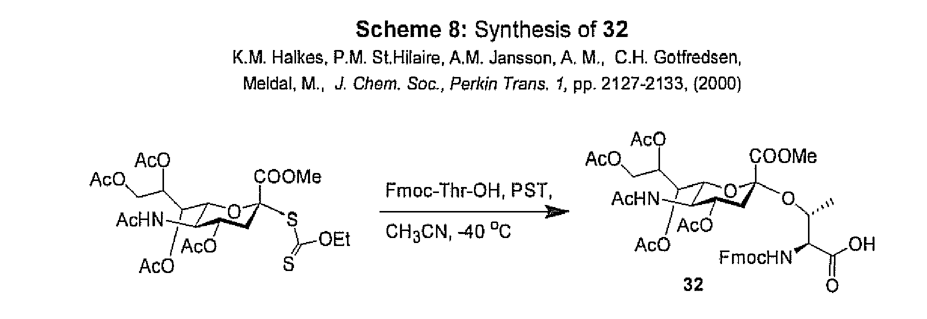

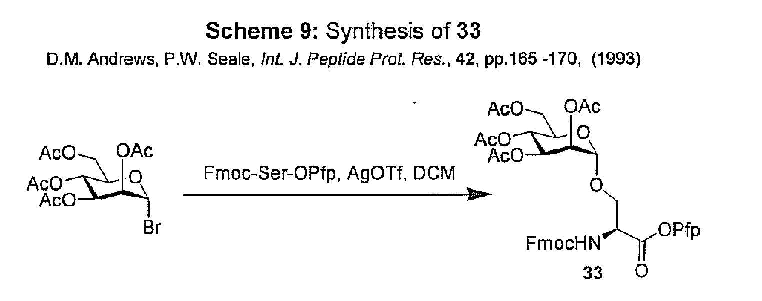

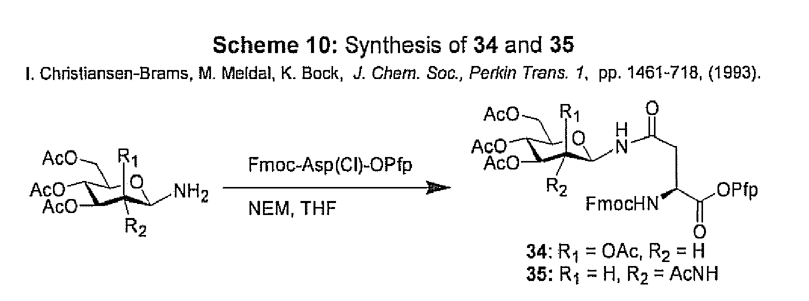

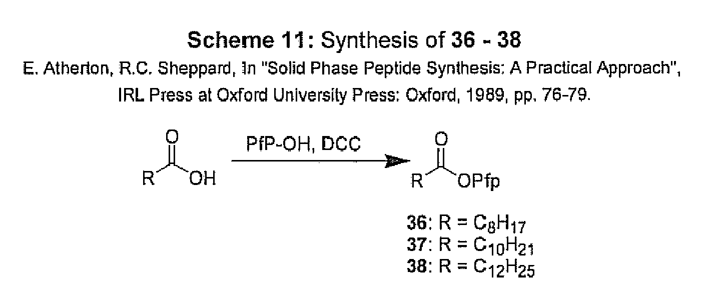

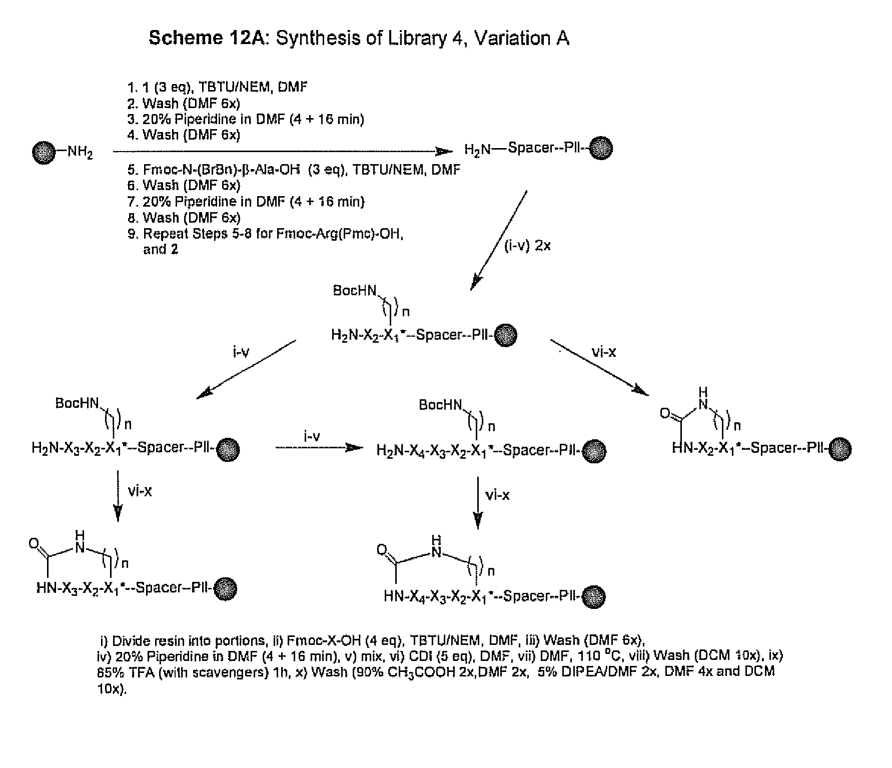

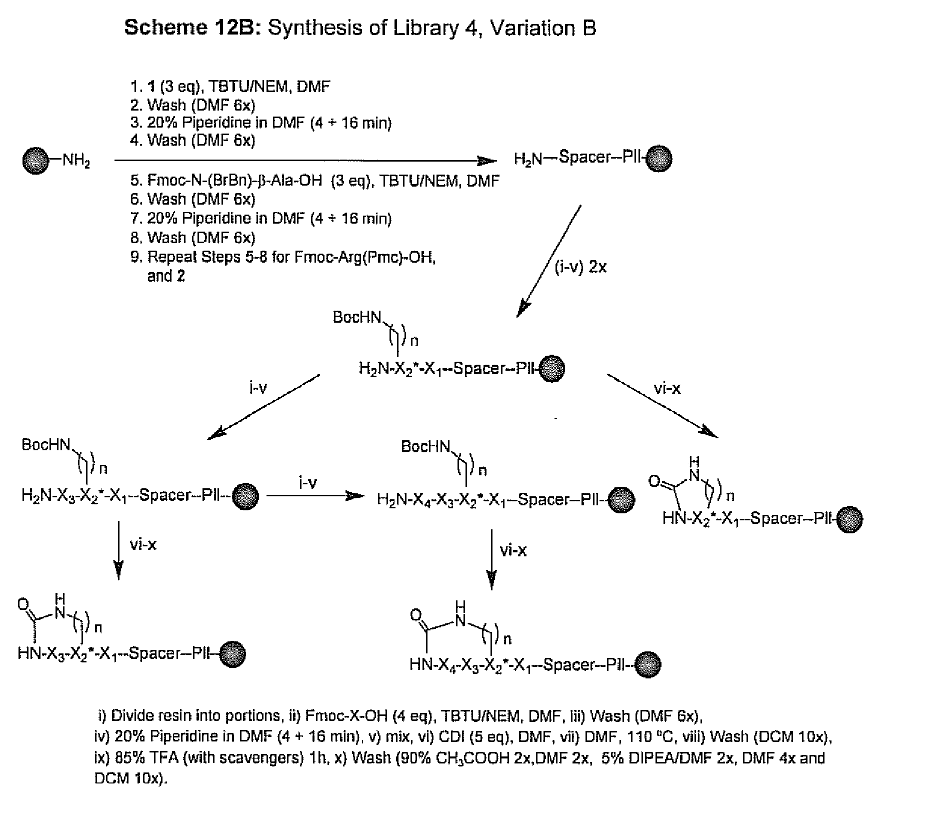

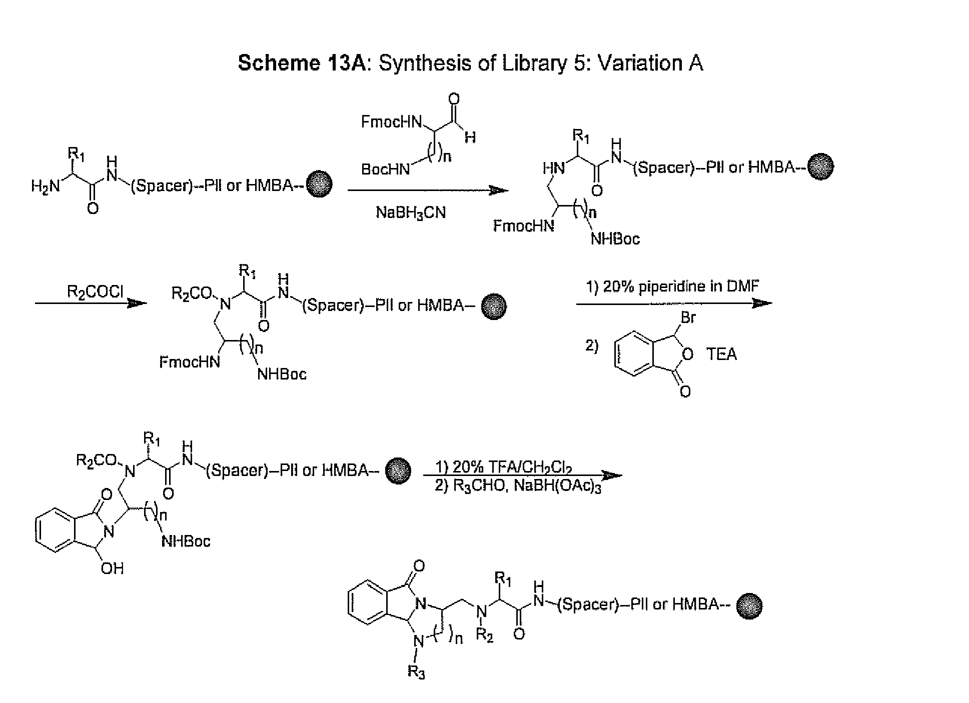

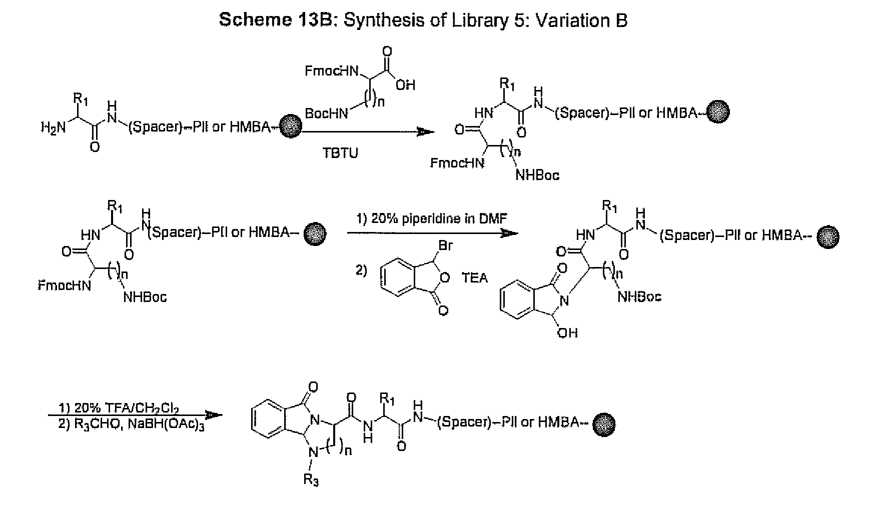

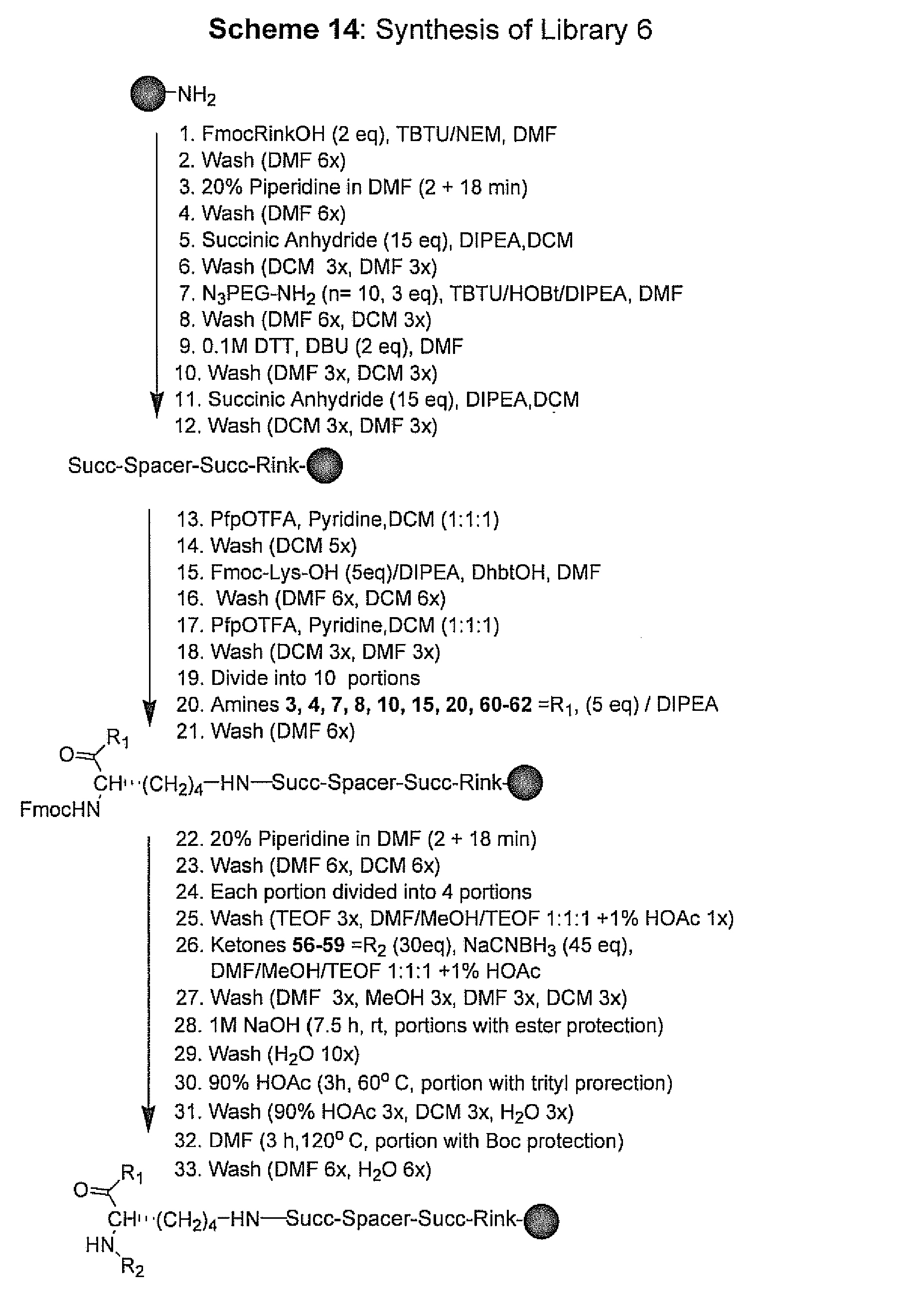

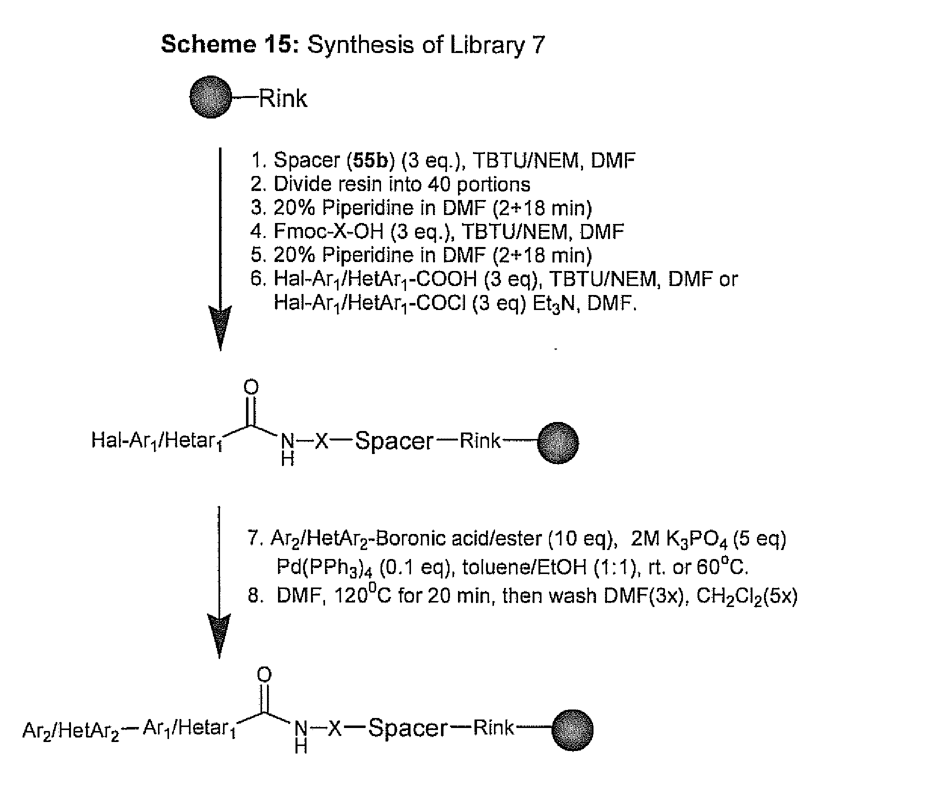

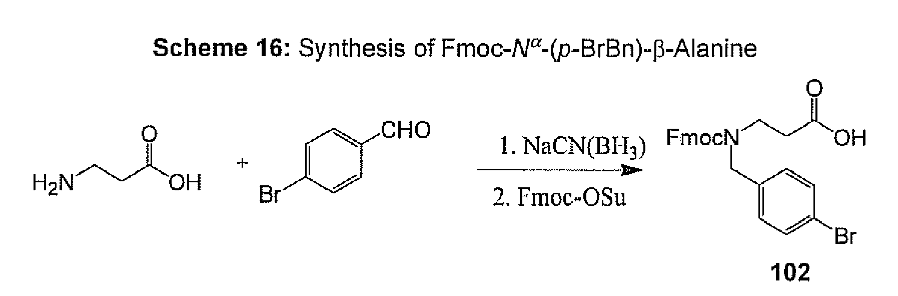

### Example 1

### Syntheses of N-(N'-Fmoc-13-amino-4,7,10-trioxa-tridecyl)-succinamic acid (2)

*N*-(*N'*-Fmoc-13-amino-4,7,10-trioxa-tridecyl)-succinamic acid (2), shown above in Table 1, was prepared as shown in Scheme 5.

4,7,10-Trioxa-1,13-tridecanediamine (5 g, 22.7 mmol, 5 mL) was dissolved in a solution of Na₂CO₃ (7 g) in H₂O (50 mL). Succinic anhydride (2.5 g, 2.5 mmol) in dioxane (50 mL) was added dropwise. The solution turned misty, then into a suspension. It was stirred at room temperature for 24 hours, then heated at 80 °C for another 1 hour. Solvent was removed under vacuum. The residue was treated with 1 N NaOH (200 mL) and extracted with DCM (2x 100 mL). The aqueous phase was separated, acidified to pH 1 with 1 N HCl, extracted with DCM (2×100 mL), then neutralized with NaHCO₃ to pH 7.

The crude material was dissolved in 50% acetone/H₂O (120 mL) and Na₂CO₃ (5 g) was added. Fmoc-OSu (7.5 g, 22.3 mmol) was added in portions over I hour while pH was kept between 9-10 by addition of I M Na₂CO₃. The solution was stirred at room temperature for 18 hours. Acetone was removed under vacuum. The residue was treated with 6 N HCl (60 mL) and extracted with 2×150 mL ethyl acetate. The extract was combined and washed with 2x60 mL brine and dried over Na₂SO₄. Solvent was removed under vacuum and the residue was put on a column. Chromatography twice, first with ethyl acetate:hexane (2:1), then DCM/MeOH (3:1) gave pure compound as oil (3.52 g, 29%). The resulting compound (2) showed the following characteristics:
¹H NMR (CHCl₃, 5) 7.76 (d, J=7.2Hz, 2H), 7.60 (d, J=7.2Hz, 2H), 7.29-7.43 (m, 4H), 4.40 (m, 2H), 4.23 (m, 1H), 3.46-3.62 (m, 14H), 3.26-3.35 (m, 4H), 2.66 (m, 2H), 2.48 (m, 2H), 1.75 (m, 4H). ¹³C NMR (CDCl₃, δ) 175.1, 172.3, 156.5, 143.7, 140.9, 127.4, 126.8, 124.8, 119.7, 70.0, 69.7, 69.6, 69.2, 68.8, 66.1, 46.9, 38.5, 37.5, 30.5, 29.6, 29.1, 28.5. ES-MS: calcd for C₂₈H₃₈N₂O₈ [M + H]⁺= 543.26, found: 543.18.

### Example 2

### Synthesis of (2S,4S)-N^{α}-Fmoc-4-N,N'-di-Boc-guanidinoproline (25a) and

### (2S, 4S)-N^{α}-Boc-4-N,N'-di-Boc-guanidinoopoline (25b)

(2S, 4S)-*N*^{α}-Fmoc-4-*N,N'*-di-Boc-guanidinoproline **(25a)** and (2S, 4S)-*N*^{α}-Boc-4-*N*, *N'*-di-Boc-guanidinoproline (25b) shown above in Table 2, were prepared from Z-Hyp-OH according to literature procedure described in Tamaki et al., 2001, J. Org. Chem. 66: 1038-1042), as shown in Scheme 6.

### Example 3

### Synthesis of Fmoc-Dapa(Pal)-OH(30a)

Fmoc-Dapa (PAL)-OH (30a) as shown above in Table 2, was prepared as shown in Scheme 7.

Fmoc-Dapa-OH (500 mg, 1.53 mmol) and diisopropylethylamine (780 mg, 6 mmol, 1 mL) were dissolved in DCM (20 mL). Palmitoyl chloride (420 mg, 1.53 mmol, 0.46 mL) was added drop-wise with stirring using a syringe. The suspension slowly became clear. After stirring at room temperature for 2 hours, the solution was concentrated under vacuum. The residue was purified by flash chromatography with DCM:EtOH (10:1) to give pure product (800 mg, 98%) as white powder:
¹H NMR (CHCl₃, δ) 7.68 (m, 2H), 7.49 (m, 2H), 7.19-7.33 (m, 4H), 4.27 (br, 2H), 4.01 (m, 1H), 3.62 (br, 1H), 2.10 (br, 2H), 1.44 (br, 2H), 1.17 (m, 28H), 0.8 (m, 3H). ¹³C NMR (CDCl₃, δ) 176.4, 157.1, 144.1, 143.9, 141.7, 141.6, 128.1, 127.5, 126.2, 125.5, 120.4, 67.7, 47.5, 42.3, 36.7, 32.3, 30.1, 30.0, 29.9, 29.8, 29.6, 23.1, 14.5.

### Example 4

### Synthesis of Boc-Dapa(Pal)-OH (30b)

Boc-Dapa (Pal)-OH (30b) as shown in Table 2, was prepared as shown in Scheme 7.

Boc-Dapa-OH (150 mg, 0.73 mmol) and triethylamine (114 mg, 1 mmol, 0.07 mL) was dissolved in THF (30 mL). Palmitoyl chloride (137 mg, 0.5 mmol, 0.15 mL) was added through a syringe. The solution was stirred at room temperature for 2 hours, then concentrated under vacuum. The residue was purified by flash chromatography with DCM:EtOH (10:1) giving 128 mg (5 1 %) of pure product as white powder: ¹H NMR (CDCl₃, δ) 5.38 (m, 2H), 3.15-3.45 (m, 12H), 2.01-2.10 (m, 4H), 1.18-1.60 (m, 26H). ¹³C NMR (CDCl₃, δ) 173.9, 157.2, 134.4, 79.7, 0.7, 37.9, 36.6, 28.7, 28.6, 26.6, 25.4.

### Examples 5

### Synthesis of Library 1

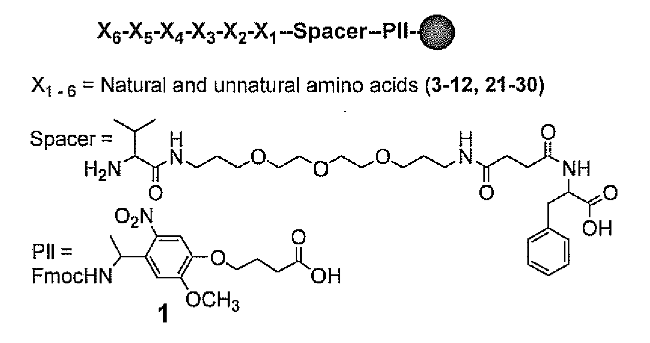

The synthetic scheme for building Library 1 having the structure X₆X₅X₄X₃X₂X₁ (X= a natural or unnatural amino acid) [SEQ ID NO: 2] is shown above as in Scheme 1. Library 1 was prepared on PEGA₄₀₀₀ resin (1 g, 0.12 mmol/g; 300-500 µm beads) using the ladder synthesis method, as previously described in St. Hilaire et al., 1998, J. Am. Chem. Soc.120: 13312-13320). Since the library was not designed for a particular class of proteins, the building blocks used were chosen arbitrarily but such that as many functional groups as possible were presented in the side chains: e.g. carboxylic acids, amines, indoles, pyridines, aliphatics, aromatics, imidazoles, hydroxyls, and the like. Library 1, X₆X₅X₄X₃X₂X₁, where X= a natural or unnatural amino acid, was produced using building blocks 3-12 and 21 - 30 [SEQ ID NO:
1]. The building blocks are as shown above in Tables 1 and 2.
To produce Library 1, as shown in Scheme 1, a photolabile linker, P11 (1) (3 equivalents) was coupled to the resin beads under TBTU activation. A photolabile linker was chosen because it is stable to a wide variety of conditions and can be readily cleaved to yield a product that does not require further purification before MS analysis. A spacer molecule composed by sequential coupling of Fmoc-Phe-OH, spacer (2) and Fmoc/Boc-Val-OH after TBTU preactivation was then added. The spacer molecule is used to enable the identification of the ligand using MALDI-TOF MS because it increases the mass of the ligand fragments to over 600 mu, i.e. away from the matrix peaks. The spacer was designed to have few or no interactions with any proteins in the mixture.

The six randomized positions of the library were generated using the split and mix approach described in Furka et al., 1991, Int. J. Peptide Protein Res., 37: 487-493 and Lam et al., 1991, Nature, 354: 82-84 in a 20-well custom-made (2.0 mL capacity) multiple column library generator. During the library synthesis, 10 % of the growing oligomer was capped using the Boc-protected amino acid analog of the Fmoc Building block. Therefore, a mixture of the Fmoc- and Boc-protected amino acid (90% Fmoc and 10% Boc, 4 equivalents) from stock solutions was activated with TBTU/NEM for 6 minutes and then added to the wells. Coupling times ranged from 4 to 12 hours and reaction completion was determined using the Kaiser test as described in Kaiser et al, 1970, Anal. Biochem., 34: 595-598. After each coupling the resin was pooled, mixed, and divided prior to Fmoc removal. After each coupling and deprotection step, the resin was washed with DMF (10x). After completion of synthesis, the Fmoc group was removed by treatment with 20% piperidine in DMF for 4 + 16 minutes. The resin was washed with DMF (6 x 2 minutes), CH₂Cl₂(10x 2 minutes) and then the acid labile side chain protecting groups were removed by treatment with 85% TFA containing 2% triisopropylsilane, 2.5% EDT, 5% thioanisole, 5% water for 1 hour. Then the resin was washed with 90% aqueous acetic acid (4 x 5 minutes), DMF (2 x 2 minutes), 5% DIPEA in DMF (2 x 2 minutes), DMF (4 x 2 minutes), CH₂Cl₂ (10 x 2 minutes) and finally methanol (5 x 2 minutes), before being dried by lyophilization overnight.

### Example 6

### Synthesis of Library 2

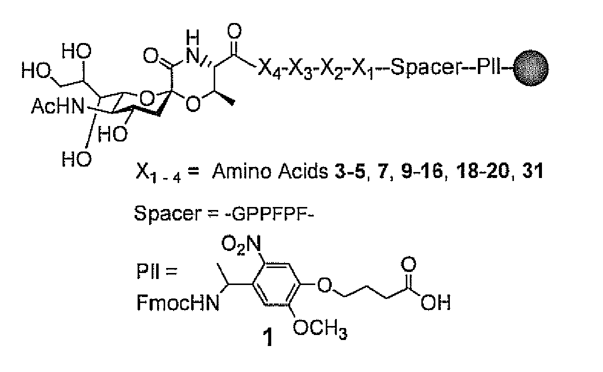

Library 2 containing the peptide X₄ X₃ X₂ X₁ where X is any amino acid of 3-5, 7, 9-16, 18-20, or 31, as shown in Tables 1 and 2 [SEQ ID NO: 3], was synthesized according to Scheme 2, shown above (large black dots represent a resin bead). Library 2 was synthesized on PEGA₁₉₀₀ resin (600 mg, ca. 250.000 beads, 300-500 µm, 0.22 mmol/g loading). The photolabile linker, P11 (1) (3 equivalents) under TBTU activation was first coupled to the resin followed by the peptide spacer, GPPFPF [SEQ ID NO: 4], in a syringe, using standard Fmoc-Opfp methodology, for example, as described in Atherton et al., 1989, In: "Solid Phase Peptide Synthesis: A Practical Approach", IRL Press at Oxford University Press: Oxford, pp. 76-79. The photolabile linker was chosen because it is stable to a wide variety of conditions and can be readily cleaved to yield a product that does not require further purify-cation before MS analysis. The peptide spacer molecule, GPPFPF, is useful to enable the identification of the ligand using MALDI-TOF MS because it increases the mass of the ligand fragments to over 600 mu, i.e. away from the matrix peaks. The spacer was designed to have few or no interactions with carbohydrate binding proteins. Library 2 was originally designed for binding to carbohydrate binding proteins particularly sialic acid binding proteins, hence the fixed sialic acid threonine lactam in position 5. The building blocks comprising the four randomized positions were chosen from natural amino acids presenting diverse functionalities in the side chain functional group: for example, amides, indoles, aliphatics, aromatics, imidazoles, hydroxyls, and the like. The four randomized positions of Library 2 were generated using the split and mix approach described, for example, in Furka et al., 1991, Int. J. Peptide Protein Res., 37: 487-493) and Lam et al., 1991, Nature, 354: 82-84) in a 20-well custom-made (2.0 mL capacity) multiple column library generator. During the library synthesis, 10 % of the growing oligomer was capped using the Boc-protected amino acid analog of the Fmoc Building block. For the coupling of non-glycosylated amino acids, a mixture of the Fmoc- and Boc-protected amino acid (90% Fmoc and 10% Boc, 4 equivalents) from stock solutions was activated with TBTU/NEM for 6 minutes and then added to the wells. Building blocks 3-5, 7, 9-16 and 18-20, as shown in Table 1 above, were used. Coupling times ranged from 4 to 12 hours and reaction completion was checked by the Kaiser test (Kaiser et al, 1970, Anal. Biochem., 34: 595-598).

After each coupling, the resin was pooled, mixed and divided prior to Fmoc removal. After each coupling and deprotection step, the resin was washed with DMF (6x). Building block 32 (2 equivalents), shown in Table 2, was activated with TBTU/NEM for 5 minutes, and then added to all wells overnight. The Fmoc group was removed by treatment with piperidine (4 + 16 minutes) and the resulting product immediately cyclized to form the lactamized analogue, as evidenced by a negative Kaiser test. The Boc groups were removed by treatment with 10% TFA in DCM for 30 minutes and the carbohydrate acetyl protecting groups were removed by hydrolysis with hydrazine hydrate (55 µL) in methanol (1 ml) for 6 hours, followed by washing with methanol (3 x 2 minutes), CH₂Cl₂ (3 x 2 minutes), methanol (3 x 2 minutes), H₂O (3 x 2 minutes), toluene (3 x 2 minutes), and finally diethyl ether (3 x 2 minutes).

### Example 7

### Synthesis of Library 3

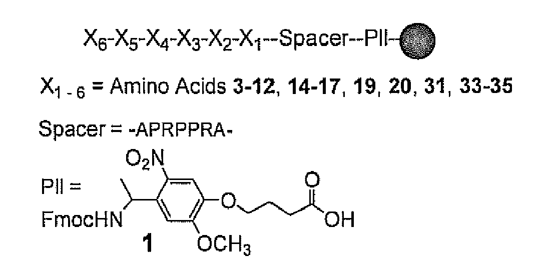

Library 3, a glycopeptide library containing the peptide X₆ X₅X₄ X₃ X₂ X₁ where X is any amino acid of **3-12,14-17, 19, 20,** or 31, (shown in Tables 1 and 2) [SEQ ID NO: 5], was synthesized on PEGA₁₉₀₀ resin (1 g, 300-500 µm beads, 0.23 mmol/g loading) according to Scheme 3. A glycopeptide library was chosen because glycopeptides can mimic oligosaccharides and therefore bind to carbohydrate binding proteins.

The glycopeptides were attached to the resin *via* photolabile linker, P11 (1), and the peptide mass spacer, APRPPRA [SEQ ID NO: 6], was synthesized in a syringe prior to library generation. The photolabile linker was chosen because it is stable to a wide variety of conditions and can be readily cleaved to yield a product that does not require further purification before MS analysis. The peptide spacer molecule, APRPPRA, was used to enable identification of ligand using MALDI-TOF MS, as it increases the mass of the ligand fragments to over 600 mu, away from the matrix peaks, and helps ionization of the fragments because of the arginine content. The spacer was designed to have few or no interactions with carbohydrate binding proteins.

Library 3 was designed for binding to carbohydrate binding proteins, particularly glucose/mannose specific proteins. The building blocks comprising the six randomized positions were chosen from natural amino acids presenting diverse functionalities in the side chain functional group: for example, carboxylic acids, amides, indoles, aliphatics, aromatics, imidazoles, hydroxyls, and the like, as well as glycosyl amino acids bearing mannose and N-acetylglucosamine residues. Natural amino acids were capped with the Boc-protected analog of the Fmoc amino acid while the glycosyl amino acids were capped using aliphatic encoding tags. Amino acids **3-12, 14-17, 19, 20, 31,** glycosylated amino acids 33-20, and aliphatic encoding tags 36-38 as shown above in Tables 1 and 2 were used. Randomized positions in the library were generated using the split synthesis approach (Furka, et al., 1991, Int. J. Peptide Protein Res., 37: 487-493 and Lam et al., 1991, Nature, 354: 82-84) using a 20 well custom-made (2.0 mL capacity) multiple column synthesizer.

After each coupling, the resin was pooled, mixed and divided before Fmoc removal with 20% piperidine (2+18 minutes). After each acylation and deprotection step, the resin was washed with DMF (6x). For the coupling of non-glycosylated amino acids, a mixture of the Fmoc- and Boc-protected amino acids (90% Fmoc and 10% Boc, 4 equivalents total) from stock solutions was activated with TBTUINEM for 5 minutes and then added to the wells. For the coupling of glycosylated amino acids, 3 equivalents of a mixture of the glycosylated amino acid (67%) and the aliphatic encoding tag (33%) was activated with Dhbt-OH and directly added to the wells (33 and 36, 35 and 37, 34 and 38). Coupling times ranged from 4 to 12 hours and reaction completion was checked by the Kaiser test (Kaiser, et al, 1970, Anal. Biochem., 34, pp.595-598).

After the final coupling, side chain protecting groups were removed using a cocktail consisting of TFA 87.5%, EDT 2.5%, thioanisole 5% and H₂O 5% for 2.5 hours. Then, the resin was washed with 90% aqueous acetic acid (4 x 5 minutes), DMF (2 x 2 minutes), 5% DIPEA in DMF (2 x 2 minutes), DMF (4 x 2 minutes), CH₂Cl₂(10 x 2 minutes), and finally methanol (5 x 2 minutes), before being dried by lyophilization overnight. Carbohydrate acetyl protecting groups were removed by hydrolysis with hydrazine hydrate (55 µL) in methanol (1 ml) for 6 hours, followed by washing with methanol (3 x 2 minutes), CH₂Cl₂ (3 x 2 minutes), methanol (3 x 2 minutes), H₂O (3 x 2 minutes), toluene (3 x 2 minutes), and finally diethyl ether (3 x 2 minutes).

### Examples 8

### Resynthesis of Active Ligands

Ligands for solid phase protein binding were resynthesized on PEGA₄₀₀₀ for the analysis of Myocyte protein and *E. coli* membrane proteins in the Examples below and on PEGA₆₀₀₀ for the Six-protein mix in the Example below, using standard Fmoc Solid Phase Peptide Synthesis methods as described, for example, in Atherton et al., 1998, In: Solid Phase Peptide Synthesis: A Practical Approach, IRL Press at Oxford University Press: Oxford, pp. 76-79.

### Example 9

### Proliferation of Myocytes

Myocytes were prepared from 1 to 5 day old neonatal Wistar rats (University of Copenhagen) according to literature procedure described in Busk et al., 2002, Cardiovasc. Res., 56: 64-75 and plated into eight P10 0 culture plates at 6 million cells/plate. Cells were grown at 37 °C and 5% CO₂ humidity in serum free Modified Eagle Media (MEM). After 2 days, the adherent cells were washed at room temperature with serum free MEM (2x) and fresh MEM was added. To four of the plates, 10 µM phenylephrine (PE) was also added. Cells were grown for two more days and then harvested as described below. Cells treated with PE were significantly enlarged at time of harvest.

### Example 10

### Proliferation of Escherichia coli DH 5α-136

A 400 mL culture of *Escherichia coli* DH 5α-136 (Carlsberg Research Center Collection) was prepared according to the procedures described in Hanahan, 1985, In: DNA Cloning, Vol 1, Glover, D., ed., IRL Press Ltd, pp. 109-135. Cells were grown from innocula in LB media at 37°C for 5 to 6 hours and harvested in the latter log phase at an optical density of 0.8 (600 nm) by centrifugation (10,000 rpm for 10 minutes at 4 °C). The media containing extracellular protein was retained and the pellet washed once with PBS, pH 7.6 (cellular weight = 2.22g).

### Example 11

### Preparation of Labeled Protein From PE-induced and Basal Myocytes

Protein was extracted from myocytes prepared as described above for Example 9, using a new procedure modified from existing protocols, primarily: Arnott, et al., 1998, Anal. Biochem. 258: 1-18. The media was removed from plates and adhered cells were treated for 10 minutes with ice cold phosphate buffer (0.25 mL, 10 mM, pH 7.5, augmented with 0.15 M NaCl 60 mM Benzamidine HCl, 5 mM EDTA, 10 µg/mL E-64, 10 µg/mL Leupeptin, 10 µg/mL Pepstatin A, and 1 mM PMSF). The cells were scraped off the plates and then lysed (on ice) in a sonicator (2x) using 10 seconds off/10 0 seconds on cycles.

The resulting suspension was augmented with CHAPS, DTT, and urea to a final concentration of CHAPS (1 % w/v), DTT (5 mM), and urea (8 M). After 10-15 minutes on ice, the solution was centrifuged for 10 minutes at 15,000 rpm at 4 °C. The supernatant was removed and protein content quantified using the NanoOrange Protein test (Molecular Probes, Eugene, Oregon): Total protein recovered: 70.5 µg for PE-treated cells and 60 µg for basal cells.

Fluorescent dye Oregon Green 514 (OR) (Molecular Probes) was used to label the healthy/basal cells while Rhodamine Red (RR) (Molecular Probes) was used to label the PE-treated cells. The labeling procedures were carried out according to the manufacturer's protocol. The protein solutions (0.25 mL, 50.4 µg for PE cells and 0.25 mL, 42.8 µg for basal cells) were dialyzed against a solution of 10 mM phosphate buffer, 0.15 M NaCl, pH 7.5 and then 1 M NaHCO₃ (0.025 mL) added to a final pH of 8.5. 10 µL of dye in dry DMF (10 mg/mL) was added and the sample stirred at room temperature for 2 hours. The protein solution was dialyzed extensively against 10 mM phosphate buffer, 0.15 M NaCl, pH 7.5, to remove excess dye. In another case, fluorescent dye Cyanine 3 (Cy 3) (Molecular Probes) was used to label the healthy/basal cells while Cyanine 2 (Cy 2) (Molecular Probes) was used to label the PE-treated cells. The labeling procedures were carried out according to the manufacturer's protocol. The protein solutions (0.35 mL, 100 µg for PE cells and 0.41 mL, 100 µg for basal cells) were dialyzed against a solution of 10 mM phosphate buffer, 0.15 M NaCl, pH 7.5 and then 1 M NaHCO₃ (0.03-0.04 mL) was added to obtain a final pH between 8.0-9.0. For efficient labeling, 400 pmol fluor (5 µL solution was prepared by addition of 2 µL of reconstituted fluorophore solution [I nmol/ µL] and 3 µL of dry DMF) was used to label 50 µg of protein. The fluorophore and the protein solution are mixed thoroughly by vortexing. The samples are then centrifuged briefly in a microfuge and left on ice for 30 min in the dark. To stop the reaction, 10 mM lysine (1 µL) was added to the samples and the samples were mixed by vortexing and centrifuged briefly in a microfuge. The samples were left on ice for 10 min in the dark. The labeled protein solutions were dialyzed extensively against 10 mM phosphate buffer, 0.15 M NaCl, pH 7.5, to remove excess dye. Labeled samples were stored for at - 70 °C in the dark until further use.

### Example 12

### Preparation of Labeled Protein From E. coli (Extracellular protein)

Extracellular protein-containing supernatant obtained from cultured *E. coli* cells prepared as described above for Example 10 (400 mL broth), was concentrated to 80 mL at 4 °C in an Amicon concentrator using a 6,000 Da molecular weight cutoff (mwco) membrane (Millipore, Bedford, Mass.). The concentrate was further concentrated to 25 mL (protein concentration = 94.5 mg/mL) using Amicon microconcentrators centrifuged at 3000 rpm for 1.5 hours. The concentrate was then dialyzed (mwco 10,000 Da) extensively against 10 mM phosphate buffer, pH 6.8 augmented with 0.15 M NaCl, 1mM ZnCl₂, 1 mM MnCl₂, 1 mM CuCl₂, 1 mM MgSO₄, 1 mM CaCl₂, and 5 mM DTT at 4 °C. After the dialysis, a protease inhibitor, PMSF, was added to a final concentration of 1mM.

The extracellular protein was labeled using an amine reactive dye, succinimidyl N-methylanthranilate (Molecular Probes), using procedures essentially as described above for labeling of myocyte protein in Example 11. 50 mg of the dye in dry DMF (5 mL) was added dropwise with stirring to the extracellular protein solution (25 mL) adjusted to pH 8.35 by the addition of 1 M NaHCO₃ (2.5 mL). The reaction was stirred at room temperature for 2 hours. The reaction was stopped by the addition of 1 M hydroxylamine hydrochloride and stirring continued for another hour. The solution was dialyzed overnight (10,000 Da mwco) at 4 °C against 10 mM phosphate buffer, pH 6.8 containing, 1mM ZnCl₂; 1 mM MnCl₂; 1 mM CuCl₂; 1 mM MgSO₄; 1 mM CaCl₂.

### Example 13

### Preparation of Labeled Protein from E. coli (Membrane Proteins)

Isolation of *E. coli* membrane proteins was achieved through modification of published literature procedures: Auer, et al., 2001, Biochemistry, 40:6628-6635, and Molloy, et al., 2000, Eur. J. Biochem. 267:2871-2881. After incubation of *E. coli* cells for 2 days, washed cells were scraped from plates and centrifuged as described above for Example 10. The cell pellet (approximately 1 g) was suspended in 50 mM Tris HCl, pH 7.5 and pressed (2x) in a French Press at 1500 Psi. The resulting suspension was centrifuged at 2500 x g for 10 minutes. The ice cold supernatant was diluted with 2.5 ml of ice-cold 0.1 M sodium carbonate buffer 11 and the solution stirred on ice for 1 hour. Ultracentrifugation was then carried out at 115, 000 x g for I to 1.5 hours at 4°C, yielding Pellet 1 and Supernatant 1. The membrane pellet 1 was resuspended in 50 mM Tris HCl, pH 7.5 and the pellet was recollected after centrifugation for an additional 20 minutes at 115,000 x g, yielding Pellet 2 and supernatant 2. Pellet 2 was solubilized in 50 mM Tris HCl, pH 7.5 containing 10 mM imidazole, 0.5 mM PMSF, 20 % glycerol, and 1 % Dodecyl Maltoside (DDM) or 33 mM Octyl Glucoside (OG) for 30 minutes at 4 °C. The suspension was then centrifuged for 10,000 g for 30 minutes. The pellet and supernatant obtained were labeled Pellet 3 and supernatant 3.

Protein content was determined by checking the absorbance of the protein at 280 nm. The protein concentration in the DDM sample was 0.93 mg/mL, while in the OG sample 0.07 mg/mL protein was obtained. The protein was dialyzed against 10 mM PBS buffer, pH 6.8, containing 1 mM ZnCl₂, I mM CaCl₂, 1 mM MnCl₂, and 1 mM MgSO₄, for 1 to 2 hours against three time buffer changes.

The protein was labeled with amino reactive succinylanthranilate dye (blue) DDM (0.49 mg dye) and OG (0.03 mg dye) according to the same protocol used in the extracellular labeling described above for Example 12. The labeling stopping reaction (hydroxylamine hydrochloride addition) was not used in this case, to avoid dilution of the protein. The mixture was dialyzed overnight against 10 mM PBS buffer, pH 6.8 containing 0.01 mM ZnCl₂, 0.01 mM CaCl₂, 0.01 mM MnCl₂, and 0.01 mM MnSO₄, against a three time buffer change.

### Example 14

### Preparation of Labeled Random Mixture of Six Proteins

The following proteins, Concanavalin A (400 µg), Lens culinaris lectin (390 µg), Pisum sativum lectin (300 µg), Wisteria floribunda lectin (260 µg), bovine serum albumin 470 µg, and glyceraldehyde-3-phosphate dehydrogenase, (450 µg), were solubilized in 10 mM PBS augmented with 1 mM CaCl₂, (500 µL) to which was added 1 M Na₂CO₃ (50 µL) for a final pH of 8.3. The protein mixture was labeled with Alexa 488 dye (Molecular Probes) according to the manufacturer's protocol. After stopping the reaction with 1.5 M hydroxylamine (15 µL) the excess dye was removed by washing (6 x 1 mL) the protein mixture in a centricon YM-10 0 spun at 5000 x g with 10 mM PBS, pH 6.9, augmented with 1 mM CaCl₂ and 1 mM MnCl₂. The protein mixture was washed until the filtrate was no longer fluorescent.

### Example 15

### Solid Phase Screening of Library 1 with Labeled Myocyte Proteins

Ligand library 1 (200 mg), prepared as described for Example 5, was transferred to a syringe fitted with a stop-valve and the ligand-beads were washed for 10 minutes (3x) with 10 mM phosphate buffer, pH 6.8, supplemented with 0.15 M NaCl, 1 mM Ca²⁺, 1 mM Zn²⁺, 1 mM Mn²⁺, 1 mM Cu²⁺, and 1 mM Mg²⁺ (3 mL). The ligand-beads were treated with a 1% BSA solution for 30 minutes, then washed with buffer (1x). A mixture of labeled myocyte proteins, including both PE-induced protein (138 µL, 40 µg) and basal protein (167 µL, 40 µg) obtained as described above for Example 11, was prepared in 1.2 mL buffer, and added to the ligand library in the syringe. The proteins and ligand library were incubated at room temperature for 16 hours. The library was then washed with buffer for 5 minutes then with water for 3 x 5 minutes. The library was examined under a fluorescence microscope and brightly fluorescent red, green, and yellow beads (yellow indicative of both dyes red and green binding; the majority of the beads) were present, as well as unlabelled beads. The fluorescent beads were separated and retained for analysis.

### Example 16

### Solid Phase Screening of Libraries with Labeled E. coli Membrane Proteins

Ligand Library 2 (200 mg) prepared as described above for Example 6, was washed in a 2 ml column (3x10 minutes) with 10 mM PBS buffer, pH 6.8 containing 1 mM ZnCl₂, 1 mM CaCl₂, 1 mM MnCl₂, and 1 mM MgSO₄. To the washed library was added a 1 % BSA solution (600 µl), and the BSA incubated with the library for 30 minutes to avoid non-specific binding. The ligand library was then washed, and *E. coli* labeled membrane protein, prepared as described above for Example 13, was added (0.1 ml). The ligand library and protein mixture was incubated overnight at room temperature. The next day, the beads were washed very well, 5 x 10 minutes with buffer, and then with water (3 x 5 minutes). The fluorescence intensity of the beads was analyzed and beads were manually sorted under a fluorescence microscope to obtain 37 fluorescent beads containing protein-ligand binding pairs were obtained for further analysis.

### Example 17

### Solid Phase Screening of Libraries with Labeled Random Mixture of Six Proteins

Ligand Library 3 (150 mg) prepared as described above for Example 7, was washed in a 5 mL syringe (3x10 minutes) with 10 mM PBS buffer, pH 6.8 containing 1 mM CaCl₂ and 1 mM MnCl₂. A solution of 1 % BSA (600 µL) was added to the washed library and incubated with the library for 30 minutes to avoid non-specific binding. The six-protein mixture prepared as described for Example 14, in 10 mM PBS buffer, pH 6.8 containing 1 mM CaCl₂ and 1 mM MnCl₂, was then added to the ligand library and incubated for 3 hours and 15 minutes. The beads were then washed very well (5 x 10 minutes) with buffer and then water (3 x 5 minutes). The fluorescence intensity of the beads was analyzed and beads were manually sorted in batches under the fluorescence microscope. 112 fluorescent beads containing protein-ligand binding pairs were retained for analysis.

### Example 18

### Sorting of Differentially Fluorescent Labeled Protein-Ligand Beads

The fluorescent ligand library beads containing bound protein obtained as described in Example 15were sorted using a COPAS (250) NF Bead Sorter (Union Biometrica, Somerville, Mass.) equipped with a single flourescence emission. Beads emitting fluorescence of only one color contain proteins from one physical state, e.g. proteins expressed only in the PE-induced hypertrophic state (red) or proteins expressed only in the basal state (green). Since the instrument measures one fluorescence emission at a time, beads containing one type of color fluorescence were first sorted and then beads were resorted for emissions from the other fluorescence color. The library was first sorted such that all beads containing significant green fluorescence, basal proteins from healthy myocytes, were isolated. The beads were then resorted to exclude those containing both green and red fluorescence (beads containing proteins from both PE-induced and basal cells). The remaining beads, containing only green fluorescence (proteins from basal cells), were sorted into brightly and less brightly fluorescent beads, to give an indication of either the strength of binding of the ligand to a particular protein or the amount of protein present in the sample binding to the ligand. The beads not containing any green florescence were resorted to isolate those with the highest red fluorescence, for example, beads containing the best ligands binding to proteins expressed only in PE-treated (hypertrophic) cells. Alternatively, the fluorescent ligand library beads containing bound protein obtained as described in Examples 47, 48, 49 and 51 were sorted using a COPAS (250) NF Bead Sorter (Union Biometrica, Somerville, Mass) equipped with a dual detector. Both the cyanine dyes used in the labeling procedure, namely Cy 2 (emission wavelength- 510 nm) and Cy 3 (emission wavelength-570 nm) were simultaneously excited at 514 nm. The bead selection window was then set-up such that all beads containing significant green fluorescence and no red flourescence were isolated. The unselected beads were then resorted to obtain beads containing red fluorescence (proteins from basal cells). The selected red and green fluorescent beads were then sorted into brightly and less brightly fluorescent beads, to give an indication of either the strength of binding of the ligand to a particular protein or the amount of protein present in the sample binding to the ligand.

### Example 19

### Identification of Ligands Attached to Fluorescent Beads by MALDI-TOF MS

Ligands can be completely or partially identified by MALDI_TOF-MS whether or not the library is synthesized by ladder synthesis. After sorting automatically or manually, the labeled protein/ligand beads were washed extensively with 0.1% aqueous TFA to remove residual sheath fluid. The ligand is cleaved from the resin bead depending on the type of linker used. In the case where ligand was attached to the resin using a photolabile linker (Libraries 1 2, 3 and 4), the beads were transferred to a stainless steel disc and irradiated with UV light for 1 to 2 hours. Peptide fragments (ladder synthesis) or single compounds were extracted from the bead with 0.5 µL CH₃CN, then 0.5 µL 70% CH₃CN/H₂O. Another 0.5 µL 70% CH₃CN/H₂O was added to the bead, followed immediately by 0.2 µL MALDI matrix α-cyano-4-hydroxycinnamic acid: CHC). In the case of the acid labile Rink linker (Library 7), the compound was released from the bead by treatment with 95% TFA or 30 min. The TFA solution was evaporated and the cleaved compounds were extracted in 2 µL 70 % acetonitrile + 0.1 % TFA. This mixture was then transferred to a stainless steel disc to which 0.4 µL of CHC matrix +1.0 % TFA was added. An additional 0.4 µL of CHC matrix +1.0 % TFA was added to complete sample application. In the case of the base labile linker (Library 5), the compound was relased from the resin bead by treatment with 0.2 µL of 0.05-0.1M NaOH for I h. The solution was neutralized by the addition of an equivalent volume of 0.2 µL of 0.1 N HCl. The mixture containing peptide fragments were transferred to a stainless steel disc to which 0.2 µL of MALDI matrix (α-cyano-4-hydroxycinnamic acid, CHC) was added. The mixture was allowed to evaporate slowly to dryness under a lamp.

The samples thus prepared were used to acquire spectra in the positive reflection mode of a MALDI time-of flight mass spectrometer (Bruker Reflex III, Bruker-Daltonics, Bremen, Germany). A typical analysis employed 100-300 laser shots. If the ligand library had been prepared by ladder synthesis (Libraries 1-4), then the sequence (hence identity) of the ligand compound on the bead was determined using the instrument's automatic Mass. Diff. program that matches the mass difference between mass peaks with the mass of one of the genetically encoded amino acids. In the case of encoding (such as the use of aliphatic encoding tags) and unnatural amino acid building blocks, the Mass. Diff. program was modified so that the mass difference between mass peaks was also matched with the expected mass difference of the tags and the unnatural amino acids. For Libraries prepared without ladder synthesis (Libraries 5 and 7) spectra were also acquired in the positive reflectron mode of a MALDI time-of flight mass spectrometer (Bruker Reflex III, Bruker-Daltonics, Bremen, Germany). A typical analysis employed 200-300 laser shots. The possible identity of the ligand was determined (Narrowed down) by comparing the recorded mass of the ligand with the masses of all possible compounds formed in the library. This comparison was carried out using an in-house created computer program. The precise identity of the ligand can then be confirmed using MS fragmentation as described in Example 41.

### Example 20

### Binding of Differentially Labeled Myocyte Proteins to Identified Ligands

Beads containing 24 different specific ligands identified as specific members of a ligand-protein binding pair in Examples 15, 18, and 19, were placed into 24 wells of a Multiwell filter plate (Multiscreen DV plates, Millipore). Beads containing PEGA₄₀₀₀ resin plus spacer linker with no ligand component were used as a control. Binding was carried out in duplicate with both basal and PE-induced protein, obtained from myocytes and labeled as described above for Example 11. The beads were washed with Millipore water for 3x5 minutes and then with 10 mM PBS buffer (described above for Example 15) for 3x5 minutes under vacuum. PE-induced protein (33 µl) and basal protein (33 µl) were added to each well respectively (10 µg PE and 8 µg Basal protein/well). The plate was covered with aluminum foil and left to incubate at room temperature overnight. The next day the solution containing unbound protein was removed from each well under suction and the beads were washed with Millipore water and 10 mM PBS buffer respectively for 3x5 minutes under vacuum. Fluorescent beads (positive hits) containing ligand-protein binding complexes were observed under a fluorescence microscope and the results documented.

### Example 21

### "Affinity Purification": Binding of Unlabelled Myocyte Proteins to Identified Ligands

Specific myocyte proteins binding to the identified ligands were isolated by affinity purification. In RNase and DNase free tubes, unlabeled PE-induced protein (32 µL, 13. 5 µg) and unlabeled basal protein (49.6 µL, 10.8 µg) were produced, as described in Example 11, and precipitated with acetone (4 volumes) overnight. The pellets were resuspended in Millipore water (PE- 130 µL and BASAL- 220 µL). Six ligands from PE-induced and Basal positive hits, isolated as described above for Example 20, were chosen and washed as above. To each PE-induced ligand-containing well, a 7 µL unlabeled PE-induced protein solution was added. To each basal ligand-containing well, 8.2 µL of unlabeled basal protein solution was added. The mixtures were incubated overnight at room temperature, and the next day wells were washed with Millipore water to remove unbound protein for 3 x 5 minutes, then with 10 mM PBS buffer (as described for Example 15) for 3 x 5 minutes under vacuum.

### Example 22

### Binding of Unlabelled E. coli Membrane Proteins to Identified Ligands

Beads containing 40 different specific ligands that bind *E. coli* protein, the binding ligands isolated by the process described for Example 16 and identified as described for Example 19, were placed into 40 wells of a Multiwell filter plate (Multiscreen DV plates, Millipore). Control beads containing PEGA₄₀₀₀ resin plus the spacer and linker with no ligand compound were used. The binding of unlabeled *E. coli* proteins was carried out in duplicate. The ligand beads were washed with Millipore water for 3x5 minutes and then with 10 mM PBS buffer (as described for Example 15) for 3 x 5 minutes under vacuum. Unlabeled *E. coli* protein, produced as described for Example 13, (400 µg) was added to each well and incubated at room temperature overnight. The next day, the unbound protein solution from each well was removed under suction and the beads were washed with Millipore water and buffer respectively for 3 x 5 minutes under vacuum.

### Example 23

### Affinity Purification of Mixture of Six Proteins

Each of eight ligand glycopeptides and peptides attached to PEGA₆₀₀₀ resin, representative of the positive hits obtained from library screening as described for Example 17, and identified and described by Example 19, were transferred to each of eight 10 mL syringes (4 mL of ligand-resin). The ligand-resin was washed with 10 mM PBS buffer, pH 6.8 containing 1 mM CaCl₂ and 1 mM MnCl₂. A mixture of unlabelled proteins, Glycerol-3-phosphate: BSA: Wisteria floribunda: Lens culinaris: Pisatum sativum in a 4:4:4:1:3:1 weight ratio was dissolved in 10 mM PBS buffer, pH 6.8 containing 1 mM CaCl₂ and 1 mM MnCl₂, The protein mix (3.5 mL, ca. 1.2 mg protein) was applied to each ligand column and allowed to bind overnight. The column was washed with the same buffer until no more protein was eluted (Abs 280 nm). Bound protein was eluted from the column using 0.5M mannose in 10 mM PBS buffer, pH 8.0 containing CaCl₂ and 1 mM MnCl₂ (buffer filtered to remove CaOH₂ formed) for glycopeptides containing only mannose, 0.5 M N-acetylglucosamine in 10 CaOH₂ mM PBS buffer, pH 8.0 containing CaCl₂ and 1 mM MnCl₂ (buffer filtered to remove CaOH₂ formed) for glycopeptides containing only GlcNAc, and both buffers for glycopeptides containing both mannose and GlcNAc, or for unglycosylated peptides. Samples were obtained in about 700 µL volume and frozen for later protein identification.

### Example 24

### Identification of Proteins from Six-protein Mixture that Bound to Ligand

The identity of each protein eluted from the ligand affinity columns from Example 23 was determined by a combination of Gel electrophoresis and Edman degradation. Gel electrophoresis was carried out using 10% Bis/Tris NuPAGE gels under reducing conditions, using MOPS and then MES buffer. Protein bands were stained using SilverXpress silver staining (NuPAGE). The individual proteins, as well as the mixture, were analyzed along with the eluted fractions. The identity of each protein was obtained by comparison of the band position from the eluted sample to that of each of the known six proteins. The eluted proteins were also identified by N-terminal sequencing of the first 10 amino acids.

### Example 25

### Protein Denaturation Prior to Tryptic Digest

Protein was cleared from beads containing protein-ligand binding "positive hits" isolated as described for Examples 21 (myocyte proteins) and 22 (*E. coli* proteins). Cleavage was carried out in a similar manner for each bead, using different methods, including enzymatic digests with trypsin, Endoproteinase Arg-C, and endoproteinase Lys-C, and chemical cleavage with CNBr, in order to increase confidence that the correct protein was identified. Cleavage was carried out on several beads or on single beads in tubes, or on single beads resting on a stainless steel disc. In some cases, proteins were denatured and the disulfide bond cleaved prior to tryptic digest, so that the digestion could go to completion. In all cases, similar results were obtained.

A single bead containing a ligand-protein binding complex was treated with 10 M Guanidine HCl (15 µL), 50 mM ammonium bicarbonate buffer, pH 7.8 (3.8 µL), and 20 mM DTT (6.2 µL). The solution was heated at 60 °C for 45-60 minutes. Total reaction volume was 25 µL. After denaturation, the reaction was allowed to cool and 50 mM of ammonium bicarbonate buffer, pH 7.8 (200 µL) was added so that the final concentration of Guanidine HCl was 0.75 M. On-bead tryptic digest was then carried out as described below for Example 26.

### Example 26

### Single Bead Tryptic Digest

A single bead containing ligand and bound denatured or undenatured protein was transferred to an RNase- and DNase-free PCR tube. The bead was washed with 15 µL water for 15 minutes with shaking. Water was removed and the bead was washed with 15 µl 100 % acetonitrile on a shaker. The bead was then placed in a speedvac until completely dry. The dry bead was mixed with 15 µL DTT (10 mM in 0.1 M ammonium bicarbonate) at 56°C for 1 hour. After cooling, the DTT was removed and 50 mM iodoacetamide in 0.1 M ammonium bicarbonate (15 µL) was added. The mixture was incubated in the dark for 30 minutes at room temperature. The iodoacetamide was removed and the bead washed with 30 µL 100 % acetonitrile. The bead was dried in the speedvac until dry. To the dried bead was added trypsin in 50 mM ammonium bicarbonate at a concentration of 12.5 ng/µL. The sample was incubated at 37°C overnight. The solution was dried and the cleaved peptides were extracted in an Eppendorf tube with 0.4 µL 70 % acetonitrile + 0.1 % TFA, followed by a 0.4 µL acetonitrile and water mixture (2:1) + 0.1 % TFA, and then 0.5 µL 0.1 % TFA. The extracts were combined, and 0.2 µL of the above mixture was transferred to a stainless steel disc to which 0.2 µL of CHC matrix +1.0 % TFA was added. In some cases, a range of 0.1-1% of TFA was used as well, as 1% formic acid sometimes facilitates better signals from the sample during MALDI-MS. The remaining solution was transferred to a new tube and stored at -20°C. Alternatively, the bead was placed onto the stainless steel disc after drying and extraction was carried out directly on the stainless steel disc. In both cases, similar results were obtained.

Tryptic digest was also carried out on 3-4 beads in an Eppendorf tube in a similar manner as described for single beads, above in this Example. Similar results were obtained. Proteins were identified as described below for Example 31.

### Example 27

### On-bead Endoproteinase Asp-N Digest

Proteolytic digestion by Endoproteinase Asp-N was carried out using the protocol described in Sturrock, et al. 1997, Biochem. Biophys. Res. Commun., 236:16-19, with modifications. Typically, after the ligand-bead-bound-protein was subjected to reduction and alkylation performed according to the protocol described in the tryptic on-bead digest method in Example 26, 20 µL of a solution containing 10 µg of endoproteinase Asp-N in 50 mM ammonium bicarbonate buffer, pH 8.0 was added. The reaction was performed for 16 hours at 37 °C. The solvents were evaporated and the cleaved peptides were extracted as described above for Example 26, and analyzed as described below for Example 31.

### Example 28

### On-bead Endoproteinase Lys-C Digest

After the ligand-bead-bound-protein was subjected to reduction and alkylation performed according to the protocol described for the tryptic on-bead digest process for Example 26, a solution of 20 µL containing 10 µg of endoproteinase Lys-C (in 50 mM ammonium bicarbonate buffer, pH 8.0) was added. The reaction was performed for 24 hours at 37 °C. The solvents were evaporated and the cleaved peptides were extracted as described for Example 26, and analyzed as described below for Example 31.

### Example 29

### On-bead Endoproteinase Arg-C Digest

After the ligand-bead-bound-protein was subjected to reduction and alkylation performed according to the protocol described for the tryptic on-bead digest process for Example 26, a solution of 20 µL containing 10 µg of endoproteinase Arg-C (in 50 mM ammonium bicarbonate buffer, pH 8.0) was added. The reaction was performed for 16 hours at 37 °C. The solvents were evaporated and the cleaved peptides were extracted as described for Example 26, and analyzed as described below for Example 31.

### Example 30

### On-bead CNBr Cleavage

CNBr cleavage was performed according to the protocol described in Youngquist et al., 1995, J. Am Chem. Soc., 117:3900-3906. To a single ligand-protein bead in a 500 µL Eppendorftube was added 15 µL of 20 mg/mL CNBr in 0.1 N HCl. The reaction was allowed to proceed at room temperature in the dark for 14 hours. The samples were dried in a speedvac and cleaved peptides were extracted as described above for Example 26, and analyzed as described below for Example 31.

### Example 31

### Protein Identification

Isolated proteins that bound to specific ligands were identified using peptide mass fingerprinting. Mass spectra were recorded on a Bruker Reflex III MALDI time-of-flight mass spectrometer (Bruker-Daltonics, Bremen, Germany) operated in the positive reflectron mode using delayed extraction. Measurements were performed using the following parameters: Power 83-84 V; lens 7.300. The sum of 200-300 shots was used for each spectrum.

The spectra were calibrated using bradykinin peptide. In some cases, internal mass calibration was performed using a porcine trypsin autolysis product. Peptide masses were searched against peptide mass maps in the National Center for Biotechnology Information (NCBI) database using the following search engines found on the world wide web (www.) for each of:
MS-FIT (prospector.ucsf.edu/ucsfhtml/msfit.htm),
Profound (129.85.19.192/profound_bin/WebProFound.exe), and
MASCOT (matrixscience.com).

A search was performed using the NCBI bacterial (*E. coli*) database and the mammalian databases for the isolated proteins from the *E. coli* and myocyte samples, respectively, and the Swiss Prot mammalian databases and the Swiss Prot viral databases were searched to identify the porcine macrophage proteins and proteins, respectively. A molecular mass range was estimated from 0-250 K Da, allowing a mass accuracy that varied from 0.1 Da (some cases 0.3 Da) for each peptide mass. A large pI range from 0-14 or 0-12 was considered for each search. If no proteins matched, the mass window was extended. Partial enzyme cleavages allowing for two missed cleavage sites and modification of cysteine by alkylation were considered in the search approaches. A protein was considered identified if the matched peptides covered at least 30 % of the complete sequence. A match of less than 30 % was considered in some cases, if prominent peaks were obtained. Usually, four or more peptides were used for identification. In some instances, hypothetical proteins or gene products, such as biochemical material, either RNA or protein, calculated from the expected expression of a gene and to which a function may be assigned based on sequence homology, were identified.

### Example 32

### Results for Myocytes/Ligand Library 1

Using the procedures described above for Examples 1-5, the ligand Library 1 (containing unnatural amino acids) as described for Example 5, myocyte proteins prepared and labeled as described for Examples 9 and 11, screening as described for Example 15, sorting and identifying as described for Examples 18 to 21, digestion and protein identification as described for Examples 25-31, previously unknown, specific, differential ligand-protein binding pairs were identified for the normal (basal) myocyte protein mixtures and the phenylephrine (PE)-treated myocyte proteins screened against the ligands of Library 1. Phenylephrine was used to provide an *in vitro* model of hypertrophy, for example, cardiac hypertrophy (Arnott et al., 1998, Anal. Biochem., 1: 1-18).

The results shown in Table 4 below demonstrate that the process of the invention can be successfully used to identify membrane proteins such as ion channels, symporters, and G-protein coupled receptors, together with specific ligands that bind to them, in one quick step. This is an important result in light of that fact that at least 50 % of all drug targets are membrane proteins. Low abundance proteins, i.e., proteins with a codon bias of <0.1, such as transcription factors, protein kinases, and phosphatases (See, for example, Gygi et al, 2000, Curr. Opin. Biotechnol., 11 pp. 396-401), were also detected. The observation that more than one protein binds to a ligand implies that either each of the proteins identified bind to the same ligand or, in some cases, proteins that work together and interact with each other (i.e. protein complexes; e.g. Entries 1 and 5) were isolated. The type of library used for screening restricts the number and type of proteins that can be identified. In practice, several different types of libraries are screened with the same protein mixture. The described procedures for library synthesis, screening, sorting, and identifying both ligand and protein can be readily automated using known procedures to render these procedures truly "high-throughput".

The proteins listed in Table 4 represent some of the proteins that are more abundant in one cellular state than the other. In this example, Entries 1 - 6 are proteins that are primarily present in basal cells but not in hypertrophied cells, while Entries 7 - 12 show the reverse situation. Both sets of proteins are therefore important in the etiology of cardiac hypertrophy and identify these specific ligand-protein pairs for use in development of new therapeutics for disease, for example, cardiac hypertrophy.

The ligands identified also provide an important tool for furthering an understanding of hypertrophy disease at the molecular level. Some of the proteins identified as binding these ligands are useful as biomarkers for cardiac hypertrophy and related disease, aiding diagnosis. The traditional therapeutic modality for the amelioration of cardiac disease including hypertrophy is primarily through the use of angiotensin converting enzyme (ACE) inhibitors, β-blockers, and ion channel modulators. In Table 4, Entries 5 and 11 are ion channels identified as binding proteins, and the effect of these on cardiac hypertrophy can now be investigated using the identified ligands. Furthermore, since ion channels are important in a wide range of diseases (e.g. epilepsy and hypertension) the identified ligands provide design templates for new drug candidates for existing diseases related to identified ion channels. It is known, for example, that one of the proteins identified by this process (see table below), myosin light chain kinase, is important in the etiology of cardiac hypertrophy (Aoki et al, 2000, Nat. Med., 6: 183-188). From genomic analysis of the genes affected in alternative models of cardiac hypertrophy, the genes that were enriched in load-induced hypertrophy and in neonatal hearts (hypertrophic state) included genes coding for protein phosphatase 1 gamma, mitochondrial NADH-dehydrogenase, and the 60S ribosomal protein L3 (Johnatty et al, 2000, J. Mol. Cell. Cardiol., 32: 805-825). In addition, mitochondrial ATP synthase gene expression in mice was down regulated after induction of hypertrophy with isoproterenol (Friddle et al, 2000, Proc. Nat. Acad. Sci., 97, 6745-6750). These proteins or closely related ones were identified as specific binding proteins in this Example, together with specific binding ligands (see Table 4, Entries 4, 7 and 11). Proteins of unknown identity or function in cardiac hypertropyhy were also observed (e.g. SPTR proteins, regulators of G-protein receptors used for signaling hypothetical proteins). The partnering ligands identified for these proteins can be used to elucidate their importance in cardiac hypertrophy.

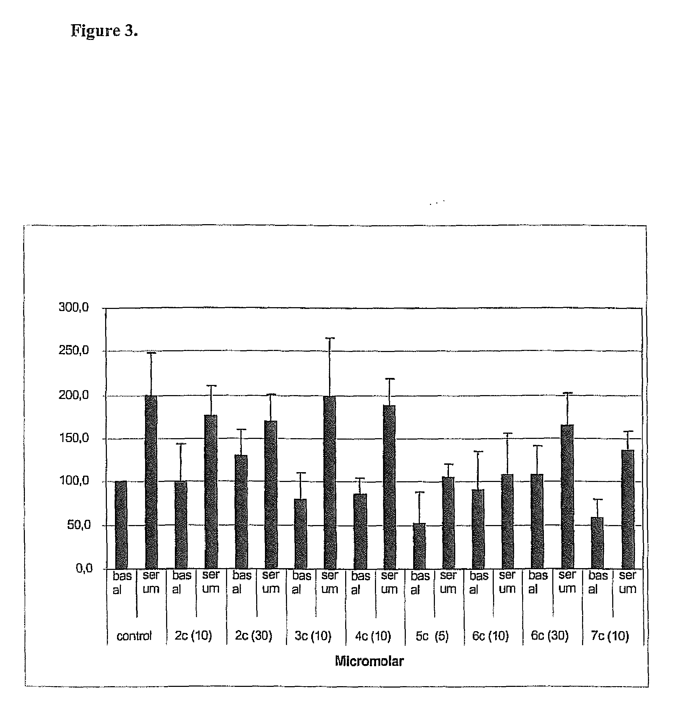

The importance of six ligands in cardiac hypertrophy was investigated by examining the effect of the ligand on the increase in protein synthesis (meaured by increase in DNA synthesis) in myocytes that had been treated with serum to induce a hypertrophic state (Figure 3). Three ligands Phe-Gua-Pal-Tyr-Gua-Tyr [SEQ ID NO: 66], Abi-Thr-Hyp-Hyp-His-[SEQ ID NO: 68], and Pya-Gua-Abi-Asp-Abi-Tyr [SEQ ID NO: 69] reduced the extent of DNA synthesis in both normal and hypertrophied myocytes. 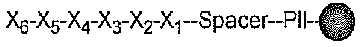 X₁₋₆ = Natural and unnatural amino acids **(3-12, 21-30)**

**Table 4: List of identified ligands and proteins for Library 1 and myocyte proteins.**

| **Entry** | **Identified Ligand** | **Identified Protein(s)** |
|---|---|---|
| **Normal Myocytes** | | |
| 1 | Pip-Pal-Pal-Phe-Pya-Pip [SEQ ID NO: 7] | Ca2+/Calmodulin activated Myosin light chain kinase (gi 284660) (**LA, MA**); Regulator of G-Protein Signalling (RGS 14) variant (gi 2708808) ATP Synthase component (subunit e) (gi 258788) (**M**);; Cytochrome P450 (gi 544086) (**M**); Ribosomal proteins (60s) (gi 21426891); SPTR (gi 20837095) (**M**) |
| 2 | Pya-Hyp-Hyp-Phe-Acm-Tyr [SEQ ID NO: 8] | Troponin T (gi 547047); Ca2+/Calmodulin activated Myosin light chain kinase (gi 284660) (**LA, MA**); cGMP-dependent protein kinase (gi 284660)(**LA**) |
| 3 | Pya-Gua-Pip-Acc-Phe-Pip [SEQ ID NO: 9] | NADH dehydrogenase; ATP binding component (gi 18598538) (**M**); Myosin heavy polypeptide 9 (gi 13543854); Histone associated proteins (gi 20893760) (**M**) |
| 4 | Phe-Aze-Gly-His-Gly-Aze [SEQ ID NO: 10] | Hypothetical proteins (gi 20474763); Cysteine and tyrosine rich proteins of unknown function (gi17064178) (**M**); |
| | | (Mitochondrial ATP synthase (gi 13386040); Ribosomal proteins (60s L series)(gi 21426891)); SPTR (gi12842570). |
| 5 | Phe-Thr-Pya-Pip-Asp-His [SEQ ID NO: 11] | (Sodium channel (gi 18591322) (**M**); Chloride channel (gi 6978663/4502867) (**M**)) Troponin I (gi 1351298);; Zn Finger protein (gi 18591322) (**MA**); SPTR (peroxisomal Ca dependent solute carrier (putative) (gi 12853685); Beta-2 adnergic receptor (gi 12699028) |
| 6 | Phe-Ppy-Acc-Ala-Ppy-Hpy [SEQ ID NO: 12] | Hypothetical proteins; Troponin T gi 547047;; (Phospholipase C (**MA**); Phosphatidylcholine sterol acyl transferase (400167;LCAT-PIG_9)). |
| 7 | Phe-Thr-Tyr-Phe-Ala-Lys [SEQ ID NO: 13]; | Serine/threonine Protein kinase (gi 5730055); Carbonic anhydrase VII (gi10304383). |
| 8 | His-Tyr-Pip-Thr-Acm-Abi [SEQ ID NO: 14]; | Chain C P27 cyclin A-CDK2 complex: (Cyclin A?) (gi2392395); Hypothetical protein XP_154035. |
| 9 | Tyr-Pip-Thr-Acm-Aze-His [SEQ ID NO: 15]; | N4-(β-glucosaminyl-L-asparaginase; (gi7435941); Membrane spanning 4-domain subfamily A member II (gi7435941). |
| 10 | Phe-Phe-Phe-Pip-Aze-Gua [SEQ ID NO: 16]; | Phosphatidyl choline-sterol acyl transferase (400167;LCAT-PIG_9). |
| 11 | Phe-Gua-Asp-Abi-His-Aze [SEQ ID NO: 17]; | Hypothetical protein XP_043250 (gi 14773490) |

| **Phenylephrine Treated (Hyperthrophic) Myocytes** | | |
|---|---|---|
| 12 | Phe-Abi-Pal-Hyp-Thr-Hyp [SEQ ID NO: 65] | Zinc finger associated protein gi 20304091; Ribosomal proteins 40S L series (gi 206736/133023); |
| 13 | Phe-Gua-Pal-Tyr-Gua-Tyr [SEQ ID NO: 66] | Glucose-6-Phosphatase (gi 6679893/15488608); Succinate dehydrogenase; ARL-interacting protein (gi 4927202) (M); SPTR (gi 12834839) (M), Nucleic acid binding protein. |
| 14 | Pal-Abi-Gly-Gly-Abi-His [SEQ ID NO: 67] | Ribosomal protein (60s + 40s) (gi 20875941/6677773 and gi 20846353); Low density lipoprotein receptor (gi 20846353). |
| 15 | Abi-Thr-Hyp-Hyp-His-[SEQ ID NO: 68] | Phosphofructokinase (gi 7331123); Selenium binding protein (gi 8848341/6677907); (Serine arginine rich protein kinase (LA); Guanylate kinase (gi 20986250) (**LA**), (**M**); SPTR (gi 12842823) (**M**, Actin interacting protein. |
| 16 | Pya-Gua-Abi-Asp-Abi-Tyr [SEQ ID NO: 69] | SPTR (gi 20869775) (**M**); Ribosomal proteins (60s) (gi 20875941/6677773); (Calcium channel (gi 3202010) **(M)**; Slo channel protein isoform (gi 3644046 ) (**M**); Potassium conductance calcium activated channel (gi 6754436,NP_034740) (**M**); ; Regulator of G-protein signalling 8 (gi 9507049). |
| 17 | Abi-Phe-Abi-Phe-CheTyr [SEQ ID NO: 18] | Cathepsin E (gi 4503145); Ribosomal proteins (60s L series) (gi 20826861).. |
| | 18 Pal-Gly-Abi-Hyp-Pya-Trp [SEQ ID NO: 56]; | NAS putative unclassified (gi 12861084); Putative Zn finger protein 64 (gi 12849329). |
| 19 | Lys-Met-Hyp-Trp-Tyr-Gua [SEQ ID NO: 57]; | Cell surface glycoprotein (gi 23603627); Hypothetical protein (XP-179829; gi 14720727). |
| 20 | Phe-Asp-Trp-Gua-Thr-Gua [SEQ ID NO: 58]; | Orphan Nuclear receptor similar to hsp40 (NRID 26166582). |

In the table above, Abi = 3-amino-3-(biphenyl)-propanoic acid; Acc = 3-Amino-carboxymethyl-caprolactame; Aze = L-Azetine-2-carboxylic acid; ARL = ADP-ribosylation like factor; Che = 1-amino-1-cyclohexanecarboxylic acid; Gua = 4-S-(Di-Boc-Guanidino)-L-Proline; Hyp = L-Hydroxyproline-OH; Pal = L-Dapa(Palmitoyl)-OH; Pip = 4-Phenyl-Piperidine-4-carboxylic acid; Ppy = 5-Phenyl-Pyrrolidine-2-carboxylic acid; Pya = L-3-PyridylAla-OH; SPTR = Hypothetical membrane transporter proteins;

Possible protein complexes, protein families or proteins that work closely together are enclosed in parentheses. The large filled circle represents a bead of resin. The letters in parentheses have the following designation: **LA**: low abundance proteins, **M**: protein is a transmembrane one or partially embedded. **MA**: protein is associated with a membrane.

### Example 33

### Results for E. coli Protein/Ligand and Library 2

Using the processes and procedures described for the Examples above, the sialic acid lactam Library 2 of Example 6, and *E. coli* membrane proteins (i.e. protein extract from *E. coli* that has been processed to access primarily inner and outer membrane proteins) produced, isolated and labeled as described in Examples 10 and 13 were mixed together and specific ligand protein binding pairs attached to resin beads were isolated as described in Example 16. The identity of the ligands were established by MS as described in Example 19, ligands were resynthesised on solid phase as in Example 8 and the protein binding partners isolated on the resin bead as detailed in Example 22. The identity of the protein binding partners for each ligand was determined by first denaturing the protein bound to the bead as in Example 25, followed by tryptic digest of one or several beads as described in Example 26 and the resulting peptides used to search databases for the identity of the proteins as described in Example 31. Of the 37 ligands isolated, 34 were conclusively identified and used for the isolation and identification of the bound proteins. The specific pairs identified thus far are shown in Table 5 below, where T(Sa) = Sialic acid threonine lactam (see Table 2 for specific ligand structures). The letters in parentheses have the following designation: **LA**: low abundance proteins, M: protein is located either on the inner or outer membrane and can be transmembrane or partially embedded, P: proteins primarily located in the periplasmic space.

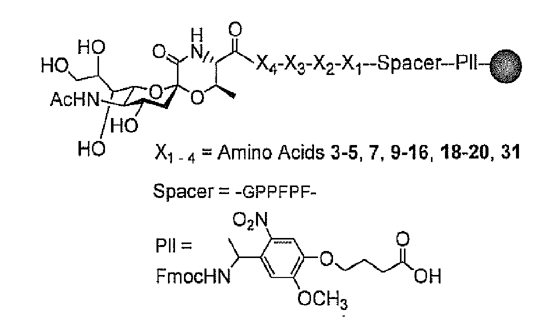

**Table 5: List of identified ligands and proteins for Library 2 and E. coli membrane proteins**

| **Entry** | **Identified Ligand** | **Identified Protein(s)** |
|---|---|---|
| 1 | T(Sa)-F-N-H-S [SEQ ID NO: 19] | Phosphate acetyl transferase (gi 1799680); acid shock protein (gi 1742632); molybdopterin biosynthesis protein C (gi 15800534). |
| 2 | T(Sa)-F-A-L-V [SEQ ID NO: 20] | Chaperone DnaK (dnak_E.coli), putative hydrolase (yhaG_E.coli), transposase (gi 158316821); Cytochrome C peroxidase (yhjA_E.coli). |
| 3 | T(Sa)-F-G-I-W [SEQ ID NO: 21] | Histidine synthetase (gi 15803037), aspartate carbamoyl transferase (pyrI_E. coli), putative permease transport protein (b0831_E.co) (**M**); Orf hypothetical protein (yids_E.coli). |
| 4 | T(Sa)-F-G-I-M [SEQ ID NO: 22] | Transposase, transcriptional regulator (gi 18265863) (**LA**), GroEL (GroEL_E.coli), protein involved in the taurine transport system (tauC_E.coli) (**M**). |
| 5 | T(Sa)-G-V-F-L [SEQ ID NO: 23] | Heme binding lipoprotein (gi 4062402/40624079) (**M**), regulator for D-glucarate, D-glycerate and D-galactarate (gi 158294209), glutamine tRNA synthetase (gi146168). |
| 6 | T(Sa)-Y-S-M-P [SEQ ID NO: | Biotin synthetase (gi 145425), UDP-glucose dehydrogenase (ugd_E.coli), tyrosine protein kinase (gi 20140365) (**LA**), fatty acid |
| 7 | 24] T(Sa)-L-S-W-W [SEQ ID NO: 25] | oxidase complex proteins (gi 145900). NAD-dependent 7-alpha-hydroxysteriod dehydrogenase (gi 15802033), homocysteine transferase, nitrate reductase (**P**), lactate dehydrogenase dld_E.coli, citrate synthetase (CISY_E.coli). |
| 8 | T(Sa)-H-W-H-I [SEQ ID NO: 26] | Mannose-1--phosphate guanyl transferase (gi 3243143/ 324314), isopropyl malate dehydrogenase (guaB_E.coli) (**M**). |
| 9 | T(Sa)-H-W-V-V [SEQ ID NO: 27] | Pyruvoyl dependent aspartate decarboxylase (gi 3212459), colicin E2. (gi809671/809683), Histidine kinase (part belongs to narQ_E.coli)(**M**). |
| 10 | T(Sa)-H-L-G-Y [SEQ ID NO: 28] | Protein involved in lipopolysaccharide biosynthesis (gi 16131496) **(M**), phosphomannose isomerase (gi147164), Cytochrome C type protein (gi 15802755), TrwC protein (TrwC_E.coli). |
| 11 | T(Sa)-I-Y-L-F [SEQ ID NO: 29] | Membrane bound ATP synthetase Fo sector subunit b (atpF_E.coli ) (**M**), ATP hydrolase (gi 1407605). |
| 12 | T(Sa)-F-G-L-M [SEQ ID NO: 30] | Hemolysin C (gi7416115; gi 7438629), high affinity potassium transport system (kdpC_E.coli) (**M**), quinone oxidoreductase (qor_E.coli) (**M**), ferrodoxin dependent NA(D)PH oxidoreductase (fpr_E.coli) (**M**). |
| 13 | T(Sa)-W-V-N-M [SEQ ID NO: 31] | Transposase (gi 161295379), inner membrane protein for phage attachment (pspA_E.coli) (**M**). |
| 14 | T(Sa)-M-V-N-W [SEQ ID NO: 32] | ATP dependent helicase (gi 2507332/16128141), mob C (gi 78702); Orf hypothetical protein (yciL_E.coli); TraI protein (Tri6_E.coli); Putative Transposase (gi 16930740). |
| 15 | T(Sa)-H-I-G-Y [SEQ ID NO: 33] | Fimbrial subunit (gi 2125931), outer membrane pyruvate kinase (gi16129807/15831818) (**LA, M**) |
| 16 | T(Sa)-L-Y-L-F [SEQ ID NO: 34] | Fimbrial protein precursor (gi120422), alkaline phosphatase (gi581186) (P), Cytochrome, Zinc sensitive ATP component (cydD_E.coli) (P), Putative aldolase. |
| 17 | T(Sa)-H-W-H-L [SEQ ID NO: 35] | Chorismate mutase (gi 1800006), xanthine dehydrogenase (gi 157999), carbamoyl phosphate synthetase (carB_E.coli); Glutamate synthase (NaDPH) (gi 2121143). |
| 18 | T(Sa)-F-V-W-H [SEQ ID NO: 36] | NADH dehydrogenase (gi 1799644) (**M**), protein involved in flagellar biosynthesis and motor switching component (gi1580237 )(**M**). |
| 19 | T(Sa)-Y-G-A-M [SEQ ID NO: 59] | Lysine-arginine-ornithine-binding protein (argT_E.coli) (P), ATP binding component of glycine-betaine-proline transport protein (gi 16130591)(**P**). |
| 20 | T(Sa)-L-Y-I-F [SEQ ID NO: 37] | Colicin (gi 809683), hypothetical membrane protein (yhiU_E.coli) (**M**) outer membrane lipoprotein (blc_E.coli) (**M**). |
| 21 | T(Sa)-S-V-W-F [SEQ ID NO: 60] | Acetly Coa carboxylase: beta subunit (gi 146364); Cytochrome b (cybC_E.coli), Phosphate acetyl transferase (gi 1073573), Urease: beta subunit (gi 418161). |
| 22 | T(Sa)-H-Y-F-F [SEQ ID NO: 61] | Molybdenum transport protein (gi1709069), Glycerol 3-phosphate dehydrogenase subunit C (gi 146179), Cell division protein (ftsN_E.coli). |
| 23 | T(Sa)-I-Y-Y-F [SEQ ID NO: 62] | Transposase (gi10955467); Serine tRNA synthetase (gi15830232); Methylase (gi1709155); Coenyzyme A transferase (gi1613082); TraD membrane protein (TraD_E.coli). |
| 24 | T(Sa)-Q-P-G-M [SEQ ID NO: 63] | ATP dependent helicase: HrpA homolog (NCBIBAA15034); Putative protease ydcP percursor (NCBI P76104). |
| 25 | T(Sa)-G-P-H-G [SEQ ID NO: 64] | Uroporphyrinogen Decarboxylase (hemE_E.coli); Putative export protein J for general secretory pathway (yheJ_E.coli). |

The results of Example 33 shown in Table 5 demonstrate that the process of the invention can be successfully used to identify membrane proteins and specific binding ligands in one quick step. This is an important result in light of that fact that at least 50 % of all drug targets are membrane proteins. The proteins identified were inner and outer membrane proteins as well as proteins from the periplasmic space and a few from the cytosol.

Proteins with a wide range of functions, including transport (e.g. protein involved in taurine transport system), protein synthesis (transposase and Chaperone DnaK), metabolism (chorismate mutase, citrate synthetase), and lipopolysaccharide biosynthesis (protein involved in lipopolysaccharide biosynthesis) were identified as well as proteins of as yet unknown function. The type of library used restricts the number and type of proteins that can be identified. In practice, several different types of libraries are screened with the same protein mixture. For more complex cell types, where, for example, thousands of proteins will be isolated, the described processes can be readily automated, using known methods.

Of the proteins identified from this analysis, one is a proven target for antibacterial drugs. The 50 S ribosomal protein (Table 5, Entry 13) is a target of chloramphenicol and macrolide antibiotics that block bacterial protein synthesis (See, for example, Section 13: Infectious disorders, Chapter 153: Antibacterial drugs, In: The Merck Manual of Diagnosis and Therapy, M.H. Beers and R. Markow (Eds), 17th ed. 1999, Merck & Co). In light of increasing bacterial resistance, there is an urgent need to develop alternate antimicrobials. Current approved antibiotics target 15 bacterial enzymes and macromolecular complexes (Strohl, W.R. (Ed): Biotechnology of antibiotics. Marcel Dekker, New York, 1997) in the area of cell-wall biosynthesis, cell membrane permeability, protein synthesis and DNA replication and repair synthesis (Section 13: Infectious Disorders, Chapter 153: "Antibacterial drugs," M.H. Beers and R. Markow (Eds), 17th ed. 1999, Merck & Co.). In the present invention, all of the proteins identified in this Example are putative drug targets for antibacterials. This premise can be rapidly tested in biological assays using the matching ligand-binding partner that has also been identified. Thus, all the binding ligands identified are putative antibacterial agents, providing that they selectively interact with bacterial proteins in the host or interact with a bacterial protein for which there is no human counterpart.

In the age of genomics, where the complete genomes of several pathogens are known, several putative, novel antibacterial targets have been postulated after intensive sequence analysis. Some of these newly recognized antibacterial drug targets have also been identified in this Example and are mentioned below. Histidine kinase (Table 5, Entry 17) has recently been recognized as a target protein for antimicrobial agents. (Matsushita et al., 2002, Bioorg. Med. Chem., 10: 855-67; Deschenes et al., 1999, Antimicrob. Agents Chemother., 43: 1700-03; Lyon et al., 2000, Proc. Nat. Acad. Sci., 97: 1330-35). A Philadelphia-based company, Chaperone Technologies (see the world wide web site: chaperonetechnologies.com/technology.htm), is based on the development of antimicrobial drugs from compounds that bind to DnaK chaperone (Table 5, Entry 10). The pharmaceutical company former SmithKline Beecham has developed methods of using nitrate reductase alpha subunit (Table 5, Entry 15) to screen for antibacterials effective against *S. aureus* (Molecular Targets, 2001, 12, pp. 13; web site: experts.co.uk). Phosphomannose isomerase (Table 5, Entry 18) is an essential enzyme in the synthesis of GDP-mannose that is utilized in the synthesis of lipopolysaccharides, glycoproteins, and exopolysaccharides (Wills et al, 2000, Emerging Therapeutic Targets, 4(3): 1-30). This enzyme has been recognized as a potential drug target for antifungals and in *Candida,* has been inhibited by sulfadiazene (Wells et al, 1996, Biochemistry, 34, pp. 7896-7903). All the aminoacyl tRNA synthetases are putative targets for antibacterial agents. In fact, the approved antibiotic, Mupirocin, inhibits the enzyme isoleucine tRNA synthetase, (Section 13: Infectious disorders, Chapter 153: Antibacterial drugs, In The Merck Manual of Diagnosis and Therapy, M.H. Beers and R. Markow (Eds), 17th ed. 1999, Merck & Co.) for which the glutamine analog has been identified by this Example of the invention (Table 5, Entry 13).

From microbial genomic analysis, several classes of proteins, such as outer membrane proteins, host-interaction factors, permeases, metabolic enzymes, DNA replication and transcription and repair apparatus, have been identified as putative antibacterial drug targets (M. Y. Galperin and E.V. Koonin, 1999, Curr. Opin. Biotechnol., 10: 571-578) and are among those binding proteins detected by this Example of the invention and are mentioned below. An analysis of virulence factors, potential drug targets in *H. pylori* and *N. meningitidis,* showed that carbamoyl phosphate synthetase (Table 5, Entry 25), NADH-ubiquinone oxidoreductase (cf. Table 5, Entry 20), fimbrial protein (Table 5, Entries 23 and 24), LPS biosynthesis protein (Table 5, Entry 18), and ATP dependent helicase (Table 5, Entry 22) were promising targets (Junaid Gamieldien, *Ph.D Dissertation,* Chapter 4: "Novel Approaches for the Identification of Virulence Genes and Drug Targets in Pathogenic Bacteria", 2001, University of Western Cape, South Africa). Furthermore, knockout analysis of selected genes in *H. pylori* showed that knocking out the gene for carbamoyl phosphate synthetase resulted in decreased colonization efficiency while the gene coding for NADH-ubiquinone oxidoreductase proved essential for the survival of the bacteria. Finally, enzymes in the aromatic amino acid biosynthetic pathway that are absent in humans make attractive drug targets. One such enzyme, chorismate mutase (Table 5, Entry 25) has been identified by this Example of the invention. Other proteins identified by this process have diverse functions that may be essential to the survival of the bacteria, or as yet unknown functions. The function of these proteins can be probed using the identified ligands as a starting point.

### Example 34

### Results for Six Protein Mixture and Ligand Library 3

Using the processes and procedures described for the Examples above, the glycopeptide Library 3 of Example 7, and the six protein mixture: Con A, *P. sativum* lectin, *L. culinaris* lectin, *W. floribunda* lectin, Glyceraldehyde 6-phosphate, and bovine serum albumin (BSA) labeled as described in Example 14 were mixed together and specific ligand protein binding pairs attached to resin beads were isolated as described in Example 17. The identity of the ligands were established by MS as described in Example 19, ligands were resynthesised on solid phase as in Example 8 and the protein binding partners isolated on the resin bead as detailed in Example 23. The identity of the protein binding partners for each ligand was determined by a combination of gel electrophoresis and Edman degradation as described in Example 24.

The identified ligand-protein pairs are shown in the Table 6 below, where ManS = Mannose linked to hydroxyl group of Ser; ManN = Mannose amine linked to the side chain carboxyl group of Asp (*N*^{α}-Mannosylasparagine) and GlcNN = *N*-Acetylglucosamine linked to the side chain carboxyl group of Asp (*N*^{α}-*N*-Acetylglucosaminylasparagine) (see Table 2 for exact structures). The large filled circle represents a resin bead.

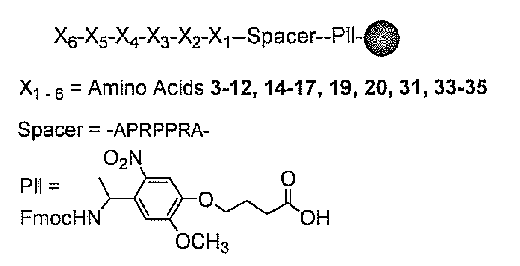

**Table 6: List of identified ligands and proteins for Library 3 and six protein mixture.**

| **Entry** | **Identified Ligand** | **Identified Protein(s)** |
|---|---|---|
| 1 | ManS-Gly-ManS-Asp-Asn-Ala [SEQ ID NO: 38] | **Con A**, *P. sativum* lectin |
| 2 | ManS-Gly-GlcNN-Asn-ManS- Tyr [SEQ ID NO: 39] | **Con A**, *P. sativum* lectin, *L. culinaris* lectin |
| 3 | ManN-Phe-Trp-Ser-Lys-His [SEQ ID NO: 40] | **Con A**, *P. sativum* lectin |
| 4 | GlcNN-Trp-Phe-Asp-Trp-Pro [SEQ ID NO: 41] | **Con A** |
| 5 | GlcNN-Val-GleNN-His-ManS-Gly [SEQ ID NO: 42] | **Con A**, *P. sativum* lectin |
| 6 | ManN-ManS-ManN-Trp-Ser-Trp [SEQ ID NO: 43] | **Con A,** *P. sativum* lectin, *L. culinaris* lectin |
| 7 | Gly-Pro-Lys-Lys-Tyr-His [SEQ ID NO: 44] | **Con A,** *P. sativum* lectin, *L. culinaris* lectin |
| 8 | His-Thr-Trp-Gly-Tyr-Trp [SEQ ID NO: 45] | **Con A** |

In this Example, some of the ligands and matching binding proteins identified are useful glyco-tools for elucidating the molecular mechanisms of lectin-ligand binding and molecular mimicry. It is interesting that GlcNN-Trp-Phe-Asp-Trp-Pro binds only to Con A and not to the other lectins, although they are reported to have similar specificity for a sugar-containing ligand. In addition, ligand 8, which does not contain a sugar residue, binds selectively to Con A, providing a tool for the study of molecular mimicry in lectin-ligand interactions. All the ligands identified, when attached to chromatographic resins (e.g. sephacryl, sepharose), are useful for affinity purification of the three lectins used in the study (some selectively). These identified binding ligands are also useful to purify novel mannose/glucose specific lectins that may be used for large-scale commercial production of proteins that bind specifically to lectins, including antibodies for clinical use and other glycoproteins.

### Example 35

### Synthesis of Library 4: Macrocyclic Ureas

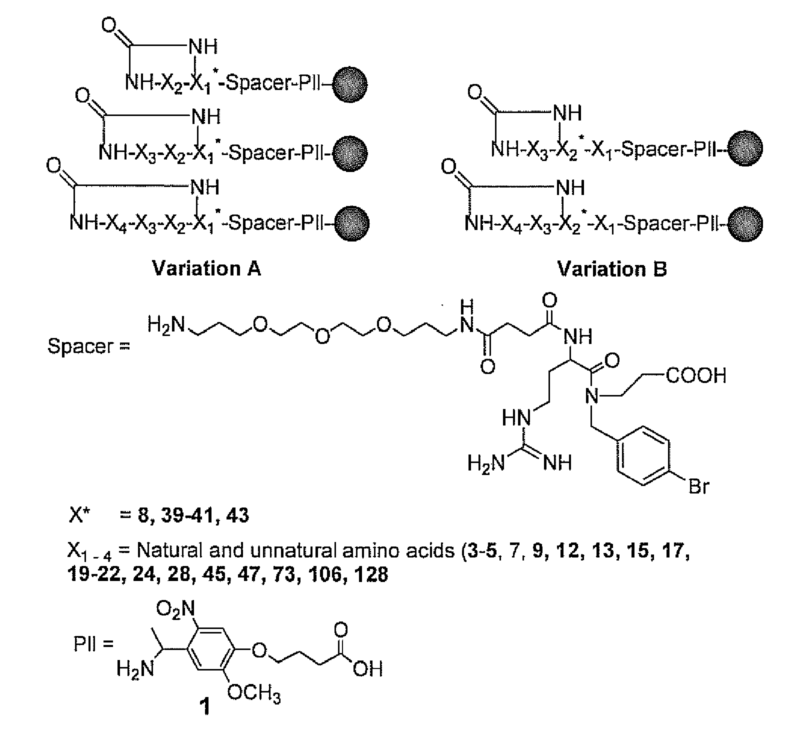

In one embodiment, Library 4, shown above, was prepared on PEGA₄₀₀₀ resin (2 g, 0.1 mmol/g; 500-700 µm beads) using the ladder synthesis method, as previously described in St. Hilaire et al., 1998, J. Am. Chem. Soc.120: 13312-13320. The synthesis of variation A is shown in Scheme 12A, where for peptides X₂ X₁* [SEQ ID NO: 46], X₃ X₂ X₁* [SEQ ID NO: 47], and X₄ X₃ X₂ X₁*[SEQ ID NO: 48], X₁* is chosen from amino acids **8, 39-41, 43,** and X₄ X₃ X₇ are each independently chosen from amino acids **3-5, 7, 9, 12, 13, 15, 17, 19-22, 24, 28, 45, 47, 73,106,128** , as shown in Tables 1, -3 , and 9. The synthesis of variation B shown in Scheme 12B was carried out similarly, where for peptides X₃ X₂* X₁ [SEQ ID NO: 49], and X₄ X₃ X₂* X₁ [SEQ ID NO: 50], X₂* is chosen from amino acids **8, 39-41, 43,** and each of X₁, X₃, X₄ is chosen from amino acids acids **3-5, 7, 9, 12, 13, 15, 17, 19-22, 24, 28, 45, 47, 73, 106, 128,** as shown in Tables 1-3, and 9. It has been shown that cyclic and aliphatic urea containing compounds are inhibitors af Cdk4 kinase (see, for example, Dolle, 2002, J Comb. Chem., 4: 369-418). It is therefore expected that a library of peptidic cyclic ureas such as Library 4 binds primarily to kinases present in the cellular protein mixture used for screening. Since no particular kinase is targeted, the library is not designed based on structure-activity function data. The building blocks used are chosen arbitrarily, and in a manner to present as many functional groups as possible in the side chains: including, for example, carboxylic acids, amines, indoles, pyridines, aliphatics, aromatics, imidazoles, hydroxyls. It is expected that proteins that are not kinases will also bind to some of the Library members. The building blocks used, , are shown in the Tables 1-3 and 9 above.

PEGA4000 (500-700 µm, 0.1 mmol/g, 2 g) was washed with 10% TFA/DCM and then DCM. In order to increase the loading of the resin, a mixture of Fmoc-Lys(Fmoc)-OH (0.6 mmol, 354 mg), TBTU (0.56 mmol, 180 mg) and NEM (1.2 mmol, 0.15 mL) in DMF (40 mL) were added to the beads after standing for 5 min. After shaking overnight, the solvent was drained and beads were washed with 4xDMF and 4xDCM, then treated with 2+18 min 20% piperidine/DMF and washed with 4xDMF and 4xDCM.

The photolabile linker **1** (1.2 mmol, 620 mg), was coupled to the resin, under TBTU activation (TBTU: 1.12 mmol, 363 mg, NEM (2.4 mmol, 0.3 mL in DMF (30 mL)) overnight, then deprotected by 20% piperidine/DMF as described above. A spacer molecule formed by sequential coupling of Fmoc-N-BrBn-β-Ala-OH, Fmoc-Arg(Pmc)-OH, and compound 2 after TBTU preactivation, was then added to the linker. The spacer molecule was used to enable the identification of a ligand using MALDI-TOF MS, as the spacer increases the mass of the ligand fragments to over 600 mu, away from the matrix peaks. The spacer was designed to have few or no interactions with proteins in the mixture. Furthermore, the spacer contained an isotopic tag (Br) to enable easy identification of the ligand peaks in a mass spectrum. Consequently, after deprotection of the linker, the resin above were treated with Fmoc-N-BrBn-β-Ala-OH (1.2 mmol, 576 mg), TBTU (1 mmol, 321 mg) and NEM (2.4 mmol, 0.3 mL) in DMF (20 mL) overnight, then deprotected by 20% piperidine/DMF as described above. After deprotection, Fmoc-Arg(Pmc)-OH (1.2 mmol, 800 mg) was coupled to the resin using activation by TBTU (1 mmol, 321 mg) and NEM (2.4 mmol, 0.3 mL) in DMF (25 mL) overnight, then deprotected by 20% piperidine/DMF as described above. Compound 2 (1.2 mmol, 650 mg), was then coupled to the resin, also using TBTU activation (TBTU (1.1 mmol, 353 mg) and NEM (2.4 mmol, 0.3 mL) in DMF (25 mL) overnight) then de-protected by 20% piperidine/DMF as described above.

Randomized positions of the library are generated using the split and mix approach described in Furka et al., 1991, Int. J. Peptide Protein Res., 37: 487-493 and Lam et al., 1991, Nature, 354: 82-84 in one or more 20-well custom-made (2.0 mL capacity) multiple column library generator. In the ladder synthesis strategy, 5 % of the growing oligomer is capped using the Boc-protected amino acid analog of the Fmoc building block. Therefore, a mixture of the Fmoc- and Boc-protected amino acid (95% Fmoc and 5% Boc, 4 equivalents) from stock solutions are activated with TBTU/NEM for 6 minutes and then added to the wells. Library 4 contains variations in the position and size of the cyclic urea formed and the positional variation is designated A and B. In Variation A, 1 g of the resin above was divided into a 20-well synthesizer. Five different di-amino acids (8,39-43) were coupled onto the beads by treating with a mixture of Fmoc- protected di-amino acid building block (45 µmol), Boc- protected di-amino acid building block (5 µmol), TBTU (16.1mg, 48 µmol) and NEM (25 µL, 200 µmol) in DMF (0.5 mL) overnight. After mixing and deprotection of the Fmoc protecting group by treatment with 20% piperidine in DMF for 4 + 16 minutes. 20 Different amino acid building blocks are coupled onto the beads by treating with a mixture of Fmoc-protected amino acid building block (45 µmol), Boc- protected amino acid building block (5 µmol), TBTU (15.4 mg, 48 µmol) and NEM (25 µL, 200 µmol) in DMF (0.5 mL) overnight. After removal of Fmoc- protection by 2+18 min 20% piperidine/DMF, 0.25% beads were taken out. The rest was coupled with another round of 20 different amino acids as described above and de-protected. 5% Beads were taken out. The rest was coupled with another round of 20 different amino acids and then de-protected.

In Variation B, 1 g of resin above was put into a 20-well synthesizer. 20 Different amino acid building blocks are coupled onto the beads by treating with a mixture of Fmoc-protected amino acid building block (45 µmol), Boc- protected amino acid building block (5 µmol), TBTU (15.4 mg, 48 µmol) and NEM (25 µL, 200 µmol) in DMF (0.5 mL) overnight. After mixing and deprotection of the Fmoc protecting group by treatment with 20% piperidine in DMF for 4 + 16 minutes, 5 different di-amino acid building blocks are coupled onto the beads by treating with a mixture of Fmoc- protected di-amino acid building block (45 µmol), Boc- protected di-amino acid building block (5 µmol), TBTU (16.1mg, 48 µmol) and NEM (25 µL, 200 µmol) in DMF (0.5 mL) overnight. After removal of Fmoc- protection, the beads was coupled with another round of 20 different amino acids and de-protected as described above. 5% of the resin was taken out and the remaining beads were coupled with another round of 20 different amino acids and then de-protected.

All portions of the resin in both variation A and B were combined and washed 6 times with DCM. The N-terminal amine is then treated with carbonyldiimidazole (CDI) (4 mmol, 650 mg) in DMF (40 mL) at room temperature overnight. The Boc protecting group was removed by treatment with 0.5+5 min 10% TFA/DCM and the resulting amine deprotonated with 10% NEM/DMF. After washing with DMF, cyclization of the peptide was effected by microwave irradiation in DMF (100 mL) at 100 °C for 1 hr. The resin was then filtered, washed with 6xDCM, deprotected with 85% TFA containing 2% triisopropylsilane, 2.5% EDT, 5% thioanisole, 5% water for 1 -2.5 hours. Then the resin is washed with 90% aqueous acetic acid (4 x 5 minutes), DMF (2 x 2 minutes), 5% DIPEA in DMF (2 x 2 minutes), DMF (4 x 2 minutes), CH₂Cl₂ (10 x 2 minutes), and finally methanol (5 x 2 minutes), before being dried by lyophilization overnight.

### Example 36

### Synthesis of Library 5: Diazepine-like compounds

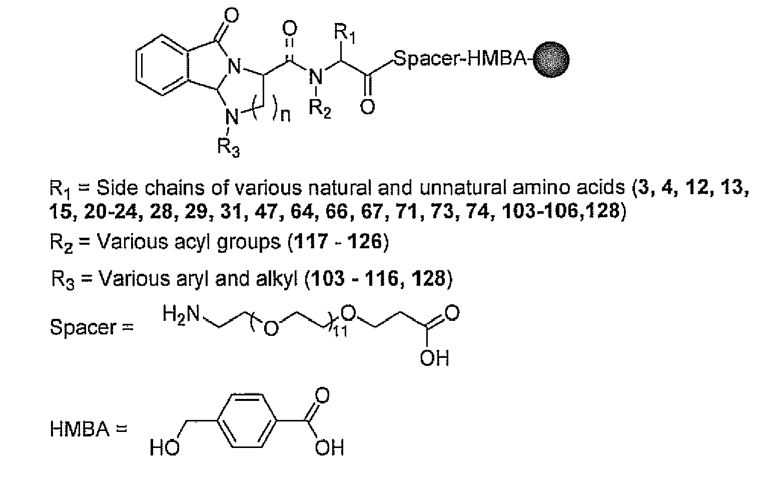

Library 5, shown above, was prepared on PEGA₄₀₀₀ resin as shown below in Schemes 13A and 13B. The library was designed to create diazepine-like templates (when n = 3) and isoindolone-type compounds.

Many benzodiazepines have potent biological activities (see Pigeon et al, 1998, Tetrahedron, 54: 1497-1506). Isoindolone-type compounds possess anti-inflammatory, analgesic, blood pressure lowering, spasmolytic, tranquilizing and anti-tussive properties. As for Libraries, 1-4, no single particular protein was targeted and the building blocks used were chosen arbitrarily, but such that as many functional groups as possible were presented in the side chains: e.g. carboxylic acids, amines, indoles, pyridines, aliphatics, aromatics, imidazoles, hydroxyls. For R₁, the building blocks used were judiciously chosen, for example, from compounds 3-127 shown in Tables 1-3 and 9 above. Compounds containing Boc-protected amines as a side chain were unsuitable for the first position. R₂ comprises various acyl groups, while R₃ is aryl or alkyl (See Table 9).

PEGA4000 resin (500-700 um, 0.087 mmol/g, 1 g) was washed with 10% TFA/DCM and then DCM. A mixture of Fmoc-Lys(Fmoc)-OH (0.3 mmol, 180 mg), TBTU (0.295mmol, 95 mg) and NEM (0.87 mmol, 0.1 mL) in DMF (18 mL) was added to the beads after standing for 5 min. After shaking for 5 hr, the solvent was drained and beads were washed with 4×DMF and 4xDCM, then treated with 2+18 min 20% piperidine/DMF and washed with 4DMF and 4×DCM. The photolabile linker, 1 or HMBA linker ((3 equivalents) was then coupled under TBTU activation. Library 5 was not synthesized by the ladder method. A spacer molecule (O-(N-Fmoc-2-aminoethyl)-O-(2-carboxyethyl)-undecaethylenglycol) facilitated the identification of active ligands by MALDI-MS. Randomized positions of the library were generated using the split and mix approach described in Furka et al., 1991, Int. J. Peptide Protein Res., 37: 487-493 and Lam et al., 1991, Nature, 354: 82-84, in one or more 20-well custom-made (2.0 mL capacity) multiple column library generators.

In the synthesis of Library 5, the spacer (O-(N-Fmoc-2-aminoethyl)-O-(2-carboxyethyl)-undecaethylenglycol (0.5 mmol, 420 mg) was coupled using r using TBTU /NEM activation. After cleavage of the Fmoc protecting group by treatment with 20% piperidine in DMF for 4 + 16 minutes, all beads above were put into a 20-well synthesizer. 92% of these beads (Portion A) were divided into 19 wells and coupled with 19 Fmoc-protected primary amino acid building blocks while the remainder (Portion B) was divided into 7 wells and coupled with 7 Fmoc- protected secondary amino acids chosen from 3, 4, **12, 13, 15, 20-24, 28, 29, 31, 47, 64, 66, 67, 71, 73, 74, 103-106** and **128** (3 equivalents), After the coupling, Portion A and Portion B were combined separately and de-protected with 20% piperidine/DMF as described above.

10% of Portion A and 33% of Portion B were combined (Portion C) and divided into two halves. They were coupled with Fmoc-Daba(Boc)-OH and Fmoc-Orn(Boc)-OH (100 µmol, TBTU 96 µmol, NEM 40 µl in 0.5 mL). The rest of Portion A (Portion D) was treated with Fmoc-Dapa(Boc)-H (1.37 mmol, 560 mg) and NaCNBH₃ (650 mg)in MeOH:DMF:triethyl orthoformate= 1:1:1 (4.5 mL) overnight. The rest of Portion B (Portion E) was treated with Fmoc-Dapa(Boc)-H (137 µmol, 56 mg) and NaCNBH₃ (80 mg)in MeOH:DMF:triethyl orthoformate=1:1:1 (0.5 mL) overnight. Portion D was divided into 10 wells and treated with acid chloride or acid-TBTU complex **(117-126)** in TEA (0.2 mL) and DMF (3 mL) overnight.

All beads from Portions C, D and E were combined and deprotected with 20% piperidine/DMF. They were then treated twice, each time 1 hr, with 3-brornophthalide (53, 230 mg, 1 mmol) and triethylamine (1 mL) in DMF (10 mL). After washing with 6xDCM, they were treated with 2× 10% TFA/DCM,

All beads above were divided into 10 wells and treated with aldehyde (100 µmol) and NaBH(OAc)₃ (1.6 mmol) in 2.5% acetic acid in DCM (2 mL) overnight. All beads are washed with 6xDCM and 6×H₂O.

### Example 38

### Synthesis of Fmoc-N^{α}-(p-BrBn)-β-Alanine (102)

Fmoc-N^{α}-(*p*-BrBn)-β-Alanine was prepared as shown in Scheme 16. β-Alanine (3 g, 34.77 mmol) and *p*-bromobenzaldehyde (6.43 g, 34.77 mmol) were stirred in MeOH (150 mL) at room temperature for 1 hr. Sodium cyanoborohydride (2.38 g, 35 mmol) was added and the solution was stirred at room temperature for 48 hr. Another portion of sodium cyanoborohydride (2.38 g, 35 mmol) was added and the solution was stirred at room temperature for another 48 hr. Solvent was removed under vacuum and the residue was purified by flash chromatography with DCM:MeOH (6:1) to give pure product (4.23 g, 45%) as an oil: ¹H NMR (CDCl₃, δ) 7.34 (d, 2H), 7.09 (d, 2H), 3.84 (s, 2H), 3.09 (m, 2H), 2.85 (t, 2H), 2.15 5 (t, 2H). ¹³C NMR (CDCl₃, δ) 136.72, 135.42, 128.05, 54.27, 48.46, 35.51. The product (2 g, 7.78 mmol) and sodium carbonate (1.77 g, 16.67 mmol) was dissolved in water (60 mL). Fmoc-OSu (3.7 g, 11.11 mmol) in THF (60 mL) was added in one portion. The solution was stirred at room temperature for 1 hours, then concentrated under vacuum. The residue was neutralized to pH 2 by 1 N HCl and extracted with ethyl acetate (300 mL). The extract was washed with brine (2 × 50 mL), dried over MgSO₄ and concentrated under vacuum. The residue was purified by flash chromatography with hexane:ethyl acetate (6:1) giving 2.69 g (72%) of pure product as an oil: ¹H NMR (CHCl₃, δ) 8.50 (br, 1H), 6.51-7.59 (m, 12H), 3.82-4.48 (m, 5H), 3.30 (br, 1H), 2.95 (br, 1H), 2.36 (br, 1H), 2.06 (br, 1H). ¹³C NMR (CDCl₃, δ) 175.3, 175.1, 163.7, 156.8, 156.4, 144.2, 141.8, 137.1, 132.0, 129.9, 129.2, 128.6, 128.1, 127.5, 125.1, 121.5, 120.4, 67.6, 50.9, 48.2, 47.7, 47.6, 37.2, 33.4, 32.1.

### Example 39

### Synthesis of Library 6

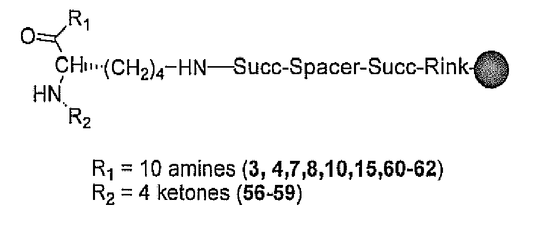

Library 6 was synthesized in parallel on PEGA₄₀₀₀ resin (0.5 g, 0.12 mmol/g, 300 -500 µm beads) according to Scheme 14 with ten different amines in position R₁ and four different ketones in position R₂, giving rise to a forty member library. The Rink linker, 54 (2 equivalents) was coupled to the resin under TBTU activation in a plastic syringe. To reverse the reactivity on the resin (from amine to carboxylate) succinic anhydride (15 equivalents) was coupled to the Rink linker using DIPEA (15 equivalents) as base (Czerwinski et al., 1998, PNAS, 95: 11520-11524). The spacer, azido-PEG-amine (n= 10, 3 equivalents), was then coupled under TBTU activation to the resin, followed by reduction of the azide with 0.1M DTT in DMF with DBU (2 equivalents). Coupling of succinic anhydride (15 equivalents) again reversed the reactivity on the resin and after Pfp activation, Fmoc-Lys-OH (5 equivalents) was coupled. After Pfp activation of the resin, the resin is divided into ten separate portions and then reacted with ten amines **(3,4,7,8,10,15,20,60-62).** The Fmoc group was removed by treatment with 20% piperidine in DMF and each one of the ten portions was further divided into four new portions (altogether 40 portions). Four different ketones (56-59, 30 equivalents) were coupled to each amine by reductive amination using NaCNBH₃ (St. Hilaire et al., 2002, J. Med. Chem. 45,: 1971-1982). Portions with ester groups were treated with 1M NaOH (7.5 h, rt.) for hydrolysis of the ester groups, the trityl groups were removed by treatment with 90% acetic acid at 60° C for 3 h and the Boc groups were removed by heating the resin in DMF at 120°C for 3 h.

### Example 40

### Synthesis of a Small Molecule Biaryl Library 7

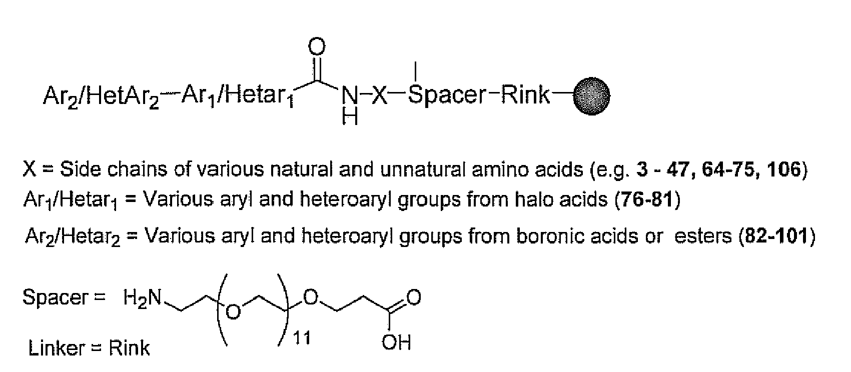

The synthetic scheme for building Library 7 having the structure Ar₂/HetAr₂-Ar₁/HetAr₁-X (Ar/HetAr_{1 and 2} aromatic or hetero aromatic ring systems (heteroatom(s): O, S, N) with various substituents e.g. Halide(s) (F, Cl), CH₃-, CF₃-, Methoxy-, thiomethyl-, alclehycle(s)- alchols- carboxylic acids-, esters-, nitrogroups etc.), the X corresponds to different amino acids, natural or unnatural chosen from **3-47** and **64-75** and **106..** In order to produce the Library 7, as shown in Scheme 15, an acid labile Fmoc-protected Rink linker (3 equivalents) was attached to NH₂-functionalised Pega₄₀₀₀ (400 mg ∼0.1 mmol/g) under TBTU activation (2.88 eq. TBTU, 4 eq. NEM, 3 eq. Fmoc-Rink-OH), in dry DMF. The acid labile linker was used in order to withstand the basic conditions used for the Suzuki cross-couplings described later. After washing with DMF (6x), the resin was treated with 20 % piperidine/DMF (for 2 + 18 min) in order to remove the Fmoc-group on the Rink linker. Addition of the spacer (Fmoc-NH-((CH₂)₂O)₁₂-CH₂-COOH) was achieved using again TBTU activation. After washing, the resin was divided into 40 wells and removal of the Fmoc group was performed with 20 % piperidine/DMF (for 2 + 18 min) and the resin washed again with DMF. To each well was coupled Fmoc-protected natural or unnatural amino acid (3 equivalents using TBTU activation. After the coupling and washing, the resin was pooled, divided into 6 portions, treated with 20 % piperidine/DMF (2 + 18 min), washed, and coupled with 6 different halo aromatic or heteroaromatic carboxylic acids or acid chlorides using TBTU activation or Bt₃N in DMF respectively. The reactions were left over night and in each case 5 eq. of the aromatic or heteroaromatic entity was used compared to the resin loading. The resins were washed and pooled again. In all of the synthetic steps described above for Library 7, Kaiser Test was used to evaluate the outcome of the reactions. The dry resins were divided into 20 vials and coupled with 20 different Aryl or Heteroaryl boronic acid or ester using modified Suzuki-Miyaura Cross-couplings (Miyaura, N.; Suzuki, A. Tetrahedron Lett. 1979, 20, 3437; Suzuki, A. Acc. Chem. Res. 1982, 15, 178). Using this strategy for the C-C coupling between the two aromatic or heteroaromatic groups, a broad variety of functional groups were tolerated. The couplings were either performed at rt. or at 60 °C. Typically 1 eq. resin was reacted with 10 eq Aryl/Heteroaryl boronic acid or ester, 5 eq. 2M K₃PO₄, 0.1 eq. Pd(PPh₃)₄ in toluene/EtOH (1:1) and the reaction mixture was purged with argon for 5 min. After the reaction, the resins were carefully washed. In some cases Pd-black was captured in the resin. However, treatment with freshly prepared 0.5% Et₂NCS₂Na/DIPEA in DMF removed Pd-black from the solid support. Finally, the resins were suspended in DMF and heated to 120 °C in a Microwave oven (<500W) for 20 min, in order to remove the Boc-protecting groups. The resins were washed with DMF (3x) and CH₂Cl₂ (5x) and dried under high vacuum overnight.

### Examples 41

### Identification of Small Molecule Ligand by MS Fragmentation

Ligands attached to single beads and isolated after screening of libraries prepared without ladder synthesis (Libraries 5 and 7) as described in Examples 36 and 40 were cleaved as described in Example 19. The samples were micropurified on C18 micor columns and fragmented using a Micromass Ultima Global. The identity of the ligand could be determined from the fragmentation.

### Example 42

### Preparation of UnLabeled Protein From PE-Treated and Basal Myocytes

Protein was extracted from myocytes prepared as described above for Example 9, using a new procedure (to preserve the activity of metalloproteases) modified from existing protocols, primarily: Arnott, et al., 1998, Anal. Biochem. 258: 1-18. The media was removed from plates and cells were washed with ice cold phosphate buffer (5ml, 10 mM, pH 7.5, supplemented with 0.15 M NaCl,) to remove traces of media components. The adhered cells were treated for 10 minutes with ice cold phosphate buffer (2 mL, 10 mM, pH 7.5, augmented with 0.15 M NaCl, 10 µg/mL E-64, 10 µg/mL Leupeptin, and 10 µg/mL Pepstatin A). The cells were scraped off the plates and then lysed (on ice) in a sonicator (2x) using 10 seconds off/10 seconds on cycles. The resulting suspension was augmented with CHAPS (1 % w/v), KCI (50 mM), NaCl (0.5 M), ZnCl₂(0.1 mM), DTT (5 mM), and urea (2 M). After 10-15 minutes on ice, the solution was centrifuged for 10 minutes at 15,000 rpm at 4 °C. The supernatant was removed and protein content quantified using the NanoOrange Protein test (Molecular Probes, Eugene, Oregon): Total protein recovered: 195 µg for PE-induced cells and 200 µg for basal cells.

The protein solutions were dialyzed extensively against 20 mM MES (2-[N-Morpholino] ethanesulfonic acid) buffer pH 6.0, supplemented with 0.15 M NaCl and 1 mM ZnCl₂to remove excess detergent and urea.

### Example 43

### Solid Phase Screening of Library 6 with UnLabetecl Myocyte Proteins

Library 6, prepared as described for Example 39, was divided into 80 portions (60-70 beads of each compound), and washed for 10 minutes (3x) with 20 mM MES (2-[N-Morpholino] ethanesulfonic acid) buffer pH 6.0, supplemented with 0.15 M NaCl, 1 mM Ca²⁺, 1mM Zn²⁺, 1 mM Mn²⁺, 1 mM Cu²⁺, and 1 mM Mg²⁺ (3 mL). Solutions (15 µL, 5 µg total protein) containing myocyte proteins, PE-induced protein or basal protein obtained as described above for Example 42, were added to the library beads. Beads containing PEGA₄ₒₒₒ resin with only linker and spacer were used as a control. The proteins and ligand libraries were incubated at 4 °C for 16 hours. The libraries were then washed with MES buffer for 5 minutes then with water for 3 x 5 min.

### Example 44

### Detection of Bound Protein by Silver Staining

In order to detect protein bound ligands, the beads were silver stained using a new procedure, modified from existing protocols described in Rabilloud, T., 1990, Electrophoresis 11(10): 785-94 and Rabilloud, T., 1992, Electrophoresis 13(7): 429-39. The library beads were fixed for 30 minutes using a solution containing ethanol (40 % v/v) and glacial acetic acid (10 % v/v). The beads were then sensitized for 30 minutes with an ethanol (30 % v/v), sodium thiosulphate (0.2 % w/w) and sodium acetate (6.8 % w/v) mixture followed by a water wash (3 x 5 min). Silver staining of the libraries was performed using a silver nitrate solution (0.25 % w/v) for 20 minutes after which excess silver from the beads was removed by washing with water (2x) for 2 minutes. A developing solution containing sodium carbonate (2.5 % w/v), formaldehyde (0.007 % w/v) and glutardialdehyde (0.125 % v/v) was added to the beads for 2-5 min. The reaction was stopped by adding a solution of sodium EDTA (1.46 % w/v). The beads were examined and compared to the control spacer beads. The beads were retained for analysis.

### Example 45

### On-bead Trypic Digest of Silver Stained Proteins

Silver stained beads containing both ligand and bound protein were transferred to RNase-and DNase-free PCR tubes. The beads were washed sequentially with water (100 µL) for 15 minutes, and acetonitrile (50 µL) then dried in a speedvac. The dry beads were mixed with 50 µL DTT (10 mM in 0.1 M ammonium bicarbonate) at 56°C for 1 hour. After cooling, the DTT was removed and 50 mM iodoacetamide in 0.1 M ammonium bicarbonate (50 µL) was added. The mixtures were incubated in the dark for 30 minutes at room temperature. The iodoacetamide was removed, the beads were washed with acetonitrile (100 µL) then dried in the speedvac. To the dried beads was added trypsin in 25 mM ammonium bicarbonate at a concentration of 75 ng/µL. The samples were incubated at 37°C for 4-5 hours. The tryptic digest supernatant was then removed and concentrated. The tryptic peptides were then micropurified using a high performance extraction disc containing octadecyl (C18) resin (3M Filtration Products, MN, USA). The discs were first swollen in acetonitrile then equilibrated with 1 % (v/v) formic acid after which the tryptic peptide sample was applied. The column was washed with 0.1% (v/v) aq TFA and the sample eluted with 1 % (v/v) TFA. The sample was analysed by MALDI-TOF-MS as described for Example 26 and the protein identified as described for Example 31.

### Example 46

### Preparation of Labeled Protein From Porcine Reproductive Respiratory Virus (PRRS) Infected and Normal Porcine Macrophages

Protein was extracted from porcine reproductive and respiratory virus infected macrophages and normal macrophages prepared using a new procedure modified from existing protocols, primarily: Arnott, et al., 1998, Anal. Biochem. 258: 1-18. After media removal, cells were treated for 10 minutes with 20 mM ice cold phosphate buffer (1 mL, pH 7.2, supplemented with 122 mM NaCl, 60 mM Benzamidine HCl, 5 mM EDTA, 10 µg/mL E-64, 10 µg/mL Leupeptin, 10 µg/mL Pepstatin A, and 1 mM PMSF. The resulting suspension was augmented with CHAPS (4 % w/v), and the cells lysed using freeze-thaw cycles. After 10-15 minutes on ice, the solution was centrifuged for 10 minutes at 15,000 rpm at 4 °C. The supernatant was removed and protein content quantified using the NanoOrange Protein test (Molecular Probes, Eugene, Oregon): Total protein recovered: 150 µg for virus-infected cells and 142.4 µg for normal cells. Fluorescent dye Cyanine 3 (Cy 3) (Amersham) was used to label protein from uninfected cells while Cyanine 2 (Cy 2) (Amersham) was used to label protein extracted from the virus-infected cells. The labeling procedures were carried out according to the manufacturer's protocol. The protein solutions (0.7 mL, 100 µg for virus-infected cells and 0.66 mL, 100 µg for normal cells) were dialyzed against a solution of 10 mM phosphate buffer, 0.15 M NaCl, pH 7.5 and then 1 M NaHCO₃ (0.03-0.06 mL) added to a final pH between 8.0-9.0. For efficient labeling, a protein:dye ratio of 50 µg:400 pmol fluorophore was maintained. The fluorophore (5 µL solution prepared by adding 2 µL of a 1 nmol/µL reconstituted fluorophore solution to 3 µL, of dry DMF) and the protein solutions were mixed thoroughly by vortexing. The samples were then centrifuged briefly in a microfuge and left on ice for 30 min in the dark. To stop the reaction, lysine (10 mM, 1 µL) was added to the samples, which were then mixed by vortexing and collected by centrifugation in a microfuge. The samples were left on ice for 10 min in the dark. The labeled protein solutions were dialyzed extensively against 10 mM phosphate buffer, 0.15 M NaCl, pH 7.5, to remove excess dye. Labeled samples were stored for at -70 °C in the dark until further use.

### Example 47

### Solid Phase Screening of a Small Molecule Library 7 with Labeled Viral and Porcine Macrophage Proteins

Ligand library 7 (200 mg), prepared as described for Example 40, was transferred to a syringe fitted with a stop-valve and the ligand-beads were washed for 10 minutes (3x) with 10 mM phosphate buffer, pH 6.8, supplemented with 0.15 M NaCl, 1 mM Ca²⁺, 1 mM Zn²⁺, 1 mM Mn²⁺, 1 mM Cu²⁺, and 1 mM Mg²⁺ (3 mL). A mixture of labeled proteins, including both porcine reproductive and respiratory viral and porcine macrophage protein mixture (660 µL, 100 µg) and macrophage protein (700 µL, 100 µg) obtained as described above for Example 46, were added to the ligand library in the syringe. The proteins and ligand library were incubated at room temperature in the dark for 16 hours. The library was then washed with buffer for 5 minutes followed by water for 3 x 5 minutes. The library was examined under a fluorescence microscope and brightly fluorescent red, green, and yellow beads (yellow indicative of both dyes red and green binding; the majority of the beads) were present, as well as unlabeled beads. The fluorescent beads separated using a bead sorter as described in Example 18.

### Example 48

### Solid Phase Screening a Small Molecule Biaryl Library 7 with Labeled Myocyte Proteins

Ligand library 7 (200 mg), prepared as described for Example 40, was transferred to a syringe fitted with a stop-valve and the ligand-beads were washed for 10 minutes (3x) with 10 mM phosphate buffer, pH 6.8, supplemented with 0.15 M NaCl, 1 mM Ca²⁺, 1 mM Zn²⁺, 1 mM Mn²⁺, 1 mM Cu²⁺, and 1 mM Mg²⁺ (3 mL). A mixture of labeled myocyte proteins, containing both PE-induced proteins (0.35 mL, 100 µg) and normal/healthy proteins (0.41 mL, 100 µg) obtained as described above for Example 11, was added to the ligand library in the syringe. The proteins and ligand library were incubated at room temperature in the dark for 16 hours. The library was then washed with buffer for 5 minutes then with water for 3 x 5 minutes. The library was examined under a fluorescence microscope and unlabelled beads, brightly fluorescent red, green, and yellow beads (yellow indicative of both dyes red and green binding; the majority of the beads) were present. The fluorescent beads were separated using a bead sorter as described in Example 18.

### Example 49

### Solid Phase Screening of Library 4 with Labeled Porcine Viral and Macrophage Proteins

Ligand library 4 (300 mg), prepared as described for Example 35, was transferred to a syringe fitted with a stop-valve and the ligand-beads were washed for 10 minutes (3x) with 10 mM phosphate buffer, pH 6.8, supplemented with 0.15 M NaCl, 1 mM Ca²⁺, 1 mM Zn²⁺, 1 mM Mn²⁺, 1 mM Cu²⁺, and 1 mM Mg²⁺ (3 mL). A mixture of labeled porcine macrophage proteins, including both porcine reproductive and respiratory virus-infected protein (660 µL, 100 µg) and normal protein (700 µL, 100 µg) obtained as described above for Example 46, was added to the ligand library in the syringe. The proteins and ligand library were incubated at room temperature in the dark for 16 hours. The library was washed with buffer for 5 minutes then with water for 3 x 5 minutes. The library was examined under a fluorescence microscope and brightly fluorescent red, green, and yellow beads (yellow indicative of both dyes red and green binding; the majority of the beads) were present, as well as unlabeled beads. The fluorescent beads were separated using a bead sorter as described in Example 18.

### Example 50

### Solid Phase Screening of Library 5 with Labeled Myocyte Protein

Ligand library 5 (250 mg), prepared as described for Example 36, was transferred to a syringe fitted with a stop-valve and the ligand-beads were washed for 10 minutes (3x) with 10 mM phosphate buffer, pH 6.8, supplemented with 0.15 M NaCl, 1 mM Ca2+, 1 mM Zn2+, 1 mM Mn2+, 1 mM Cu2+, and 1 mM Mg2+ (3 mL). A mixture of labeled myocyte proteins, including both PE-induced protein (138 µL, 40 µg) and basal protein (167 µL, 40 µg) obtained as described above for Example 11, was prepared in 1.2, mL buffer, and added to the ligand library in the syringe. The proteins and ligand library were incubated at room temperature in the dark for 16 hours. The library was washed with buffer for 5 minutes then with water for 3 x 5 minutes. The library was examined under a fluorescence microscope and brightly fluorescent red, green, and yellow beads (yellow indicative of both dyes red and green binding; the majority of the beads) were present, as well as unlabeled beads. The fluorescent beads were separated using a bead sorter as described in Example 18.

### Example 51

### Identifcation of Protein and Ligand Attached to the Same Single Bead

After sorting automatically or manually, a single bead containing labeled protein(s) bound to ligand was washed extensively with water (2X), 1 % (v/v) acetic acid (2X) and water (2X) to remove residual sheath fluid. To the bead was added trypsin in 25 mM ammonium bicarbonate solution at a concentration of 75 ng/µL. The sample was incubated at 37°C for 4-5 hours. The tryptic digest supernatant was then removed and concentrated. The tryptic peptides were then micropurified using a high performance extraction disc containing octadecyl (C18) resin (3M Filtration Products, MN, USA) The discs were initially treated with acetonitrile and then equilibrated with 1 % (v/v) formic acid. The identity of the bound protein(s) was then determined as described in Example 31. After removal of the tryptic peptide supernatant, the bead was transferred to a new Eppendorf tube (RNAse and DNAse free) and the ligand cleaved using the conditions appropriate to the linker as described in Example 19. The samples for ligand identification thus prepared were further analysed using MALDI-TOF-MS as described in Example 19 and/or using MS fragmentation as described in Example 41.

### Example 52

### Results for Myocyte Protein Screening of Library 6

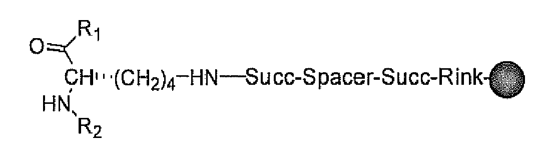

R₁ = 10 amines **(3, 4,7,8,10,15,60-62)**
R₂ = 4 ketones **(56-59)**

**Table 10: List of identified ligands and proteins for Library 6 and myocyte proteins.**

| **Entry** | **Identified Ligand** | **Identified Protein(s)** |
|---|---|---|
| **Normal Myocytes** | | |
| 1 | 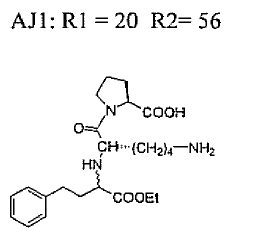 | Angiotensin converting enzyme (P22967); U2 nuclear ribonucleoprotein auxilary factor (gi 2842676). |
| 2 | 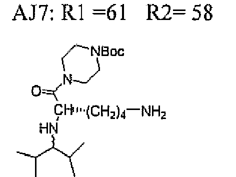 | Angiotensin converting enzyme (P22967). |
| 3 | 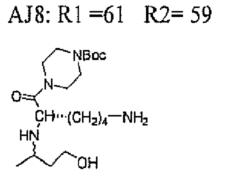 | Angiotensin converting enzyme (P22967). |
| 4 | 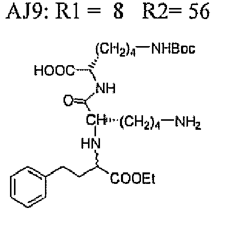 | Angiotensin converting enzyme (P22967). |
| 5 | 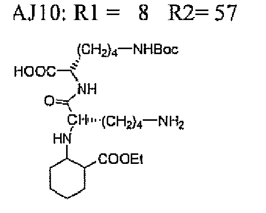 | Angiotensin converting enzyme (P22967); U2 nuclear ribonucleoprotein auxilary factor (gi 2842676). |
| 6 | 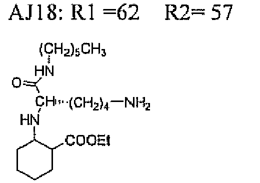 | Inward rectifier potassium channel 13 (Q9QZ65, IRKD_CAVPO); Sulfonylurea receptor 2 (Q63563); small conductance potassium channel (P58391). |
| 7 | 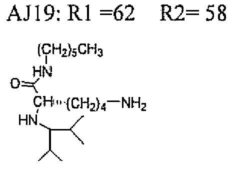 | Heat Shock protein 70kDa protein 12A (Q8KOU4); Serine/threonine protein kinase (088866); Ras GTPase activating protein 2 (GAP1M) (P58069). |
| 8 | 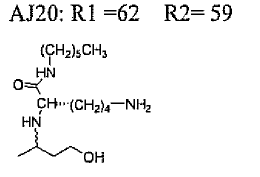 | Myosine heavy chain, nonmuscular type (AQ62812); Hypothetical protein E330017A01 (XP-148690.1). |
| 9 | 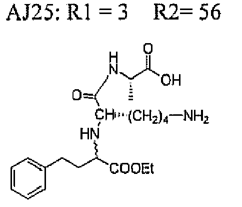 | Testase 1 or disintegrin 5 containing metalloproteinase domain 24 (gi 31981793); ADP-ribosylation factor like protein 8 (Q96KC2). |
| 10 | 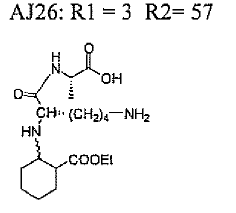 | Golgi coiled coil protein GCC185 (Q8CHG3). |
| 11 | 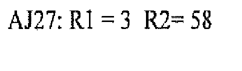 | Myosin heavy chain nonmuscular type (AQ62812). |

| **PE-Treated Myocytes** | | |
|---|---|---|
| | 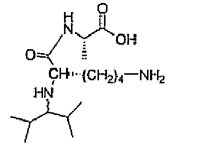 | |
| 12 | 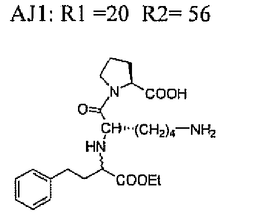 | Angiotensin converting enzyme (P22967); U2 nuclear ribonucleoprotein auxilary factor (gi 2842676). |
| 13 | 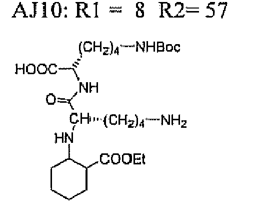 | Angiotensin converting enzyme (P22967); U2 nuclear ribonucleoprotein auxilary factor (gi 2842676). |
| 14 | 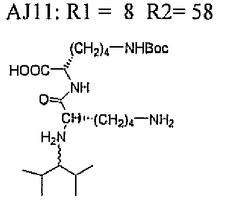 | Laminin beta 2 chain precursor B3 (P1580); ubiquitin protein ligase E3 (AO08759). |
| 15 | 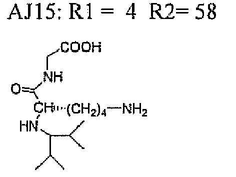 | Glycogen synthase kinase-3 beta factor (AQ9WV60). |
| 16 | 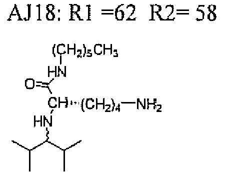 | Heat Shock protein 70kDa protein 12A (Q8KOU4). |

### Example 53

### Results for Viral/Macrophage Protein Screening of Library 7

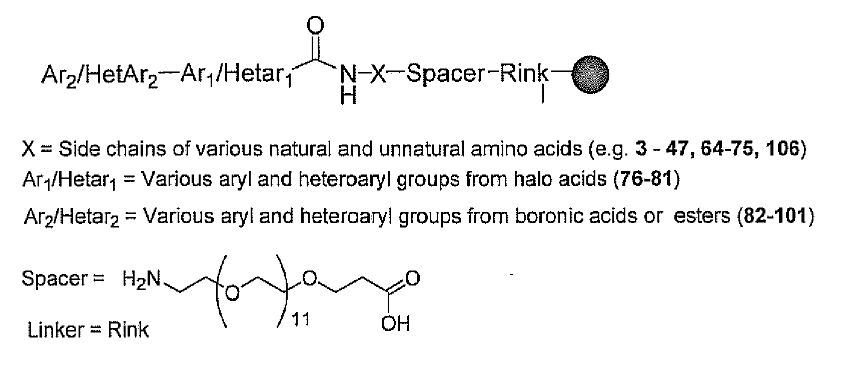

**Table 11. List of identified ligands and proteins for Library 7 and macrophage/viral proteins.**

| **Entry** | **Identified Ligand** | **Identified Protein(s)** |
|---|---|---|
| **Normal Macrophages** | | |
| 1 | 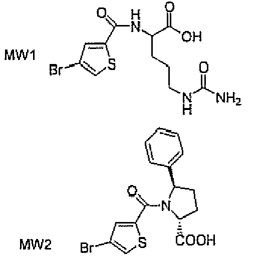 | APK1 antigen (2134769); Hyaluronan synthase 2 (gil2049586) |
| 2 | 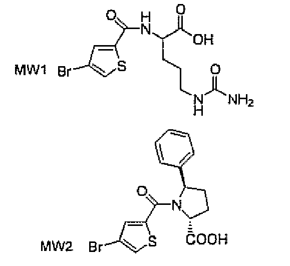 | Calreticulin 63 kDa (gi 253851); Calcium binding protein |
| 3 | 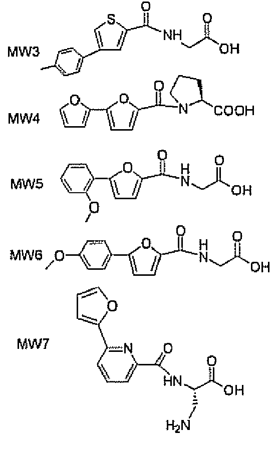 | Salt inducible kinase 2 (27529963); PVHL-ubiquitin interacting enzyme 2 (23208620); Similar to glyceral-dehyde-3-phosphate dehydrogenase (gi27686413) |

| **Virus-infected Macrophages** | | |
|---|---|---|
| 4 | 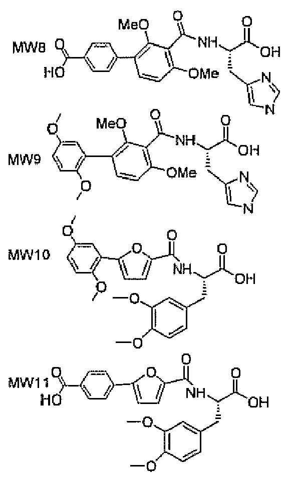 | Carbohydrate (chondroitin synthase 2) (28466981); Glucosidase II alpha-subunit (gi5452936); Similar to chromosome 9 reading frame 24 (gi29437020) (V) Envelope glycoprotein C2V3 region (gi3925092); Envelope glycoprotein (gi15705767) |
| 5 | 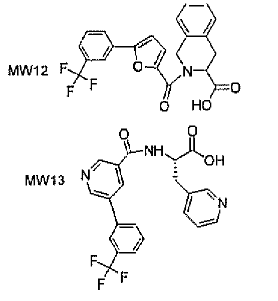 | Similar to Rho guanine nucleotide exchange factor (GEF) (gi 28510159/XP_109946.2); Similar to glyceraldehyde-3-phosphate dehydrogenase(gi 28546240/XP_205430.2); Hypothetical protein MGC7050 (gi 23601917) (V) Rev protein (gi13172687); Coat protein (gi19919954); Glycoprotein precursor (gi 731113); Leucine rich repeat:similar to Amsacta moorei Entomopoxvirus (gi 9631418) |
| 6 | 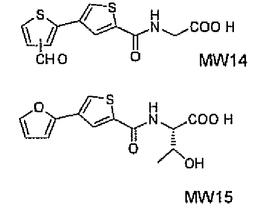 | Unnamed protein product (26336134); Serine/threonine kinase 9/cylin dependent Serine/threonine kinase 9(23271297/4507281); Caveolin 1(gi 4972627/AAD347221) (V) Envelope glycoprotein (gi 3832399) |
| 7 | 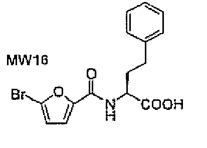 | 7 Transmembrane helix receptor (gi 21928697); DE AD (Asp-Glu-Ala-Asp) box polypeptide. (gi 19527256); 60s ribosomal protein L12 mito precursor (gi 1710600) (V) HLA-binding protein (gi 1279435); Large subunit of ribonucleotide reductase (gi 30984467) |

**(V) indicates virus proteins.**

### Example 54

### Results for Myocyte Protein Screening of Library 7

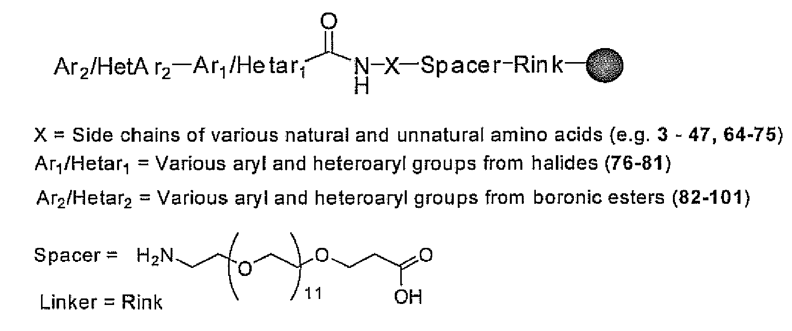

**Table 12: List of identified ligands and proteins for Library 7 and myocyte proteins.**

| **Entry** | **Identified Ligand** | **Identified Protein(s)** |
|---|---|---|
| **Normal Myocytes** | | |
| 1 | 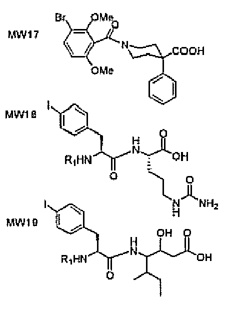 | Centaurin gamma 3 (Q96P47); Poly [ADP-ribose] polymerase- (PARP-1) (ADPRT) (NAD(+) ADP-ribosyltransferase-1) (Poly[ADP-ribose] synthetase-1) (P09874); Aortic preferentially expressed protein 1 (APEG-1) Aortic preferentially expressed protein 1 (APG1_RAT, Q63638). |
| 2 | 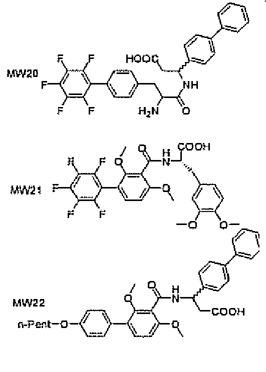 | Cytochrome P450 2C23 (Arachidonic acid epoxygenase) (CYPIIC23, P24470); Splice isoform Calcitonin, variant displaced from P41547 Calcitonin precursor (CAL0_CANFA, P22892); Adaptor protein complex AP-1 gamma-1 subunit with Golgi adaptor HA1/AP1 adaptin gamma-1 subunit) (P41547-00-00-00). |
| 3 | 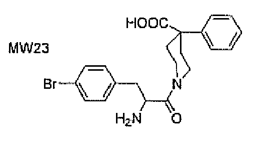 | Zinc finger protein 198 (Fused in myeloproliferative disorders protein) (Q9UbW7); Unnamed protein product (gi 12844245). |
| 4 | 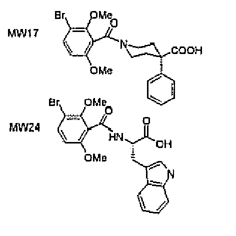 | Cytochrome P450 2C31(CYPIIC31) (CPCV_CAPAE,Q29478); Fatty acid-binding protein, (FABPI, P02693); Similar to the alpha subunit of protein kinase CK2 (gi 28527943). |
| 5 | 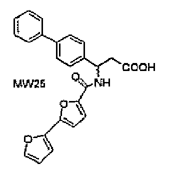 | Thyroid receptor interacting protein 11 I (TRIP-11,Q15643); Unnamed protein product (gi 26338976). |
| 6 | 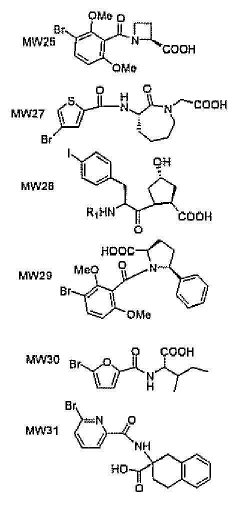 | Gap junction beta-5 protein (Connexin 31.1), (Q02739); Hypothetical protein XP_288677 (gi 208598980). |

| **Serum induced (hypertrophied) Myocytes** | | |
|---|---|---|
| 7 | | Similar to Urinary protein 3 precursor (gi 27719895); Glutamine synthetase (gi 29150629); ATP-binding cassette (sub-family B, Member 10, (Q9NRK6). |
| 8 | 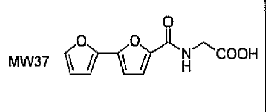 | Serine-threonine kinase (gi 474842); Hemopexin precursor (Beta-1B-glycoprotein) (P02790); 116 kDa U5 small nuclear ribonucleoprotein component (008810), |
| 9 | 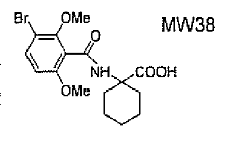 | Translin-associated protein X (Q99598); Hypothetical Protein ORF-1137 (P1 1260); Integrin alpha-1 (Laminin and collagen receptor VLA-1,CD49a) (P56199). |

### Example 55

### Results for Viral/Macrophage Protein Screening of Library 4

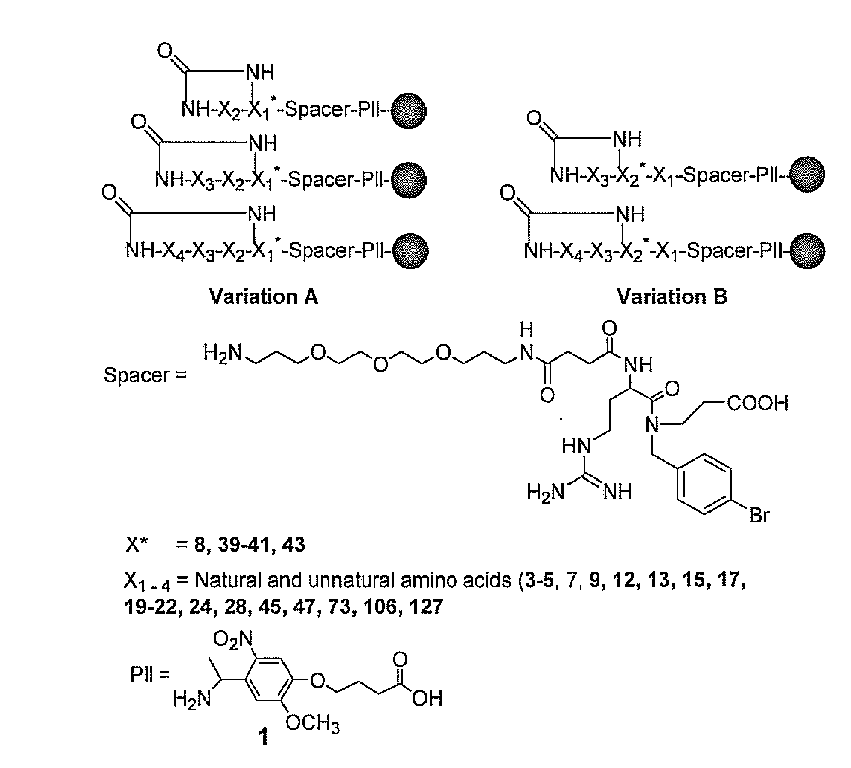

**Table 13: List of identified ligands and proteins for Library 4 and macrophage/viral proteins.**

| **Entry** | **Identified Ligand** | **Identified Protein(s)** |
|---|---|---|
| **Normal Macrophages** | | |
| 1 | 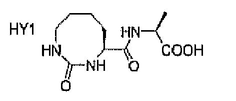 | Protein kinase C binding protein 1 also contains a zinc finger domain with protein 8) (Q9U1U4); Guanine nucleotide-binding protein G(S), alpha subunit (Adenylate cyclase-stimulating G alpha protein) (P048949). |
| 2 | 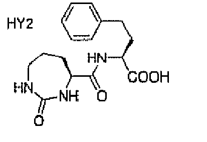 | Protein kinase byr2 (Protein kinase ste8) (MAP kinase) (P28829); Similar to protein kinase 2 (Testicular) (Q885R6); Thiamine-triphosphatase (Q8CgV7). |

### Example 56

### Results for Myocyte Protein Screening of Library 5

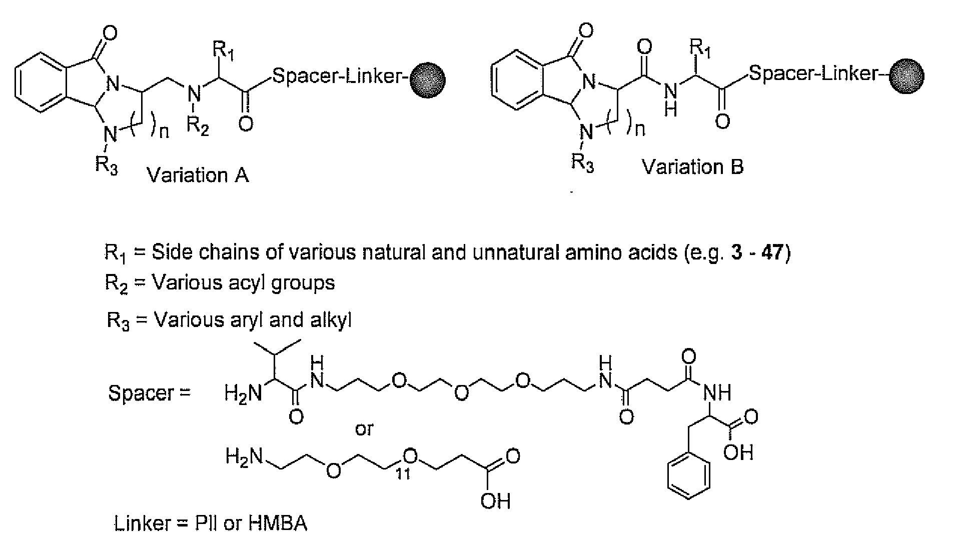

**Table 14: List of identified ligands and proteins for Library 5 and Myocyte proteins.**

| **Entry** | **Identified Ligand** | **Identified Protein(s)** |
|---|---|---|
| **Normal Myocytes** | | |
| 1 | 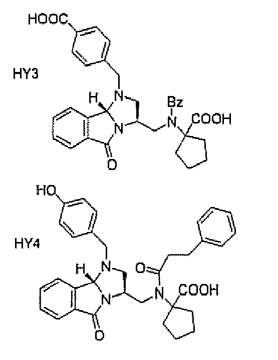 | NADH-ubiquinone oxidoreductase B 14 subunit (Q9CQZ5). |
| 2 | 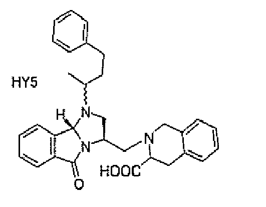 | Myosin Va (Myosin 5A, dilute myosin heavy chain, non muscle (Q9Y411). |

| **Serum induced Myocytes** | | |
|---|---|---|
| 3 | 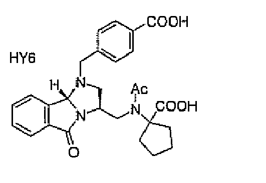 | Myosin chain (Q63358); NF-kappa B-repressing factor (Transcription factor ITBA4 protein) (O15226). |
| 4 | 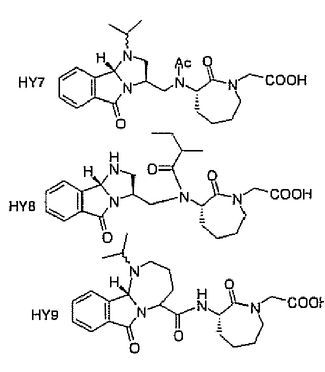 | Zinc finger protein 339 (Q9BRPO); DNA repair protein RAD52 homolog (P43351). |

This disclosure includes numerous literature and patent citations, each which is hereby incorporated by reference for all purposes. The invention is meant to be broadly construed and defined in the following claims. ITEMS
1. A process for identifying specific members of a previously unknown protein-ligand binding pair, comprising the steps of:
   (a) synthesizing a ligand library onto resin beads to form an immobilized ligand library, wherein each bead of the immobilized library comprises one member of the ligand library;
   (b) incubating the immobilized ligand library with two or more differentially labeled protein mixtures;
   (c) detecting an immobilized ligand-protein binding pair from the incubation mixture;
   (d) identifying the ligand of the specific ligand-binding pair; and
   (e) identifying the protein of the ligand-protein binding pair,
   wherein the identified ligand and protein are specific members of a previously unknown differential ligand-protein binding pair.
2. A process for identifying specific members of a previously unknown protein-ligand binding pair, comprising the steps of:
   (a) synthesizing a ligand library onto resin beads comprising polyethylene glycol to form an immobilized ligand library, wherein each bead of the immobilized library comprises one member of the ligand library;
   (b) incubating the immobilized ligand library with one or more protein mixture;
   (c) detecting an immobilized ligand-protein binding pair from the incubation mixture;
   (c) identifying the ligand of the ligand-binding pair; and
   (d) identifying the protein of the ligand-binding pair;
   wherein the identified ligand and protein are specific members of a previously unknown ligand-protein binding pair.
3. A process for identifying specific members of a previously unknown protein-ligand binding pair, comprising the steps of:
   (a) synthesizing a ligand library comprising small organic molecules onto resin beads to form an immobilized ligand library, wherein each bead of the immobilized library comprises one member of the ligand library;
   (b) incubating the immobilized ligand library with one or more protein mixture;
   (c) detecting an immobilized ligand-protein binding pair from the incubation mixture;
   (d) identifying the ligand of the ligand-binding pair; and
   (e) identifying the protein of the ligand-binding pair;
   wherein the identified ligand and protein are specific members of a previously unknown ligand-protein binding pair.
4. The process according to any of items 2 and 3, wherein the process comprises incubation with two or more differentially labeled protein mixtures.
5. The process according to any of items 1 and 4, wherein the step of detecting an immobilised ligand-protein binding pair comprises detecting a ligand of the library that binds differentially with the differentially labeled protein mixtures to form a differential ligand-protein binding pair.
6. The process according to any of items 1 and 3, wherein the resin comprises polyethylene glycol.
7. The process according to any of items 1 and 2, wherein the library comprises small organic molecules.
8. The process according to any of items 1 to 3, wherein the resin is PolyEthyleneGlycol Acrylamide copolymer (PEGA), Super Permeable Organic Combinatorial Chemistry (SPOCC) or PolyOxyEthylene-PalyOxyPropylene (POEPOP) resin.
9. The process according to any of items 1 to 3, wherein the ligand library comprises a parallel array of random modifications of one or more ligand.
10. The process of item 9, wherein said library comprises a parallel array of random modifications of a known compound and wherein said protein mixture comprises protein not previously known to bind said compound.
11. The process according to any of items 2 and 3, wherein each protein mixture is not labeled prior to incubation with the ligand library, and wherein each ligand-protein binding pair is detected after incubation by addition of a detection probe.
12. The process of item 11, wherein the detection probe is silver.
13. The process according to any of items 1 to 3, wherein each protein mixture is labeled with a detection probe, and wherein each ligand-protein binding pair is detected by detection of the probe.
14. The process of item 13, wherein at least one detection probe produces fluorescence.
15. The process of item 14, wherein at least one detection probe is selected from the group consisting of Oregon Green 514, anthranilic acid, Rhodamine red, cyanine dye 2, cyanine dye 3 and cyanine dye 5.
16. The process according to any of items 1 to 3, wherein at least one mixture of proteins is a mixture of mammalian tissue cell proteins.
17. The process according to any of items 1 to 3, wherein at least one protein mixture is a mixture of viral proteins.
18. The process according to any of items 1 to 3, wherein at least one protein mixture is a mixture of bacterial proteins.
19. The process according to any of items 1 to 3, wherein at least one protein mixture is a mixture of mammalian proteins.
20. The process according to any of items 1 to 3, wherein at least one protein mixture is a mixture of human proteins.
21. The process according to any of items 1 to 3, wherein at least one protein mixture is a mixture of plant proteins.
22. The process according to any of items 1 and 2, wherein the ligand library is a peptide library.
23. The process of item 22, wherein the ligand library comprises glycopeptides.
24. The process of item 22, wherein the ligand library comprises lipopeptides.
25. The process of item 22, wherein the ligand library comprises modified peptide scaffolds.
26. The process according to any of items 1 to 3, wherein the ligand library comprises peptidomimetics.
27. The process according to any of items 1 and 2, wherein the ligand library comprises small organic molecules.
28. The process according to any of items 1 to 3, wherein the ligand library consists of small organic molecules.
29. The process according to any of items 1 to 3, wherein at least one protein mixture comprises a family of proteins, and wherein the ligand- protein binding pair is detected by immunoassay.
30. The process according to any of items 1 to 3, wherein the ligand is identified using mass spectrometry.
31. The process according to any of items 1 to 3, wherein the ligand is identified using NMR spectroscopy.
32. The process according to any of items 1 to 3, wherein the protein is identified using mass spectrometry.
33. The process according to any of items 1 to 3, further comprising isolating a resin bead containing the immobilized ligand-protein binding pair from the bulk of the ligand library.
34. The process according to item 33, wherein the steps of identifying the ligand and identifying the protein are carried out on the isolated resin bead.
35. The process according to item 33, wherein identifying the protein involves protease treatment of the protein on the resin bead.
36. A ligand according to formula I, 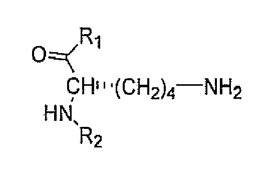 wherein said ligand is selected from the group consisting of 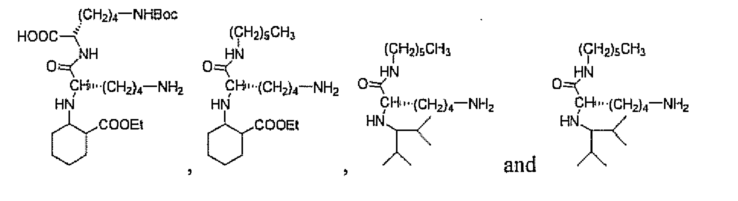
37. An isolated ligand-protein binding pair consisting of a ligand according to item 36 and a protein, wherein said isolated ligand-protein binding pair is selected from the group consisting of i) 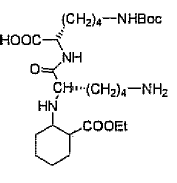 and (Angiotensin converting enzyme (P22967) or U2 nuclear ribonucleoprotein auxilary factor (gi 2842676); ii) 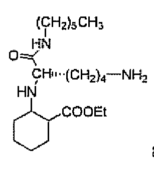 and Inward rectifier potassium channel 13 (Q9QZ65, IRKD_CAVPO); Sulfonylurea receptor 2 (Q63563); or small conductance potassium channel (P58391); iii) 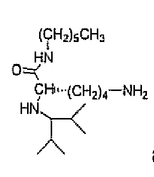 and Heat Shock protein 70kDa protein 12A (Q8KOU4); Serine/threonine protein kinase (088866) or Ras GTPase activating protein 2 (GAP1m)(P58069); and iv) 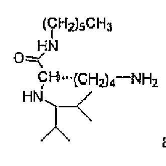 and Glycogen synthase kinase-3 beta factor (AQ9WV60).
38. A ligand according to formula II 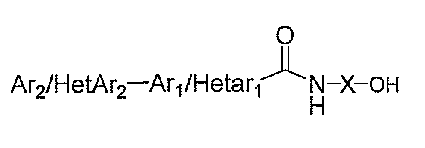 wherein X is a side chain of a compound selected from compounds 3 to 47, 64 to 75 and 106 of tables 1, 2, 3 and 7; Ar₁/Hetar₁ is selected from the group consisting of compounds 76 to 81 as indicated in table 7 and Ar₂/Hetar₂ is selected from the group consisting of compounds 82 to 101 as indicated in table 8.
39. The ligand according to item 38, wherein said ligand is selected from the group consisting of 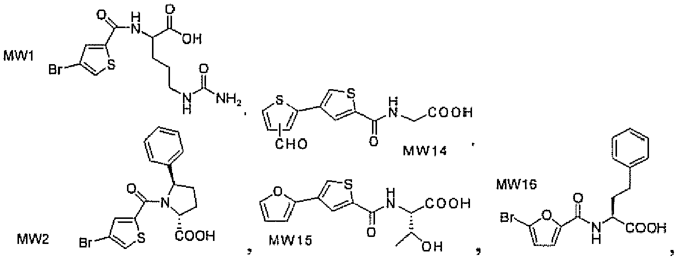 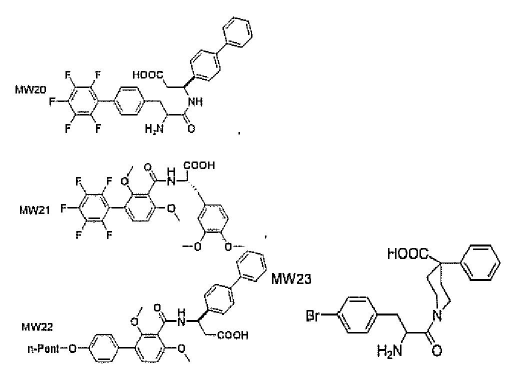 and 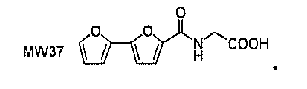
40. An isolated ligand-protein binding pairs comprising the ligand according to any of items 38 and 39; and a protein.
41. The isolated ligand-protein binding pair according to item 40, wherein said pair is selected from the group consising of
   a) MW1 and (APK1 antigen (2134769) or Hyaluronan synthase 2 (gi12049586));
   b) MW2 and (APK1 antigen (2134769) or Hyaluronan synthase 2 (gi12049586));
   c) MW14 and (Unnamed protein product (26336134); Serine/threonine kinase 9/cylin dependent Serine/threonine kinase 9(2327129714507281); Caveolin 1(gi 4972627/AAD347221) or (V) Envelope glycoprotein (gi 3832399));
   d) MW15 and (Unnamed protein product (26336134); Serine/threonine kinase 9/cylin dependent Serine/threonine kinase 9(2327129714507281); Caveolin 1(gi 4972627/AAD347221) or (V) Envelope glycoprotein (gi 3832399);
   e) MW16 and (7 Transmembrane helix receptor (gi 21928697); DEAD (Asp-Glu-Ala-Asp) box polypeptide. (gi 19527256); 60s ribosomal protein L12 mito precursor (gi 1710600); (V) HLA-binding protein (gi 1279435) or Large subunit of ribonucleotide reductase (gi 30984467));
   f) MW20 and (Cytochrome P450 2C23 (Arachidonic acid epoxygenase) (CYPIIC23, P24470); Splice isoform Calcitonin, variant displaced from P41547 Calcitonin precursor (CALO_CANFA, P22892) or Adaptor protein complex AP-1 gamma-1 subunit with Golgi adaptor HA1/AP1 adaptin gamma-1 subunit) (P41547-00-00-00));
   g) MW21 and (Cytochrome P450 2C23 (Arachidonic acid epoxygenase) (CYPIIC23, P24470); Splice isoform Calcitonin, variant displaced from P41547 Calcitonin precursor (CALO_CANFA, P22892) or Adaptor protein complex AP-1 gamma-1 subunit with Golgi adaptor HA1/AP1 adaptin gamma-1 subunit) (P41547-00-00-00));
   h) MW22 and (Cytochrome P450 2C23 (Arachidonic acid epoxygenase) (CYPIIC23, P24470); Splice isoform Calcitonin, variant displaced from P41547 Calcitonin precursor (CALO_CANFA, P22892) or Adaptor protein complex AP-1 gamma-1 subunit with Golgi adaptor HA1/AP1 adaptin gamma-1 subunit) (P41547-00-00-00));
   i) MW23 and (Zinc finger protein 198 (Fused in myeloproliferative disorders protein) (Q9UbW7) or Unnamed protein product (gi 12844245)); and
   j) MW37 and Serine-threonine kinase (gi 474842); Hemopexin precursor (Beta-1B-glycoprotein) (P02790) or 116 kDa U5 small nuclear ribonucleoprotein component (008810).
42. A ligand selected from the group consisting of 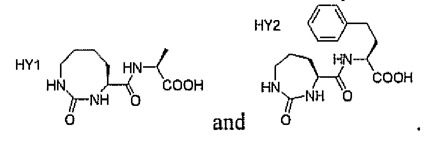
43. An isolated ligand-protein binding pair comprising a ligand according to item 42 and a protein, wherein said pair is selected from the group consisting of
   a) HY1 and (Protein kinase C binding protein 1 also contains a zinc finger domain with protein 8) (Q9U1U4) or Guanine nucleotide-binding protein G(S), alpha subunit (Adenylate cyclase-stimulating G alpha protein) (P048949)); and
   b) HY2 and (Protein kinase byr2 (Protein kinase ste8) (MAP kinase) (P28829); Similar to protein kinase 2 (Testicular) (Q885R6) or Thiamine-triphosphatase (Q8CgV7)).
44. A ligand according to formula IV: 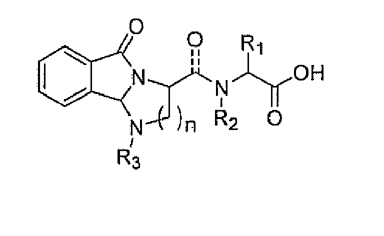 Wherein R₁ is the side chain of an amino acid selected from the group consisting of compound 3 to 47, 64, 66, 67, 71, 73, 74, 103 to 106 and 128 of tables 1, 2, 3, 7 and 9, R₂ is selected from the group consisting of compounds 117 to 126 as outlined in table 9 and hydrogen and R₃ is selected from the group consisting of compounds 103 to 116 and 128 of table 9.
45. The ligand according to item 44, wherein said ligand is selected from the group consisting of 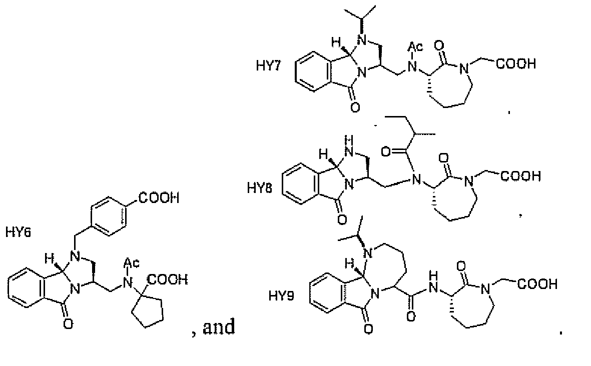
46. An isolated ligand-protein binding pair comprising a ligand according to any of items 44 and 45; and a protein.
47. The isolated ligand-protein binding pair according to item 46, wherein said binding pair is selected from the group consisting of:
   a) HY6 and (Myosin chain (Q63358) or NF-kappa B-repressing factor (Transcription factor ITBA4 protein) (015226)).
   b) HY7 and (Zinc finger protein 339 (Q9BRPO) or DNA repair protein RAD52 homolog (P43351)).
   c) HY8 and (Zinc finger protein 339 (Q9BRPO) or DNA repair protein RAD52 homolog (P43351).
   d) HY9 and Zinc finger protein 339 (Q9BRPO) or DNA repair protein RAD52 homolog (P43351).
48. A ligand comprising or consisting of Pya-Hyp-Hyp-Phe-Acm-Tyr [SEQ ID NO: 8].
49. An isolated ligand-protein binding pair comprising:
   a) The ligand according to item 48; and
   b) Troponin T, Growth hormone receptor, or Protein kinase.
50. A ligand comprising or consisting His-Tyr-Pip-Thr-Acm-Abi [SEQ ID NO: 14];
51. An isolated ligand-protein binding pair comprising:
   a) The ligand according to item 50; and
   b) Chain C P27 cyclin A-CDK2 complex: (Cyclin A?) (gi2392395); Hypothetical protein XP_154035.
52. A ligand comprising or consisting of Phe-Thr-Pya-Pip-Asp-His [SEQ ID NO: 11].
53. An isolated ligand-protein binding pair comprising:
   a) The ligand according to item 52; and
   b) Sodium channel (gi 18591322) (M); Chloride channel (gi 6978663/4502867) (M)) Troponin I (gi 1351298);; Zn Finger protein (gi 18591322) (MA); SPTR (peroxisomal Ca dependent solute carrier (putative) (gi 12853685); Beta-2 adnergic receptor (gi 12699028)
54. A ligand comprising or consisting of Phe-Abi-Pal-Hyp-Thr-Hyp [SEQ ID NO: 65].
55. An isolated ligand-protein binding pair comprising:
   a) The ligand according to item 54; and
   b) Zinc finger associated protein, Ribosomal proteins, or Protein phosphatase.
56. A ligand comprising or consisting of Phe-Gua-Pal-Tyr-Gua-Tyr [SEQ ID NO: 66].
57. An isolated ligand-protein binding pair comprising:
   a) The ligand according to item 56; and
   b) Glucose-6-Phosphatase, Succinate dehydrogenase, ARL-interacting protein,
   SPTR, or Nucleic acid binding protein.
58. A ligand comprising or consisting of Abi-Thr-Hyp-Hyp-His-- [SEQ ID NO: 67].
59. An isolated ligand-protein binding pair comprising:
   a) The ligand according to item 58; and
   b) Phosphofructokinase, Selenium binding protein, Serine arginine rich protein kinase, Guanylate kinase, Protein tyrosine kinase, Alkaline phosphatase, Symporter, SPTR, WAP-protein, GTP Hydrolase, or Actin filament.
60. A ligand comprising or consisting of Pya-Gua-Abi-Asp-Abi-Tyr [SEQ ID NO: 69].
61. An isolated ligand-protein binding pair comprising:
   a) The ligand according to item 60; and
   b) SPTR (gi 20869775) (M); Ribosomal proteins (60s) (gi 20875941/6677773); (Calcium channel (gi 3202010) (M); Slo channel protein isoform (gi 3644046) (M); Potassium conductance calcium activated channel (gi 6754436,NP_034740 ) (M); ; Regulator of G-protein signalling 8 (gi 9507049)..
62. A ligand comprising or consisting of Abi-Phe-Abi-Phe-Che-Tyr [SEQ ID NO: 18].
63. An isolated ligand-protein binding pair comprising:
   a) The ligand according to item 62; and
   b) Cathepsin E, Ribosomal protein, Actin binding protein, or Amino acid transferase.
64. A ligand comprising or consisting of Pal-Gly-Abi-Hyp-Pya-Trp [SEQ ID NO: 56
65. An isolated ligand-protein binding pair comprising:
   a) The ligand according to item 64; and
   b) NAS putative unclassified (gi 12861084); Putative Zn finger protein 64 (gi 12849329).
66. A ligand comprising or consisting of T(Sa)-F-G-I-W [SEQ ID NO: 21].
67. An isolated ligand-protein binding pair comprising:
   a) The ligand according to item 66; and
   b) Histidine synthetase or aspartate carbamoyl transferase.
68. A ligand comprising or consisting of T(Sa)-G-V-F-L [SEQ ID NO: 23].
69. An isolated ligand-protein binding pair comprising:
   a) The ligand according to item 68; and
   b) 50 S ribosomal protein, heme binding lipoprotein, regulator for D-glucarate, D-glycerate and D-galactarate, or glutamine tRNA synthetase.
70. A ligand comprising or consisting of T(Sa)-H-W-V-V [SEQ ID NO: 27].
71. An isolated ligand-protein binding pair comprising:
   a) The ligand according to item 70; and
   b) Pyruvoyl dependent aspartate decarboxylase, colicin E2, or Histidine kinase.
72. A ligand comprising or consisting of T(Sa)-H-L-G-Y [SEQ ID NO: 28].
73. An isolated ligand-protein binding pair comprising:
   a) The ligand according to item 72; and
   b) phosphomannose isomerase.
74. A ligand comprising or consisting of T(Sa)-M-V-N-W [SEQ ID NO: 32].
75. An isolated ligand-protein binding pair comprising:
   a) The ligand according to item 74; and
   b) ATP dependent helicase or mob C.
76. A ligand comprising or consisting of T(Sa)-H-W-H-L [SEQ ID NO: 35].
77. An isolated ligand-protein binding pair comprising:
   a) The ligand according to item 76; and
   b) Chorismate mutase, xanthine dehydrogenase, or carbamoyl phosphate synthetase.
78. A ligand comprising or consisting of T(Sa)-Q-P-G-M [SEQ ID NO: 63]
79. An isolated ligand-protein binding pair comprising:
   a) The ligand according to item 78; and
   b) ATP dependent helicase: HrpA homolog (NCBIBAA15034); Putative protease ydcP percursor (NCBI P76104)..
80. A ligand comprising or consisting of Gly-Pro-Lys-Lys-Tyr-His [SEQ ID NO: 44].
81. An isolated ligand-protein binding pair comprising:
   a) The ligand according to item 80; and
   b) Con A, *P. sativum* lectin, or *L. culinaris* lectin.
82. Use of a protein selected from the group consisting of Sodium channel (gi 18591322) Chloride channel (gi 6978663/4502867) ); Zn Finger protein (gi 18591322) SPTR (peroxisomal Ca dependent solute carrier (putative) (gi 12853685); Beta-2 adrenergic receptor (gi 12699028), Zinc finger associated protein (gi 20304091); Glucose-6-Phosphatase (gi 6679893/15488608), Succinate dehydrogenase, ARL-interacting protein (gi 4927202); Nucleic acid binding protein; Phosphofructokinase (gi 7331123), Selenium binding protein (gi 8848341/6677907), Serine arginine rich protein kinase; Guanylate kinase (gi 20986250),Calcium channel (gi 3202010 Slo channel protein isoform (gi 3644046) Potassium conductance calcium activated channel (gi 6754436,NP_034740) Regulator of G-protein signalling 8 (gi 9507049); Regulator of G-protein signalling 14, Cathepsin E (gi 4503145), Putative Zn finger protein 64 (gi 12849329); Histidine synthetase (gi 15803037); aspartate carbamoyl transferase (pyrI_E.coli); 50 S ribosomal protein; heme binding lipoprotein (gi 4062402/40624079glutamine tRNA synthetase (gi 146168); Pyruvoyl dependent aspartate decarboxylase (gi 3212459), Histidine kinase (part belongs to narQ_E.coli); phosphomannose isomerase (gi 147164); ATP dependent helicase (gi 2507332/16128141); mob C (gi 78702); Chorismate mutase (gi 1800006), carbamoyl phosphate synthetase (carB_E.coli); ATP dependent helicase: HrpA homolog (NCBIBAA15034); Inward rectifier potassium channel 13 (Q9QZ65, IRKD_CAVPO); Sulfonylurea receptor 2 (Q63563); small conductance potassium channel (P58391), Serine/threonine protein kinase (088866); Ras GTPase activating protein 2 (GAP1m) (P58069), Glycogen synthase kinase-3 beta factor (AQ9WV60), Serine/threonine kinase 9/cylin dependent Serine/threonine kinase 9(23271297/4507281); NF-kappa B-repressing factor (Transcription factor ITBA4 protein) (015226), HLA-binding protein (gi 1279435) and Large subunit of ribonu-cleotide reductase (gi 30984467) as drug target, in a method to identify one or more drugs for the treatment of a clinical condition.
83. Use according to item 82, wherein the protein is selected from the group consisting of Chaperone DnaK (dnak_E.coli), Histidine synthetase (gi 15803037), aspartate carbamoyl transferase (pyrI_E. coli), glutamine tRNA synthetase (gi 146168). tyrosine protein kinase (gi 20140365), citrate synthetase (CISY_E.coli), Pyruvoyl dependent aspartate decarboxylase (gi 3212459), colicin E2. (gi809671/809683), Histidine kinase (part belongs to narQ_E.coli), Protein involved in lipopolysaccharide biosynthesis (gi 16131496), phosphomannose isomerase (gi147164), high affinity potassium transport system (kdpC_E.coli), ATP dependent helicase (gi 2507332/16128141), mob C (gi 78702); Orf hypothetical protein (yciL_E.coli), outer membrane pyruvate kinase (gi 16129807/15831818), alkaline phosphatase, , Chorismate mutase (gi 1800006), carbamoyl phosphate synthetase (carB_E,coli); Glutamate synthase (NaDPH) (gi 2121143), protein involved in flagellar biosynthesis and motor switching component,), Serine tRNA synthetase (gi15830232) and ATP dependent helicase: HrpA homolog (NCBIBAA15034).
84. Use according to item 82, wherein the protein is selected from the group consisting of transpoase, proteins involved in Chaperone DnaK (dnak_E.coli), transposase (gi 158316821), Histidine synthetase (gi 15803037), aspartate carbamoyl transferase (pyrI_E. coli), transcriptional regulator (gi 18265863 glutamine tRNA synthetase (gi146168). tyrosine protein kinase (gi 20140365), citrate synthetase (CISY_E.coli), Pyruvoyl dependent aspartate decarboxylase (gi 3212459), Histidine kinase (part belongs to narQ_E.coli), Protein involved in lipopolysaccharide biosynthesis (gi 16131496), phosphomannose isomerase (gi 147164), ATP dependent helicase (gi 2507332/16128141), Chorismate mutase (gi 1800006), carbamoyl phosphate synthetase (carB_E.coli); Glutamate synthase (NaDPH) (gi 2121143), Serine tRNA synthetase (gir15830232), ATP dependent helicase: HrpA homolog (NCBIBAA15034), HLA-binding protein (gi 1279435); Large subunit of ribonucleotide reductase (gi 30984467)
85. Use according to any of items 83 and 84, wherein the clinical condition is an infection.
86. Use according to item 82, wherein the protein is selected from the group consisting of Ca2+/Calmodulin activated Myosin light chain kinase (gi 284660), Regulator of G-Protein Signalling (RGS14) variant (gi 2708808), SPTR (gi 20837095), Sodium channel (gi 18591322); Chloride channel (gi 6978663/4502867); Zn Finger protein (gi 18591322); SPTR (peroxisomal Ca dependent solute carrier (putative) (gi 12853685); Beta-2 adnergic receptor (gi 12699028); Serine/threonine Protein kinase (gi 5730055); Chain C P27 cyclin A-CDK2 complex: (Cyclin A?) (gi2392395); Membrane spanning 4-domain subfamily A member II (gi7435941); Zinc finger associated protein (gi 20304091); Serine arginine rich protein kinase; SPTR (gi 20869775); Calcium channel (gi 3202010); Slo channel protein isoform (gi 3644046); Potassium conductance calcium activated channel (gi 6754436,NP_034740);; Regulator of G-protein signalling 8 (gi 9507049); Cathepsin E (gi 4503145); NAS putative unclassified (gi 12861084); Putative Zn finger protein 64 (gi 12849329); Cell surface glycoprotein (gi 23603627); Hypothetical protein (XP-179829; gi 14720727); Orphan Nuclear receptor similar to hsp40 (NRID 26166582, Inward rectifier potassium channel 13 (Q9QZ65, IRKD_CAVPO); Sulfonylurea receptor 2 (Q63563); small conductance potassium channel (P58391), Serine/threonine protein kinase (088866); Ras GTPase activating protein 2 (GAP1m) (P58069), Glycogen synthase kinase-3 beta factor (AQ9WV60), Serine/threonine kinase 9/cylin dependent Serine/threonine kinase 9(23271297/4507281); NF-kappa B-repressing factor (Transcription factor ITBA4 protein) (015226),.
87. Use according to item 82, wherein the proteinis selected from the group consisting of Ca2+/Calmodulin activated Myosin light chain kinase (gi 284660), Regulator of G-Protein Signalling (RGS14) variant (gi 2708808), SPTR (gi 20837095), Zn Finger protein (gi 18591322); SPTR (peroxisomal Ca dependent solute carrier (putative) (gi 12853685); Beta-2 adrenergic receptor (gi 12699028); Serine/threonine Protein kinase (gi 5730055); Chain C P27 cyclin A-CDK2 complex: (Cyclin A?) (gi2392395); Zinc finger associated protein (gi 20304091); Serine arginine rich protein kinase; SPTR (gi 20869775); Regulator of G-protein signalling 8 (gi 9507049); Cathepsin E (gi 4503145); Putative Zn finger protein 64 (gi 12849329); Orphan Nuclear receptor similar to hsp40 (NRID 26166582),Inward rectifier potassium channel 13 (Q9QZ65, IRKD_CAVPO); small conductance potassium channel (P58391), Serine/threonine protein kinase (088866); Ras GTPase activating protein 2 (GAP1m) (P58069), Glycogen synthase kinase-3 beta factor (AQ9WV60), Serine/threonine kinase 9/cylin dependent Serine/threonine kinase 9(23271297/4507281); NF-kappa B-repressing factor (Transcription factor ITBA4 protein) (O15226),
88. Use according to any of items 86 and 87, wherein the clinical condition is a cardiovascular disease.

## Claims

1. A process for identifying specific members of a previously unknown protein-ligand binding pair, comprising the steps of:
(a) synthesizing a ligand library onto resin beads to form an immobilized ligand library, wherein each bead of the immobilized library comprises one member of the ligand library;
(b) incubating the immobilized ligand library with one or more protein mixture;
(c) detecting an immobilized ligand-protein binding pair from the incubation mixture;
(d) isolating the resin bead comprising the ligand-protein binding pair; and
(e) identifying the ligand of the ligand-binding pair on the isolated resin bead, wherein at least part of the identification process is performed directly on the bead; and
(f) identifying the protein of the ligand-binding pair on the isolated resin bead, wherein at least part of the identification process is performed directly on the bead;
wherein the identified ligand and protein are specific members of a previously unknown ligand-protein binding pair.

2. The process according to claim 1, wherein the resin comprises polyethylene glycol.

3. The process according to claim 1, wherein the library comprises small organic molecules , and wherein said small organic molecules are non-oligomeric, carbon containing compounds having a size of less than 600 mass units;

4. The process according to claim 1, wherein each protein mixture is not labelled prior to incubation with the ligand library, and wherein each ligand-protein binding pair is detected after incubation by addition of a detection probe.

5. The process according to claim 1, wherein each protein mixture is labelled with a detection probe, and wherein each ligand-protein binding pair is detected by detection of the probe.

6. The process of claim 5, wherein at least one detection probe produces fluorescence.

7. The process according to claim 1, wherein at least one protein mixture is derived from living cells.

8. The process according to claim 1, wherein at least one protein mixture is a mixture of mammalian proteins.

9. The process according to claim 1, wherein at least one protein mixture is a mixture of plant proteins.

10. The process according to claim 1, wherein the ligand library is a peptide library.

11. The process according to claim 1, wherein the ligand library comprises peptidomimetics.

12. The process according to claim 1, wherein at least one protein mixture comprises a family of proteins, and wherein the ligand- protein binding pair is detected by immunoassay.

13. The process according to claim 1, wherein the ligand is identified using mass spectrometry.

14. The process according to claim 1, wherein the ligand is identified using NMR spectroscopy.

15. The process according to claim 1, wherein the protein is identified using mass spectrometry.

16. A ligand according to formula IV: 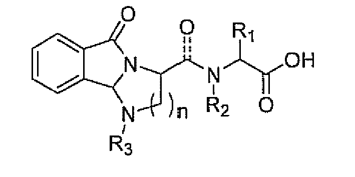 Wherein R₁ is the side chain of an amino acid selected from the group consisting of compound 3 to 47, 64, 66, 67, 71, 73, 74, 103 to 106 and 128 of tables 1, 2, 3, 7 and 9, R₂ is selected from the group consisting of compounds 117 to 126 as outlined in table 9 and hydrogen and R₃ is selected from the group consisting of compounds 103 to 116 and 128 of table 9.

17. The ligand according to claim 16, wherein R₁ is the side chain of an amino acid selected from the group consisting of compound 3, 4, 12, 13, 15, 20 to 24, 28, 29, 31, 47, 64, 66, 67, 71, 73, 74, 103 to 106 and 128 of tables 1, 2, 3, 7 and 9; R₂ is selected from the group consisting of compounds 117 to 126 as outlined in table 9 and hydrogen, with the proviso that if the optional carbonyl group is present R₂ is hydrogen, and if the optional carbonyl group is not present, R₂ is an acyl group selected from the group consisting of compounds 117-126 as outlined in table 9; and R₃ is selected from the group consisting of compounds 103 to 116 and 128 of table 9.

18. The ligand according to claim 17, wherein n = 3 or n = 1.

19. The ligand according to claim 16, wherein said ligand is selected from the group consisting of


SEQUENCE LISTING

<110> Novo Nordisk A/S

<120> AFFINITY FISHING FOR LIGANDS AND PROTEIN RECEPTORS

<130> P 782 EP01

<160> 52

<170> PatentIn version 3.1

<210> 1

<211> 6

<212> PRT

<213> Artificial Sequence

<220>

<223> Peptide Spacer

<400> 1

Gly Pro Pro Phe Pro Phe
1               5

<210> 2

<211> 7

<212> PRT

<213> Artificial Sequence

<220>

<223> Peptide Spacer

<400> 2

Ala Pro Arg Pro Pro Arg Ala
1               5

<210> 3

<211> 6

<212>  PRT

<213>  Artificial Sequence

<220>

<223>  Binding ligand from peptide library

<220>

<221>  MISC_FEATURE

<222>  (1)..(1)

<223>  Xaa is Pip

<220>

<221>  MISC_FEATURE

<222>  (2)..(3)

<223>  Xaa is Pal

<220>

<221>  MISC_FEATURE

<222>  (5)..(5)

<223>  Xaa is Pya

<220>

<221>  MISC_FEATURE

<222>  (6)..(6)

<223>  Xaa is Pip

<400>  3

Xaa Xaa Xaa Phe Xaa Xaa
1               5

<210>  4

<211>  6

<212>  PRT

<213>  Artificial Sequence



<220>

<223>  Binding ligand from peptide library

<220>

<221>  MISC_FEATURE

<222>  (1)..(1)

<223>  Xaa is Pya



<220>

<221>  MISC_FEATURE

<222>  (2)..(3)

<223>  Xaa is Hyp



<220>

<221>  MISC_FEATURE

<222>  (5)..(5)

<223>  Xaa is Acm



<400>  4

Xaa Xaa Xaa Phe Xaa Tyr
1               5


<210>  5

<211>  6

<212>  PRT

<213>  Artificial Sequence

<220>

<223>  Binding ligand from peptide library

<220>

<221>  MISC_FEATURE

<222>  (1)..(1)

<223>  Xaa is Pya


<220>

<221>  MISC_FEATURE

<222>  (2)..(2)

<223>  Xaa is Gua


<220>

<221>  MISC_FEATURE

<222>  (3)..(3)

<223>  Xaa is Pip


<220>

<221>  MISC_FEATURE

<222>  (4)..(4)

<223>  Xaa is Acc


<220>

<221>  MISC_FEATURE

<222>  (6)..(6)

<223>  Xaa is Pip


<400>  5

Xaa Xaa Xaa Xaa Phe Xaa
1               5

<210>  6

<211>  6

<212>  PRT

<213>  Artificial Sequence

<220>

<223>  Binding ligand from peptide library

<220>

<221>  MISC_FEATURE

<222>  (2)..(2)

<223>  Xaa is Aze

<220>

<221>  MISC_FEATURE

<222>  (6)..(6)

<223>  Xaa is Aze

<400>  6

Phe Xaa Gly His Gly Xaa
1               5

<210>  7

<211>  6

<212>  PRT

<213>  Artificial Sequence

<220>

<223>  Binding ligand from peptide library

<220>

<221>  MISC_FEATURE

<222>  (3)..(3)

<223>  Xaa  is Pya

<220>

<221>  MISC_FEATURE

<222>  (4)..(4)

<223>  Xaa  is Pip

<400>  7

Phe Thr Xaa Xaa Asp His
1               5

<210>  8

<211>  6

<212>  PRT

<213>  Artificial Sequence

<220>

<223>  Binding ligand from peptide library

<220>

<221>  MISC_FEATURE

<222>  (2)..(2)

<223>  Xaa  is Ppy

<220>

<221>  MISC_FEATURE

<222>  (3)..(3)

<223>  Xaa  is Acc

<220>

<221>  MISC_FEATURE

<222>  (5)..(5)

<223>  Xaa  is Ppy

<220>

<221>  MISC_FEATURE

<222>  (6)..(6)

<223>  Xaa  is Hpy

<400>  8

Phe Xaa Xaa Ala Xaa Xaa
1               5

<210>  9

<211>  6

<212>  PRT

<213>  Artificial Sequence

<220>

<223>  Binding ligand from peptide library

<220>

<221>  MISC_FEATURE

<222>  (2)..(2)

<223>  Xaa is Abi

<220>

<221>  MISC_FEATURE

<222>  (3)..(3)

<223>  Xaa is Pal

<220>

<221>  MISC_FEATURE

<222>  (4)..(4)

<223>  Xaa is Hyp

<220>

<221>  MISC_FEATURE

<222>  (6)..(6)

<223>  Xaa is Hyp

<400>  9

Phe Xaa Xaa Xaa Thr Xaa
1               5

<210>  10

<211>  6

<212>  PRT

<213>  Artificial Sequence

<220>

<223>  Binding ligand from peptide library

<220>

<221>  MISC_FEATURE

<222>  (2)..(2)

<223>  Xaa is Gua

<220>

<221>  MISC_FEATURE

<222>  (3)..(3)

<223>  Xaa is Pal

<220>

<221>  MISC_FEATURE

<222>  (5)..(5)

<223>  Xaa is Gua


<400>  10

Phe Xaa Xaa Tyr Xaa Tyr
1               5


<210>  11

<211>  6

<212>  PRT

<213>  Artificial Sequence


<220>

<223>  Peptide

<220>

<221>  MISC_FEATURE

<222>  (1)..(1)

<223>  Xaa is Pal


<220>

<221>  MISC_FEATURE

<222>  (2)..(2)

<223>  Xaa is Abi


<220>

<221>  MISC_FEATURE

<222>  (5)..(5)

<223>  Xaa is Abi

<400>  11

Xaa Xaa Gly Gly Xaa His
1               5


<210>  12

<211>  6

<212>  PRT

<213>  Artificial Sequence


<220>

<223>  Binding ligand from peptide library

<220>

<221>  MISC_FEATURE

<222>  (1)..(1)

<223>  Xaa is Abi


<220>

<221>  MISC_FEATURE

<222>  (3)..(4)

<223>  Xaa is Hyp


<220>

<221>  MISC_FEATURE

<222>  (6)..(6)

<223>  Xaa is unknown


<400>  12

Xaa Thr Xaa Xaa His Xaa
1               5

<210>  13

<211>  6

<212>  PRT

<213>  Artificial Sequence

<220>

<223>  Binding ligand from peptide library

<220>

<221>  MISC_FEATURE

<222>  (1)..(1)

<223>  Xaa is Pya

<220>

<221>  MISC_FEATURE

<222>  (2)..(2)

<223>  Xaa is Gua

<220>

<221>  MISC_FEATURE

<222>  (3)..(3)

<223>  Xaa is Abi

<220>

<221>  MISC_FEATURE

<222>  (5)..(5)

<223>  Xaa is Abr

<400>  13

Xaa Xaa Xaa Asp Xaa Tyr
1               5

<210>  14

<211>  6

<212>  PRT

<213>  Artificial Sequence

<220>

<223>  Binding ligand from peptide library

<220>

<221>  MISC_FEATURE

<222>  (1)..(1)

<223>  Xaa is Abi

<220>

<221>  MISC_FEATURE

<222>  (3)..(3)

<223>  Xaa is Abi

<220>

<221>  MISC_FEATURE

<222>  (5)..(5)

<223>  Xaa is Che

<400>  14

Xaa Phe Xaa Phe Xaa Tyr
1               5

<210>  15

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)


<400>  15

Xaa Phe Asn His Ser
1               5


<210>  16

<211>  5

<212>  PRT

<213>  Artificial Sequence


<220>

<223>  Binding ligand from peptide library

<220>

<221>  MISC_FEATURE

<222>  (1)..(1)

<223>  Xaa is T(Sa)


<400>  16

Xaa Phe Ala Leu Val
1               5


<210>  17

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  17

Xaa Phe Gly Ile Trp
1               5

<210>  18

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  18

Xaa Phe Gly Ile Met
1               5

<210>  19

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  19

Xaa Gly Val Phe Leu
1               5

<210>  20

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  20

Xaa Tyr Ser Met Pro
1               5

<210>  21

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  21

Xaa Leu Ser Trp Trp
1               5

<210>  22

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  22

Xaa His Trp His Ile
1               5

<210> 23

<211> 5

<212> PRT

<213> Artificial Sequence

<220>

<223> Binding ligand from peptide library

<220>

<221> MISC_FEATURE

<222> (1)..(1)

<223> Xaa is T(Sa)

<400> 23

Xaa His Trp Val Val
1               5

<210> 24

<211> 5

<212> PRT

<213> Artificial Sequence

<220>

<223> Binding ligand from peptide library

<220>

<221> MISC_FEATURE

<222> (1)..(1)

<223> Xaa is T(Sa)

<400> 24

Xaa His Leu Gly Tyr
1               5

<210> 25

<211> 5

<212> PRT

<213> Artificial Sequence

<220>

<223> Binding ligand from peptide library

<220>

<221> MISC_FEATURE

<222> (1)..(1)

<223> Xaa is T(Sa)

<400> 25

Xaa Ile Tyr Leu Phe
1               5

<210> 26

<211> 5

<212> PRT

<213> Artificial Sequence

<220>

<223> Binding ligand from peptide library

<220>

<221> MISC_FEATURE

<222> (1)..(1)

<223> Xaa is T(Sa)

<400> 26

Xaa Phe Gly Leu Met
1               5

<210>  27

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  27

Xaa Trp Val Asn Met
1               5

<210>  28

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  28

Xaa Met Val Asn Trp

1               5

<210>  29

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  29

Xaa His Ile Gly Tyr
1               5

<210>  30

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  30

Xaa Leu Tyr Leu Phe
1               5

<210>  31

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  31

Xaa His Trp His Leu
1               5

<210>  32

<211>  5

<212>  PRT

<213>  Artificial Sequence

<220>

<223>  Binding ligand from peptide library

<220>

<221>  MISC_FEATURE

<222>  (1)..(1)

<223>  Xaa is T(Sa)

<400>  32

Xaa Phe Val Trp His
1               5


<210>  33

<211>  5

<212>  PRT

<213>  Artificial Sequence



<220>

<223>  Binding ligand from peptide library

<220>

<221>  MISC_FEATURE

<222>  (1)..(1)

<223>  Xaa is T(Sa)


<400>  33

Xaa Leu Tyr Ile Phe
1               5


<210>  34

<211>  6

<212>  PRT

<213>  Artificial Sequence



<220>

<223>  Binding ligand from peptide library

<220>

<221>  MISC_FEATURE

<222>  (1)..(1)

<223>  Xaa is ManS

<220>

<221>  MISC_FEATURE

<222>  (3)..(3)

<223>  Xaa is ManS

<400>  34

Xaa Gly Xaa Asp Asn Ala
1               5

<210>  35

<211>  6

<212>  PRT

<213>  Artificial Sequence

<220>

<223>  Binding ligand from peptide library

<220>

<221>  MISC_FEATURE

<222>  (1)..(1)

<223>  Xaa is ManS

<220>

<221>  MISC_FEATURE

<222>  (3)..(3)

<223>  Xaa is GlcNN

<220>

<221>  MISC_FEATURE

<222>  (5)..(5)

<223>  Xaa is ManS

<400>  35

Xaa Gly Xaa Asn Xaa Tyr
1               5


<210>  36

<211>  6

<212>  PRT

<213>  Artificial Sequence


<220>

<223>  Binding ligand from peptide library

<220>

<221>  MISC_FEATURE

<222>  (1)..(1)

<223>  Xaa is ManN


<400>  36

Xaa Phe Trp Ser Lys His
1               5


<210>  37

<211>  6

<212>  PRT

<213>  Artificial Sequence


<220>

<223>  Binding ligand from peptide library

<220>

<221>  MISC_FEATURE

<222>  (1)..(1)

<223>  Xaa is GlcNN

<400>  37

Xaa Trp Phe Asp Trp Pro
1               5


<210>  38

<211>  6

<212>  PRT

<213>  Artificial Sequence


<220>

<223>  Binding ligand from peptide library

<220>

<221>  MISC_FEATURE

<222>  (1)..(1)

<223>  Xaa is GlcNN


<220>

<221>  MISC_FEATURE

<222>  (3)..(3)

<223>  Xaa is GlcNN


<220>

<221>  MISC_FEATURE

<222>  (5)..(5)

<223>  Xaa is ManS


<400>  38

Xaa Val Xaa His Xaa Gly
1               5

<210> 39

<211> 6

<212> PRT

<213> Artificial Sequence

<220>

<223> Binding ligand from peptide library

<220>

<221> MISC_FEATURE

<222> (1)..(1)

<223> Xaa is ManN

<220>

<221> MISC_FEATURE

<222> (2)..(2)

<223> Xaa is ManS

<220>

<221> MISC_FEATURE

<222> (3)..(3)

<223> Xaa is ManN

<400> 39

Xaa Xaa Xaa Trp Ser Trp
1               5

<210> 40

<211> 6

<212> PRT

<213> Artificial Sequence

<220>

<223>  Binding ligand from peptide library

<400>  40

Gly Pro Lys Lys Tyr His
1               5


<210>  41

<211>  6

<212>  PRT

<213>  Artificial Sequence



<220>

<223>  Binding ligand from peptide library

<400>  41

His Thr Trp Gly Tyr Trp
1               5


<210>  42
<400>  42

000

<210>  43
<400>  43

000


<210>  44

<211>  6

<212>  PRT

<213>  Artificial



<220>

<221>  MISC_FEATURE

<223>  Binding ligand

<400>  44

Gly Pro Lys Lys Tyr His
1               5


<210>  45

<211>  6

<212>  PRT

<213>  Artificial



<220>

<221>  MISC_FEATURE

<223>  Binding ligand



<400>  45

His Thr Trp Gly Tyr Trp
1               5


<210>  46

<211>  6

<212>  PRT

<213>  Artificial



<220>

<221>  MISC_FEATURE

<223>  Binding ligand



<400>  46

Phe Thr Tyr Phe Ala Lys
1               5


<210>  47
<400>  47

000

<210> 48
<400> 48

000

<210> 49
<400> 49

000

<210> 50
<400> 50

000

<210> 51
<211> 6
<212> PRT
<213> Artificial sequence

<220>
<223> Binding ligand from peptide library

<400> 51

Phe Thr Tyr Phe Ala Lys
1               5

<210> 52
<400> 52

000

<210> 53
<400> 53

000

<210> 54
<400> 54

000

<210> 55
<400> 55

000

<210>  56
<400>  56

000


<210>  57

<211>  6

<212>  PRT

<213>  Artificial


<220>

<221>  MISC_FEATURE

<223>  Binding ligand


<220>

<221>  MISC_FEATURE

<222>  (3)..(3)

<223>  Xaa is Hyp


<220>

<221>  MISC_FEATURE

<222>  (6)..(6)

<223>  Xaa is Gua


<400>  57

Lys Met Xaa Trp Tyr Xaa
1               5


<210>  58

<211>  6

<212>  PRT

<213>  Artificial

<220>

<221>  MISC_FEATURE

<223>  Binding ligand


<220>

<221>  MISC_FEATURE

<222>  (4)..(4)

<223>  Xaa is Gua


<220>

<221>  MISC_FEATURE

<222>  (6)..(6)

<223>  Xaa is Gua


<400>  58

Phe Asp Trp Xaa Thr Xaa
1               5


<210>  59

<211>  5

<212>  PRT

<213>  Artificial


<220>

<221>  MISC_FEATURE

<223>  Binding ligand


<220>

<221>  MISC_FEATURE

<222>  (1)..(1)

<223>  Xaa is T(Sa)

<400>  59

Xaa Tyr Gly Ala Met
1               5

<210>  60

<211>  5

<212>  PRT

<213>  Artificial

<220>

<221>  MISC_FEATURE

<223>  Binding ligand

<220>

<221>  MISC_FEATURE

<222>  (1)..(1)

<223>  Xaa is T(Sa)

<400>  60

Xaa Ser Val Trp Phe
1               5

<210>  61

<211>  5

<212>  PRT

<213>  Artificial

<220>

<221>  MISC_FEATURE

<223>  Binding ligand

<220>

<221>  MISC_FEATURE

<222>  (1)..(1)

<223>  Xaa is T(Sa)

<400>  61

Xaa His Tyr Phe Phe
1               5

<210>  62

<211>  5

<212>  PRT

<213>  Artificial

<220>

<221>  MISC_FEATURE

<223>  Binding ligand

<220>

<221>  MISC_FEATURE

<222>  (1)..(1)

<223>  Xaa is T(Sa)

<400>  62

Xaa Ile Tyr Tyr Phe
1               5

<210>  63

<211>  5

<212>  PRT

<213>  Artificial

<220>

<221>  MISC_FEATURE

<223>  Binding ligand

<220>

<221>  MISC_FEATURE

<222>  (1)..(1)

<223>  Xaa is T(Sa)

<400>  63

Xaa Gln Pro Gly Met
1               5

<210>  64

<211>  5

<212>  PRT

<213>  Artificial

<220>

<221>  MISC_FEATURE

<223>  Binding ligand

<220>

<221>  MISC_FEATURE

<222>  (1)..(1)

<223>  Xaa is T(Sa)

<400>  64

Xaa Gly Pro His Gly
1               5

1

1

12